# EUROPEAN PATENT SPECIFICATION

(11) **EP 3 455 376 B1**
(45) Date of publication and mention of the grant of the patent: **31.07.2024**
(21) Application number: 17737065.7
(22) Date of filing: 26.06.2017
(51) Int. Cl.: C12Q 1/68, C12N 15/10

(54) **METHOD FOR PRODUCING A PLURALITY OF DNA PROBES AND METHOD FOR ANALYZING GENOMIC DNA USING THE DNA PROBES**
VERFAHREN ZUR HERSTELLUNG EINER VIELZAHL AN DNA-SONDEN UND VERFAHREN ZUR ANALYSE VON GENOMISCHER DNA UNTER VERWENDUNG DER DNA-SONDEN
PROCÉDÉ DE PRODUCTION D'UNE PLURALITÉ DE SONDES D'ADN ET PROCÉDÉ D'ANALYSE D'ADN GÉNOMIQUE À L'AIDE DE LA SONDE D'ADN

(30) Priority: 29.06.2016 JP 2016129080
(43) Date of publication of application: 20.03.2019
(73) Proprietor: TOYOTA JIDOSHA KABUSHIKI KAISHA, Toyota-shi, Aichi-ken, 471-8571 (JP)
(72) Inventor: ENOKI Hiroyuki, Toyota-shi, Aichi-ken, 471-8571 (JP); TAKEUCHI Yoshie, Toyota-shi, Aichi-ken, 471-8571 (JP); INAMORI Minoru, Toyota-shi, Aichi-ken, 471-8571 (JP)
(74) Representative: Winter, Brandl - Partnerschaft mbB
(86) International application number: PCT/JP2017/023343
(87) International publication number: WO 2018/003727

(56) References cited:
- EP-A1- 1 721 970
- EP-A1- 2 514 820
- WO-A2-2008/101701

## Description

### Technical Field

The present invention relates to a method for producing a DNA library that can be used for analyzing a DNA marker or other purposes and a method for gene analysis using such DNA library.

### Background Art

In general, genomic analysis is performed to conduct comprehensive analysis of genetic information contained in the genome, such as nucleotide sequence information. However, an analysis aimed at determination of the nucleotide sequence for whole genome is disadvantageous in terms of the number of processes and the cost. In cases of organisms with large genomic sizes, in addition, genomic analysis based on nucleotide sequence analysis has limitations because of genome complexity.

Patent Literature 1 discloses an amplified fragment length polymorphism (AFLP) marker technique wherein a sample-specific marker is incorporated into a restriction-enzyme-treated fragment that had been ligated to an adaptor and only a part of the sequence of the restriction-enzyme-treated fragment is to be determined. According to the technique disclosed in Patent Literature 1, the complexity of genomic DNA is reduced by treating genomic DNA with a restriction enzyme, the nucleotide sequence of a target part of the restriction-enzyme-treated fragment is determined, and the target restriction-enzyme-treated fragment is thus determined sufficiently. The technique disclosed in Patent Literature 1, however, requires processes such as treatment of genomic DNA with a restriction enzyme and ligation reaction with the use of an adaptor. Thus, it is difficult to achieve a cost reduction.

Meanwhile, Patent Literature 2 discloses as follows. That is, a DNA marker for identification that is highly correlated with the results of taste evaluation was found from among DNA bands obtained by amplifying DNAs extracted from a rice sample via PCR in the presence of adequate primers by the so-called RAPD (randomly amplified polymorphic DNA) technique. The method disclosed in Patent Literature 2 involves the use of a plurality of sequence-tagged sites (STSs, which are primers) identified by particular sequences. According to the method disclosed in Patent Literature 2, a DNA marker for identification amplified with the use of an STSprimer is detected via electrophoresis. However, the RAPD technique disclosed in Patent Literature 2 yields significantly poor reproducibility of PCR amplification, and, accordingly, such technique cannot be generally adopted as a DNA marker technique.

Patent Literature 3 discloses a method for producing a genomic library wherein PCR is carried out with the use of a single type of primer designed on the basis of a sequence that appears relatively frequently in the target genome, the entire genomic region is substantially uniformly amplified, and a genomic library can be thus produced. While Patent Literature 3 describes that a genomic library can be produced by conducting PCR with the use of a random primer containing a random sequence, it does not describe any actual procedures or results of experimentation. Accordingly, the method described in Patent Literature 3 is deduced to require nucleotide sequence information of the genome so as to identify the genome appearing frequency, which would increase the number of procedures and the cost. According to the method described in Patent Literature 3, in addition, the entire genome is to be amplified, and complexity of genomic DNA cannot be reduced, disadvantageously.

Patent Literature 4 discloses a high-throughput technique associated with markers that involves reduction in genome complexity by restriction enzyme treatment in combination with an array technique. According to the technique associated with markers disclosed in Patent Literature 4, genomic DNA is digested with restriction enzymes, an adaptor is ligated to the resulting genomic DNA fragment, a DNA fragment is amplified with the use of a primer hybridizing to the adaptor, and a DNA probe used for detection of such DNA fragment is then designed on the basis of the nucleotide sequence of the amplified DNA fragment.

In addition, Non-Patent Literature 1 discloses the development of high-density linkage map containing several thousands of DNA markers for sugarcane and wheat by making use of the technique disclosed in Patent Literature 4. Also, Non-Patent Literature 2 discloses the development of a high-density linkage map containing several thousands of DNA markers for buck wheat by making use of the technique disclosed in Patent Literature 4.

Further, Patent Literature 5 discloses a method involving the use of a random primer as a sample to be reacted with an array on which a probe is immobilized. However, Patent Literatures does not discloses a method in which a random primers is used to obtain an amplified fragment and the resulting amplified fragment is used to construct a DNA library. Further, Patent Literature 6 describes the producing of organism-specific probe sequences by amplifying genomic DNA with a primer sequence comprising repetitive nucleic acid units. Patent Literature 7 describes a method for designing probe sequences by restriction enzyme digesting genomic DNA. Patent Literature 8 describes a method for preparing a library of genomic sequences

### Citation List

### Patent Literature

PTL 1: JP 5 389 638
PTL 2: JP 2003 - 79 375 A
PTL 3: JP. 3 972 106
PTL 4: JP. 5 799 484
PTL 5: JP 2014 - 204 730 A
PTL 6: WO 2008 / 101 701 A2
PTL 7: EP 2 514 820 A1
PTL 8: EP 1 721 970 A1

### Non Patent Literature

NPL 1: DNA Research 21, 555-567, 2014
NPL 2: Breeding Science 64: 291-299, 2014

### Summary of Invention

### Technical Problem

A technique for genome information analysis, such as genetic linkage analysis conducted with the use of a DNA marker, is desired to produce a DNA library in a more convenient and highly reproducible manner. In addition, such technique is desired to produce a DNA probe capable of detecting a DNA fragment contained in a DNA library with high accuracy. As described above, a wide variety of techniques for producing a DNA library and a DNA probe are known. To date, however, there have been no techniques known to be sufficient in terms of convenience and/or reproducibility. Under the above circumstances, it is an object of the present invention to provide a method for producing a DNA probe that is applicable to a DNA library produced by a method with more convenience and higher reproducibility, and it is another object to provide a method for analyzing genomic DNA with the use of such DNA probe.

### Solution to Problem

This object is solved by the subject matter of the independent claims 1 and 4. Further aspects are disclosed in the subclaims. The present inventors have conducted concentrated studies in order to attain the above objects. As a result, they discovered that a DNA library could be produced with high reproducibility by conducting PCR with the use of a random primer while designating the concentration of such random primer within a designated range in a reaction solution and that a DNA probe could be easily designed on the basis of the nucleotide sequences of the DNA library to be produced. This has led to the completion of the present invention.

### Advantageous Effects of Invention

In the method for producing a plurality of DNA probes according to the present invention, a nucleotide sequence of a DNA probe is designed based on the nucleotide sequence of DNA fragments produced by the method of nucleic acid amplification using a random primer at a high concentration. According to the method of nucleic acid amplification using a random primer at a high concentration, DNA fragments can be amplified with excellent reproducibility. According to the present invention, therefore, a DNA probe applicable to a DNA fragment that can be obtained while achieving excellent reproducibility can be produced in a simple manner.

According to the method for producing a plurality of DNA probes according to the present invention, also, a DNA probe applicable to a DNA fragment can be produced while achieving excellent reproducibility, and the resulting DNA probe can be used for genetic analysis, such as genetic linkage analysis, involving the use of a DNA fragment of interest as a DNA marker.

The method for analyzing genomic DNA with the use of DNA probes according to the present invention involves the use of a DNA probe applicable to a DNA fragment produced in a simple manner with excellent reproducibility. Accordingly, genomic DNA can be analyzed in a cost-effective manner with high accuracy.

### Brief Description of Drawings

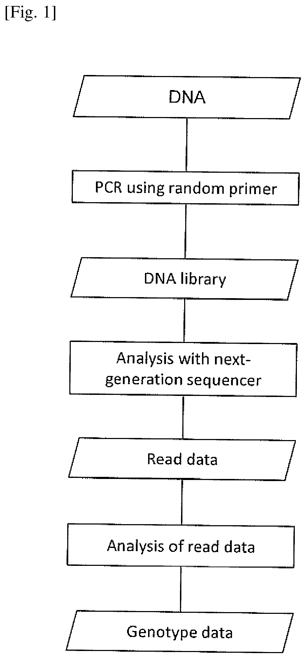

[Fig. 1]Fig. 1 shows a flow chart demonstrating a method for producing a DNA library and a method for genetic analysis with the use of the DNA library.

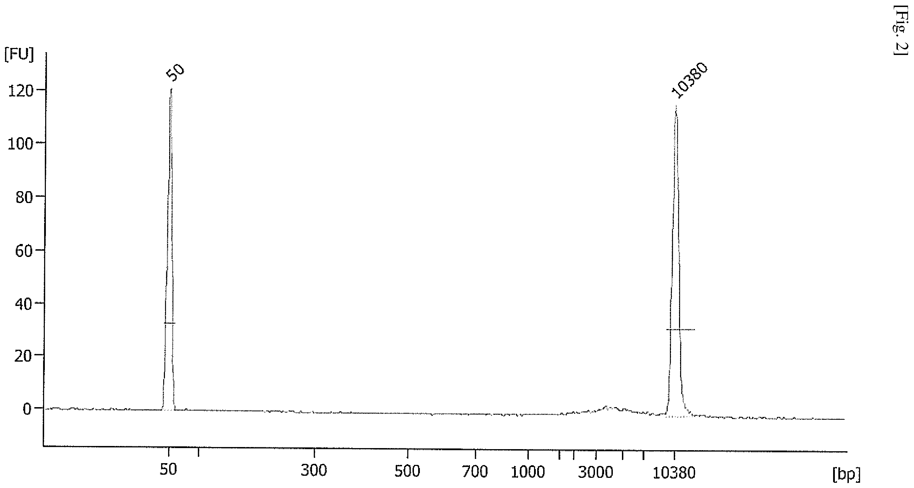

[Fig. 2]Fig. 2 shows a characteristic diagram demonstrating a correlation between an amplified fragment length and a fluorescence unit (FU) in which the amplified fragment length is determined based on an electrophoretic pattern of the DNA library amplified via PCR using DNA of the sugarcane variety NiF8 as a template under general conditions.

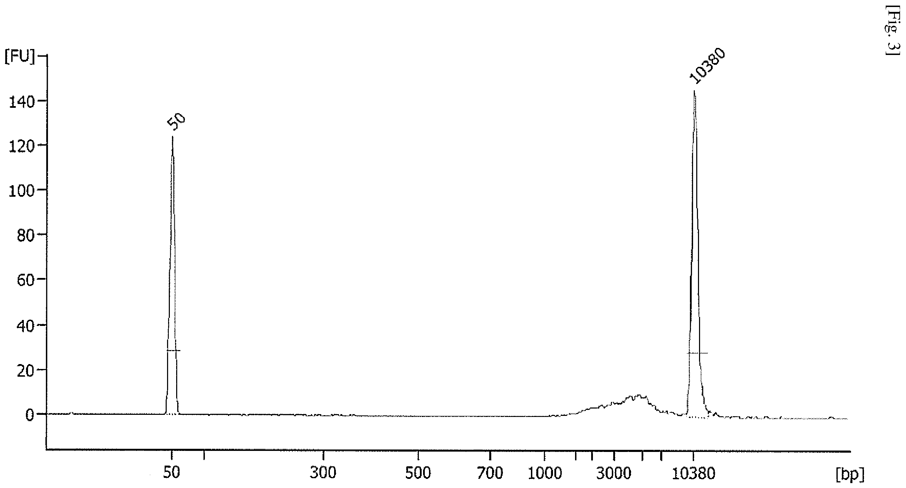

[Fig. 3]Fig. 3 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template at an annealing temperature of 45 degrees C.

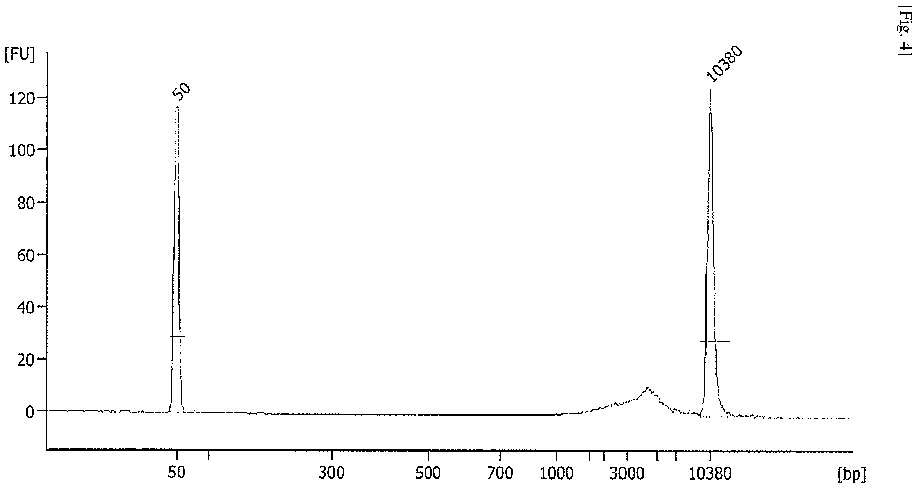

[Fig. 4]Fig. 4 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template at an annealing temperature of 40 degrees C.

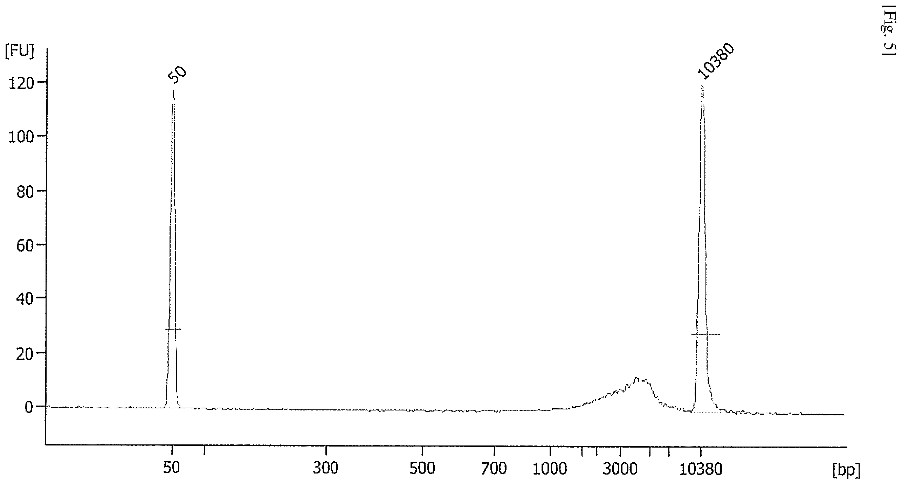

[Fig. 5]Fig. 5 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template at an annealing temperature of 37 degrees C.

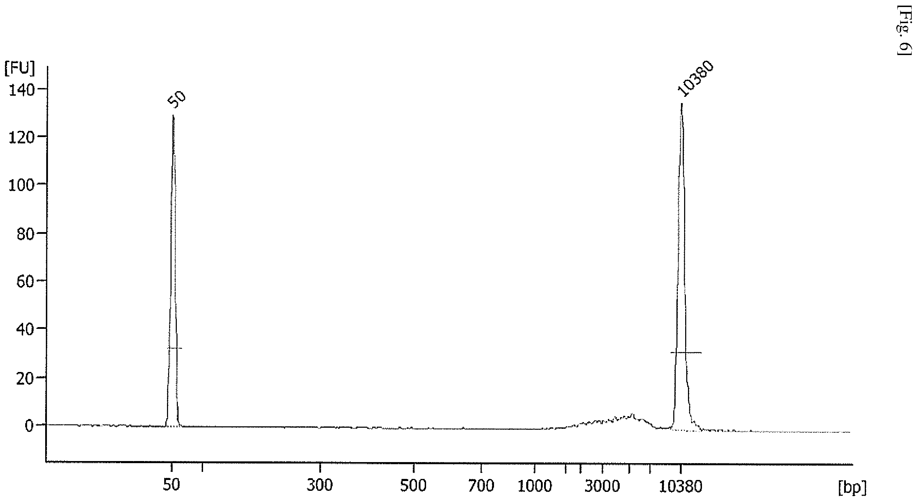

[Fig. 6]Fig. 6 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and 2.5 units of an enzyme.

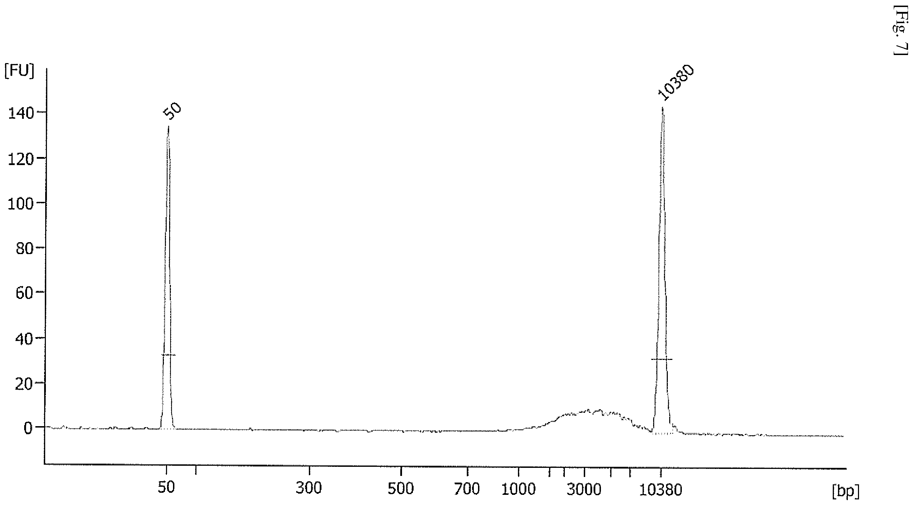

[fig.7]Fig. 7 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and 12.5 units of an enzyme.

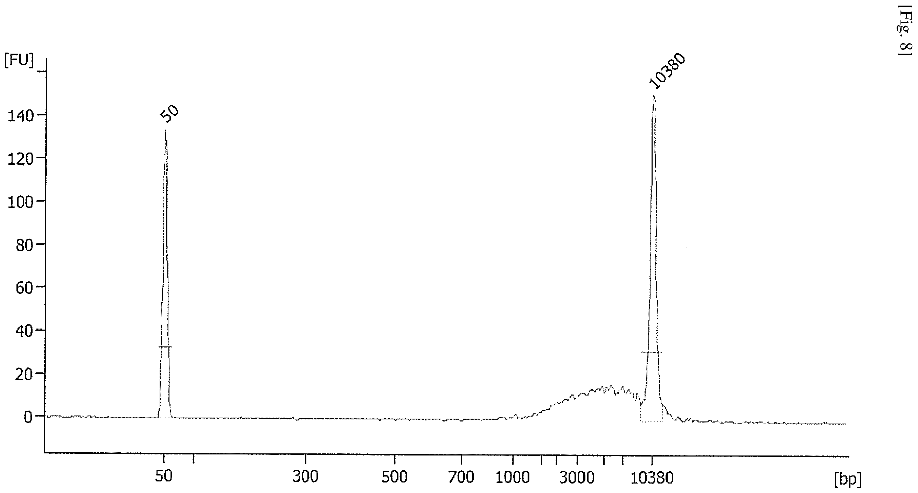

[fig.8]Fig. 8 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and MgCl₂ at the concentration doubled from the original level.

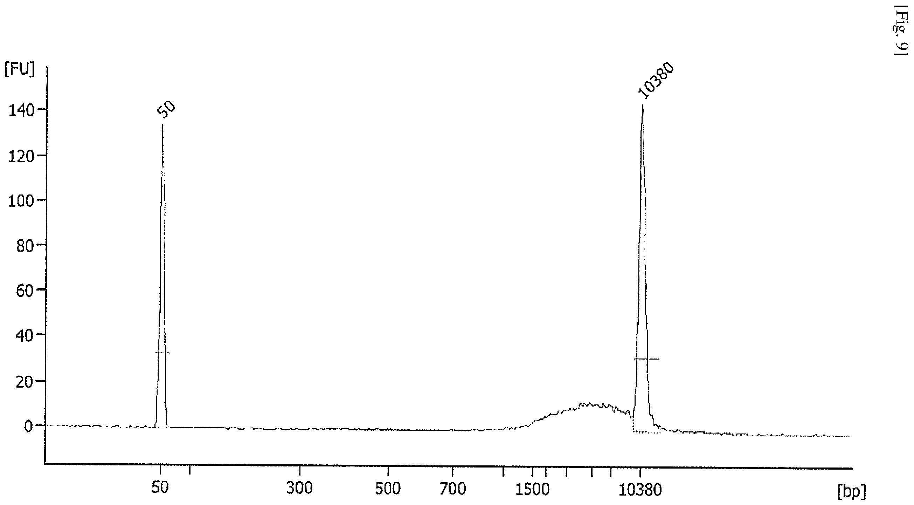

[fig.9]Fig. 9 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and MgCl₂ at the concentration tripled from the original level.

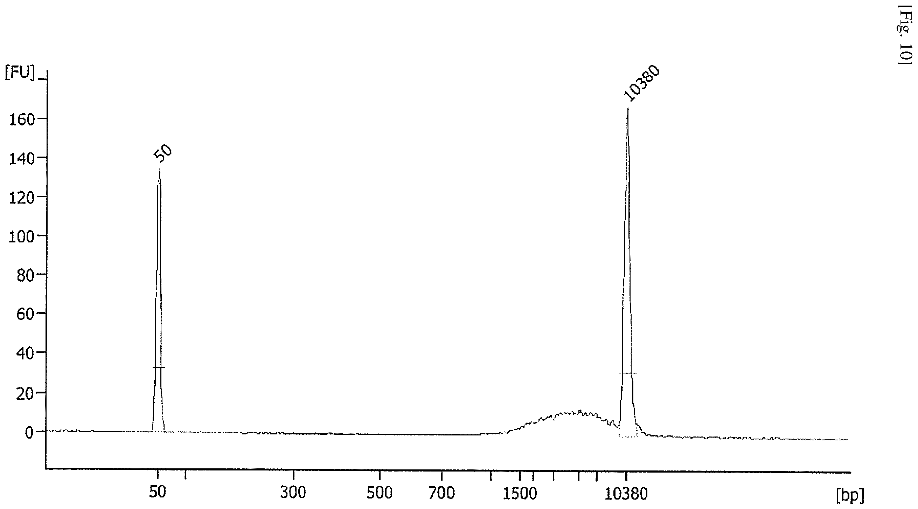

[fig. 10]Fig. 10 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and MgCl₂ at the concentration quadrupled concentration.

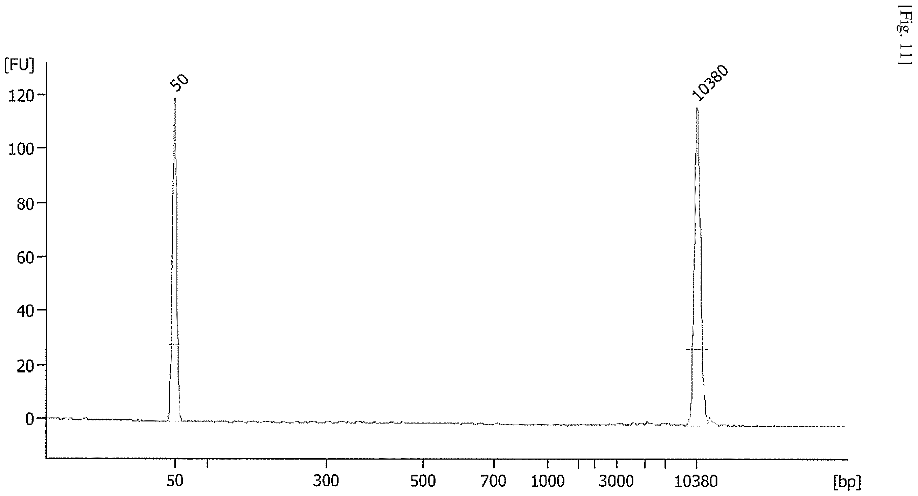

[fig.11]Fig. 11 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 8 bases.

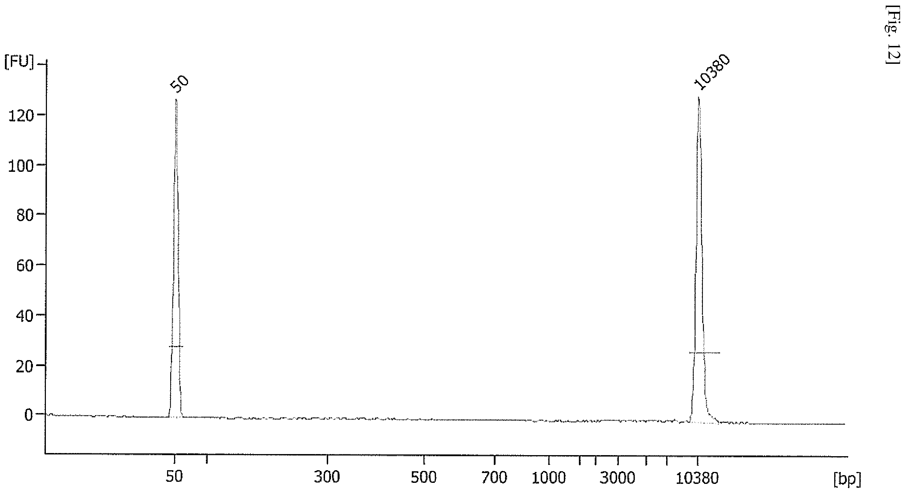

[fig.12]Fig. 12 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 9 bases.

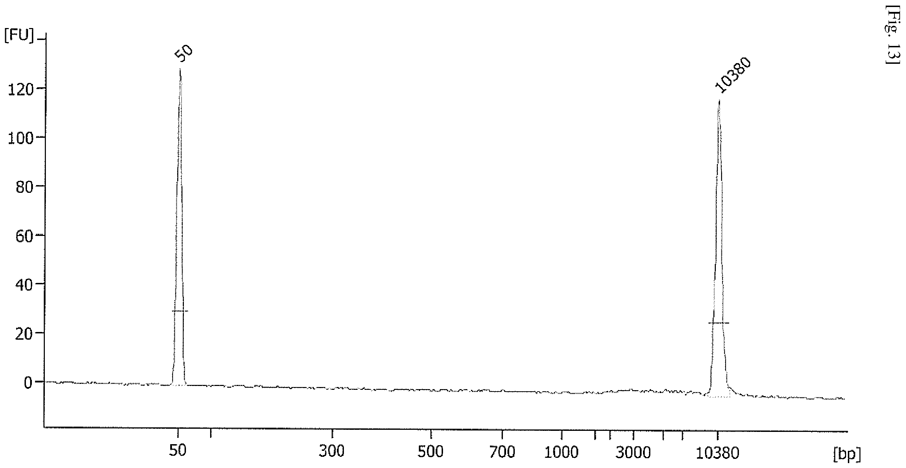

[fig. 13]Fig. 13 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 11 bases.

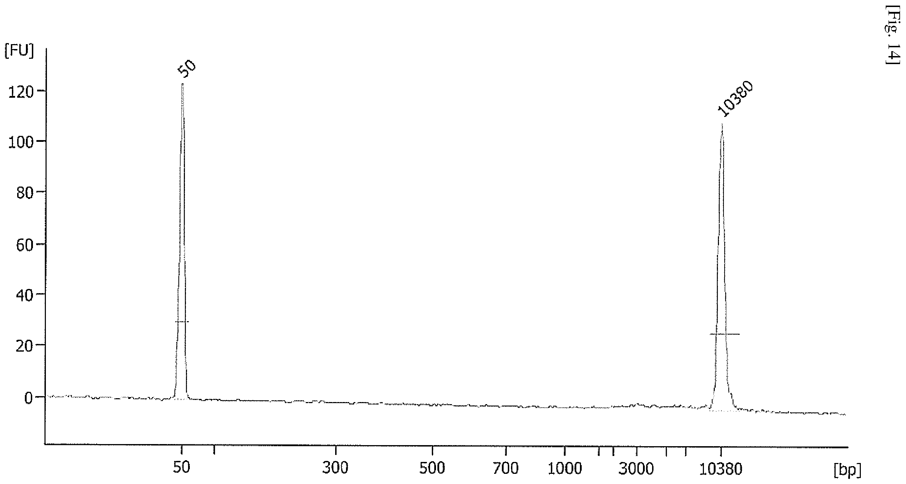

[fig.14]Fig. 14 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 12 bases.

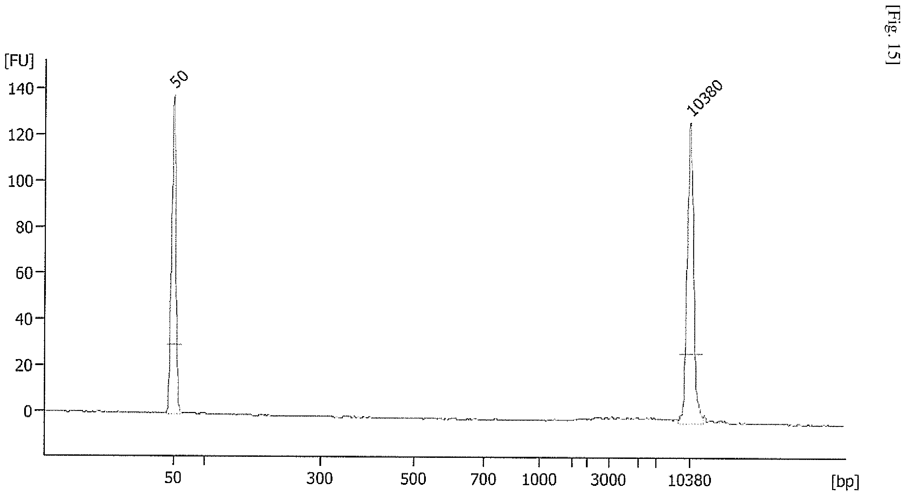

[fig.15]Fig. 15 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 14 bases.

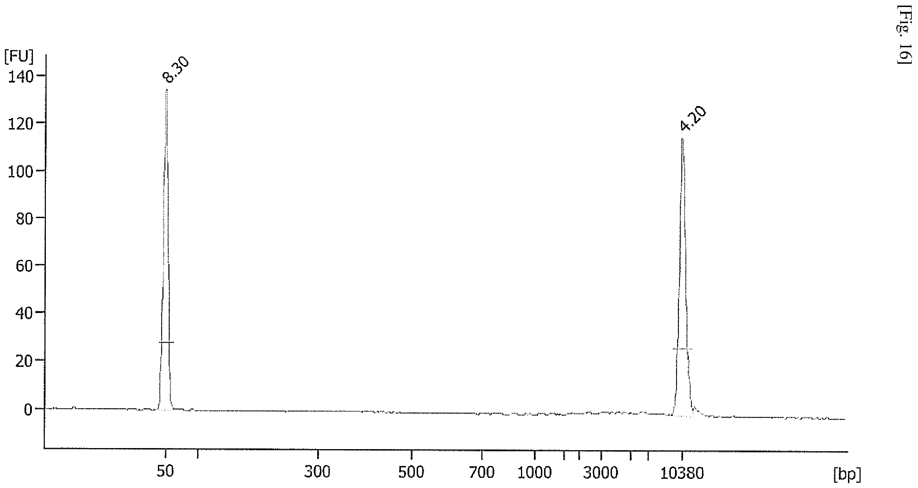

[fig.16]Fig. 16 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 16 bases.

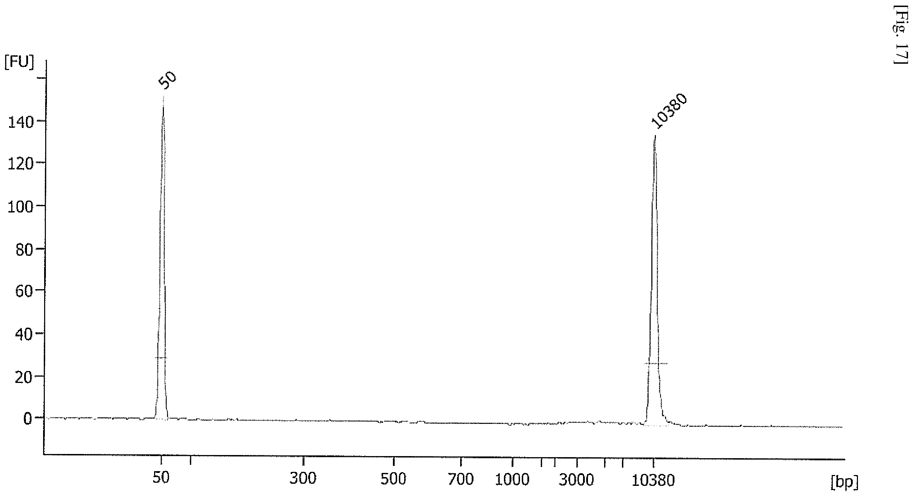

[fig.17]Fig. 17 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 18 bases.

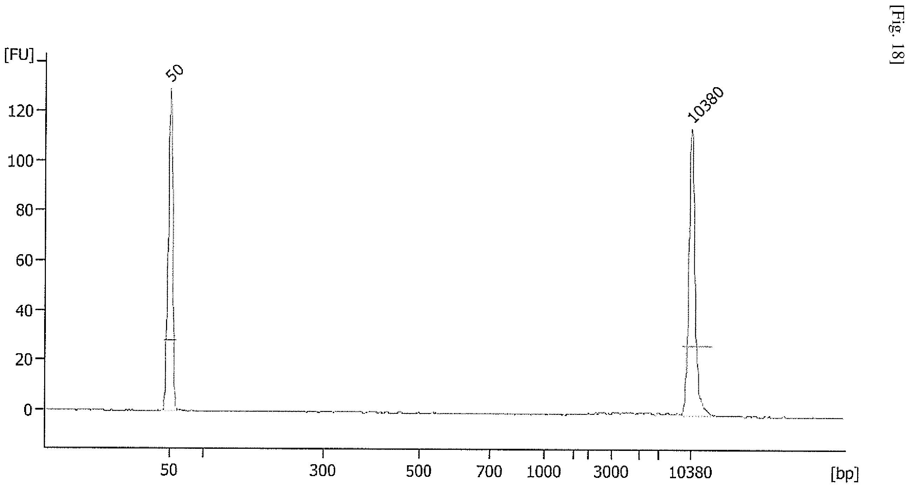

[fig.18]Fig. 18 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer comprising 20 bases.

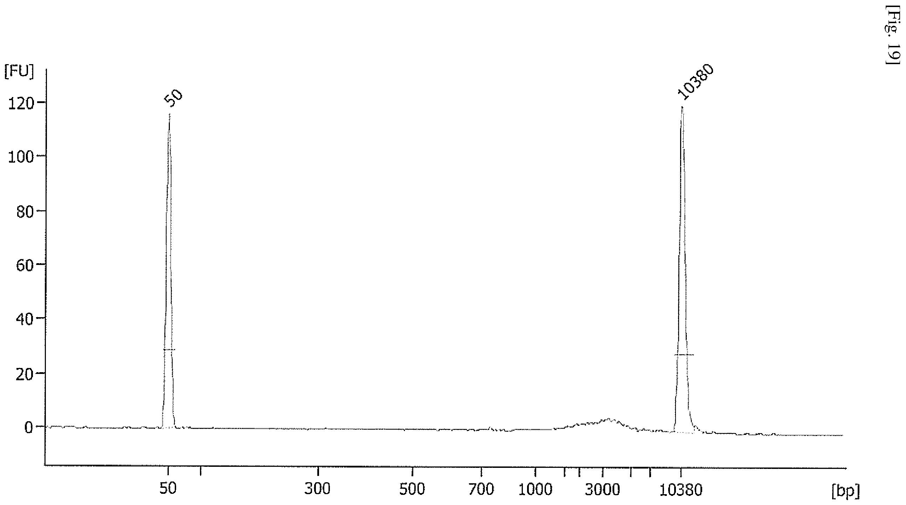

[fig.19]Fig. 19 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 2 microM.

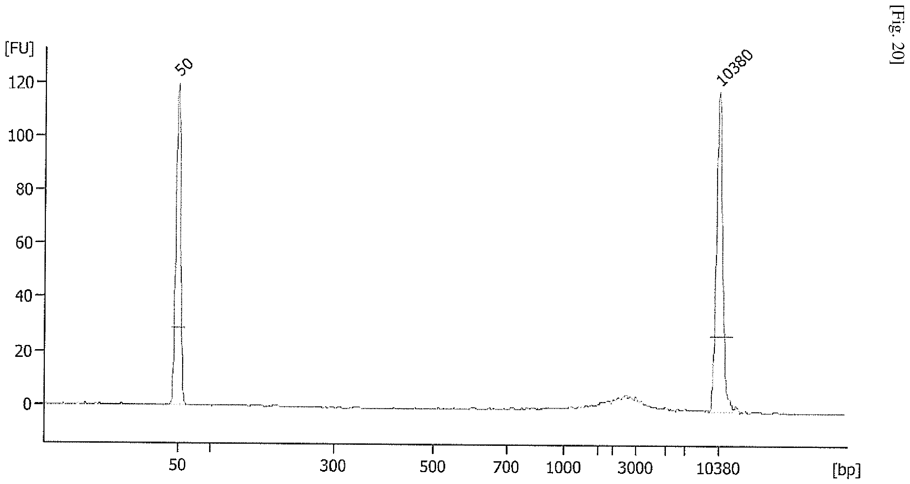

[fig.20]Fig. 20 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 4 microM.

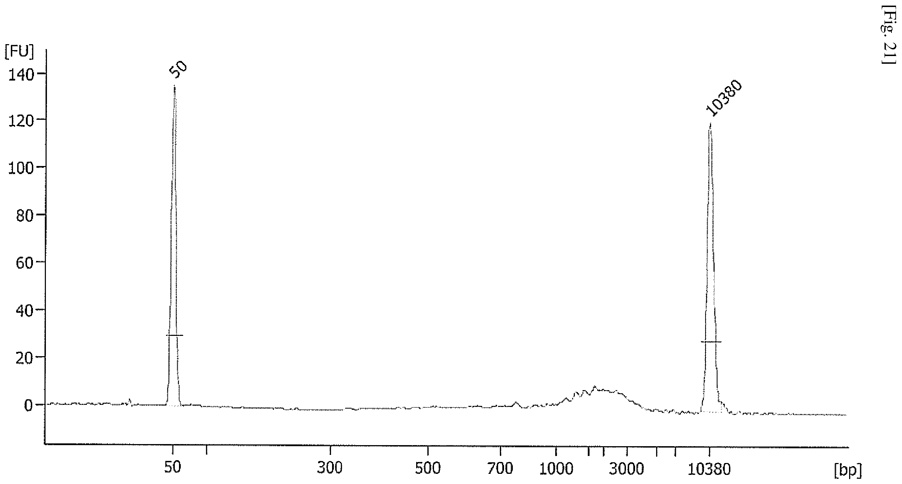

[fig.21]Fig. 21 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 6 microM.

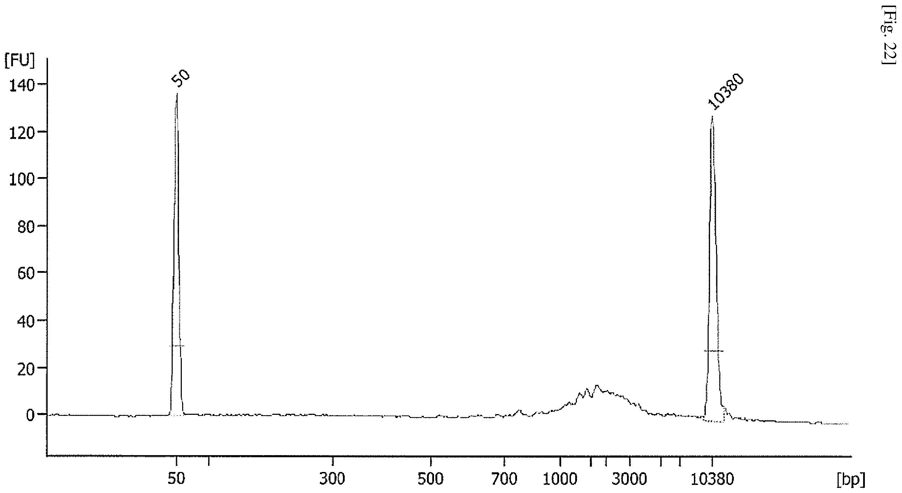

[fig.22]Fig. 22 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 6 microM.

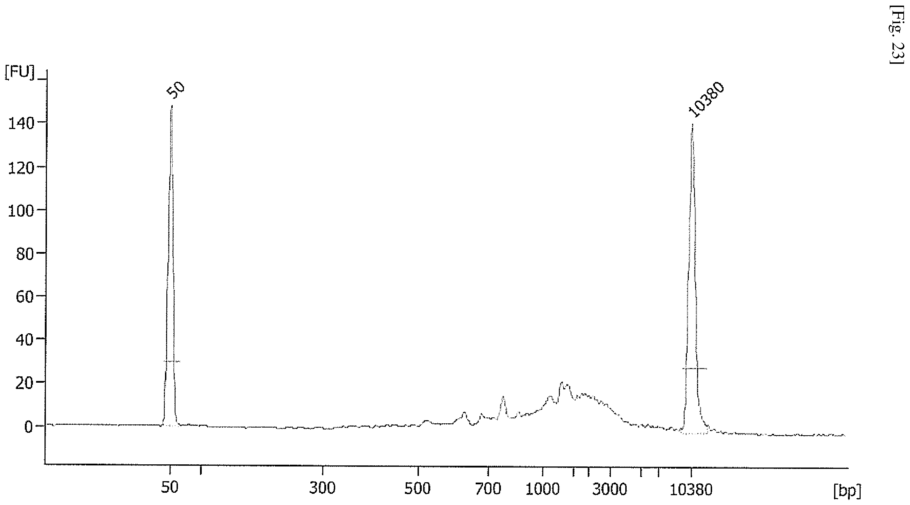

[fig.23]Fig. 23 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 8 microM.

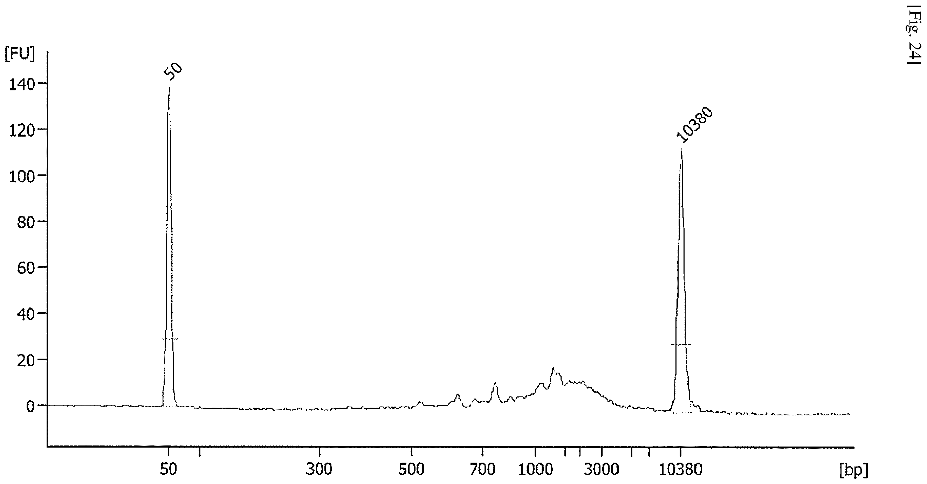

[fig.24]Fig. 24 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 8 microM.

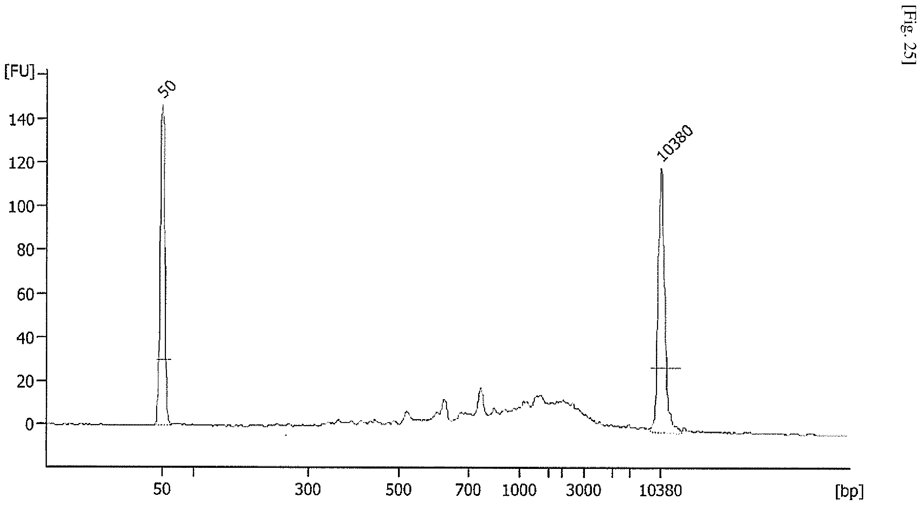

[fig.25]Fig. 25 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 10 microM.

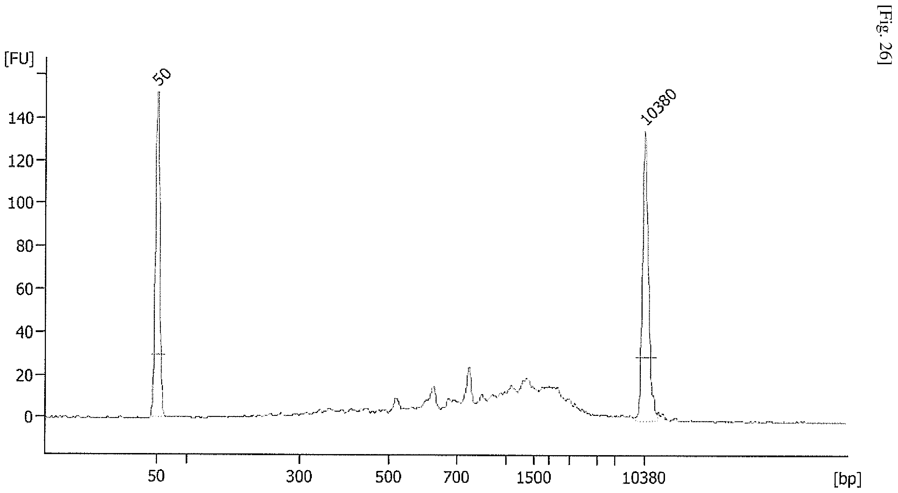

[fig.26]Fig. 26 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 10 microM.

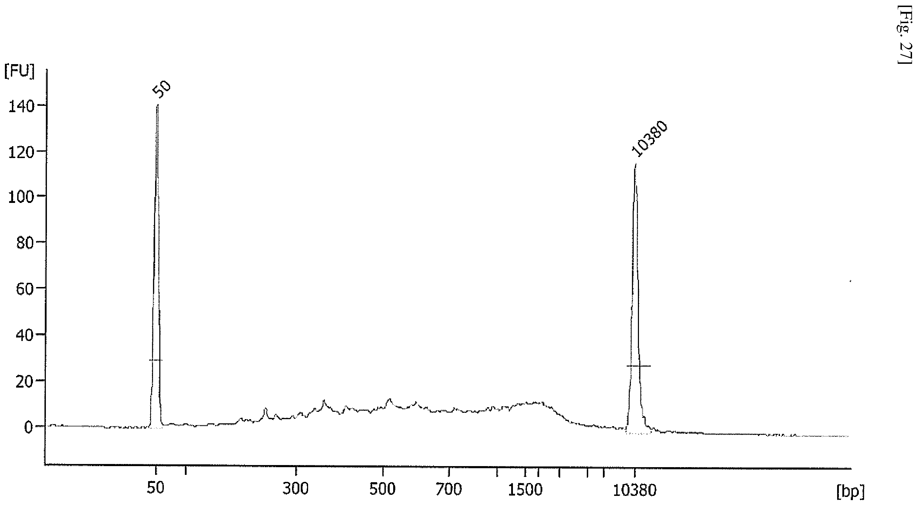

[fig.27]Fig. 27 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 20 microM.

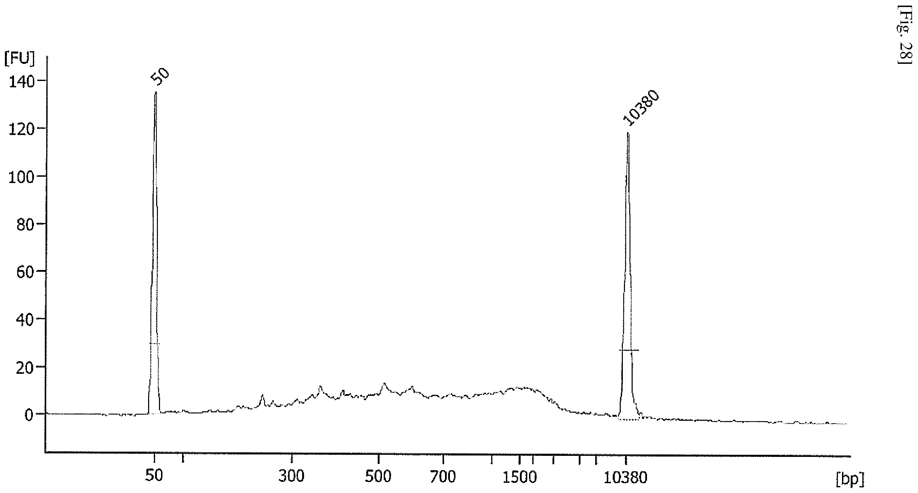

[fig.28]Fig. 28 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 20 microM.

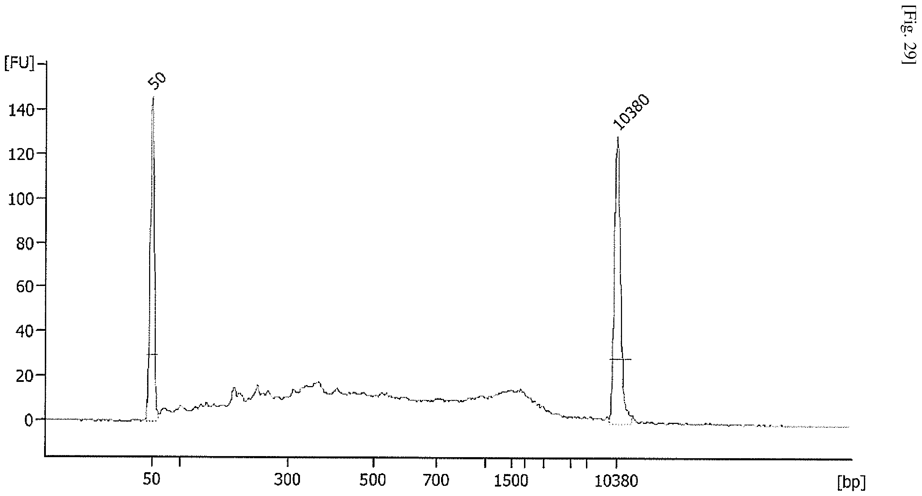

[fig.29]Fig. 29 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 40 microM.

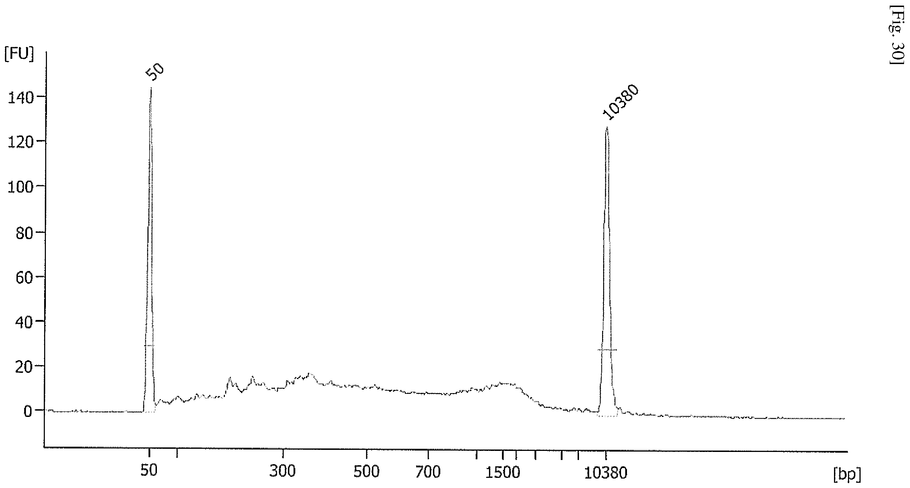

[fig.30]Fig. 30 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 40 microM.

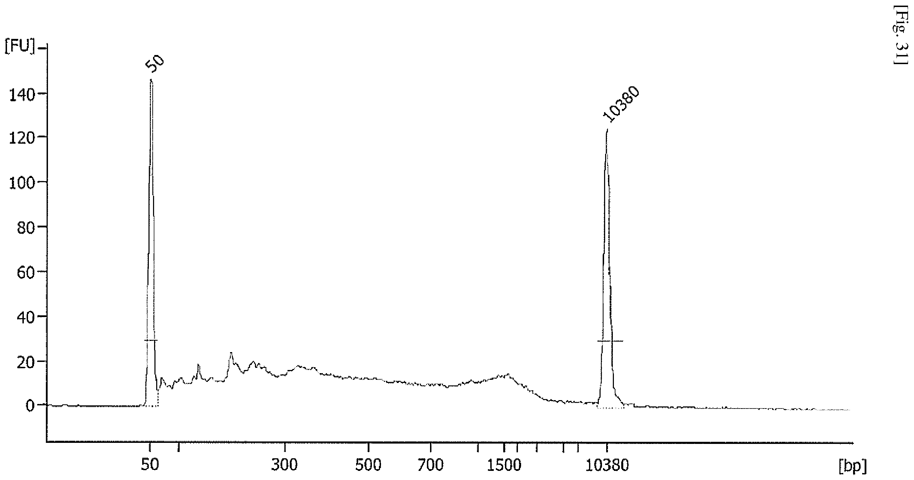

[fig.31]Fig. 31 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 60 microM.

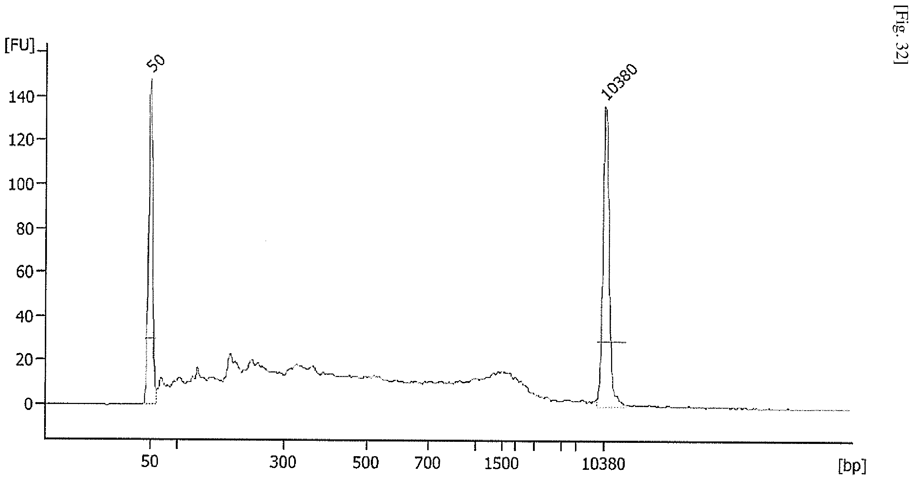

[fig.32]Fig. 32 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 60 microM.

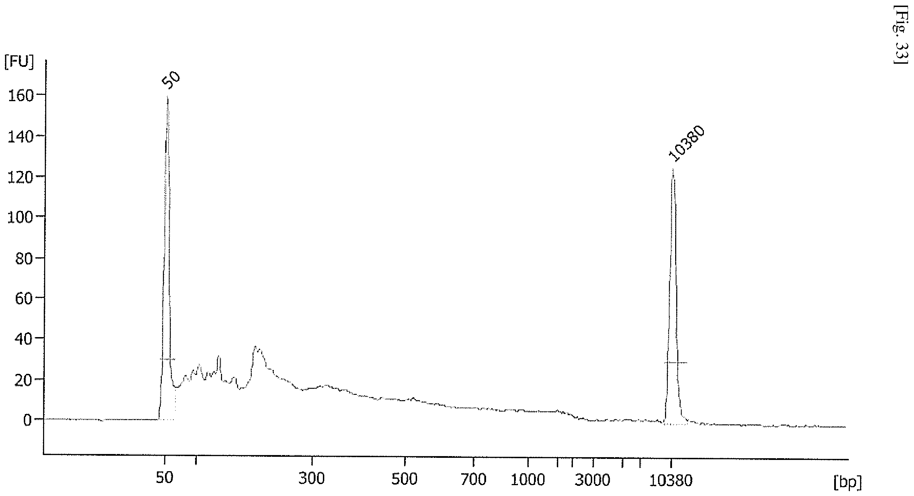

[fig.33]Fig. 33 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 100 microM.

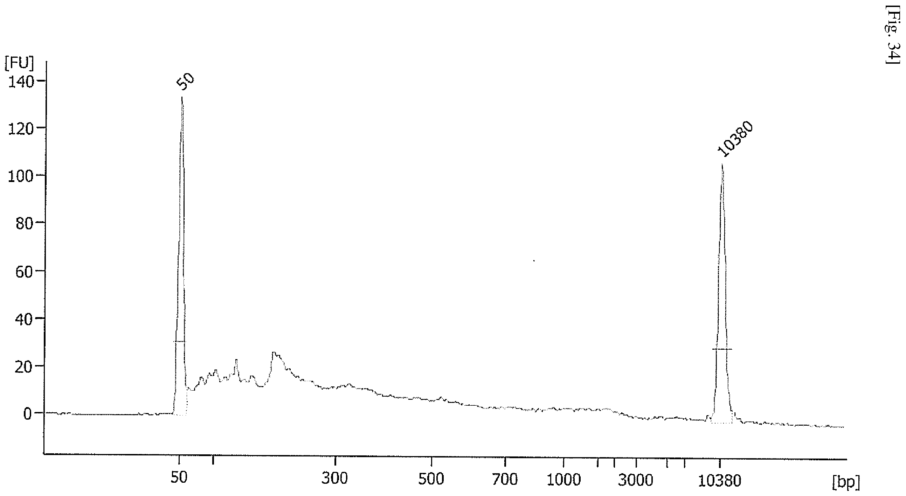

[fig.34]Fig. 34 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 100 microM.

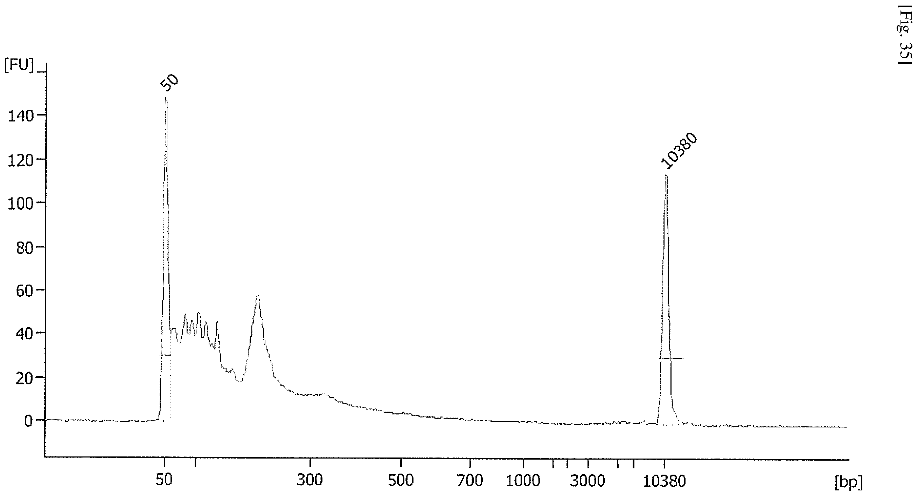

[fig.35]Fig. 35 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 200 microM.

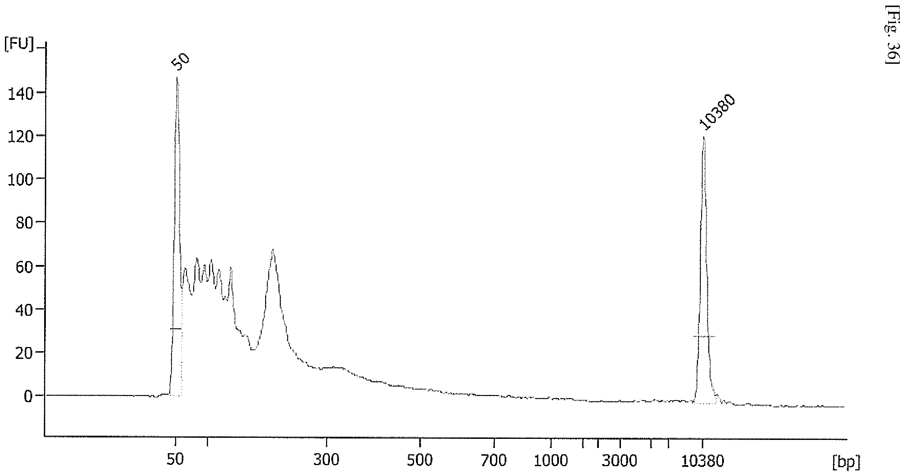

[fig.36]Fig. 36 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 200 microM.

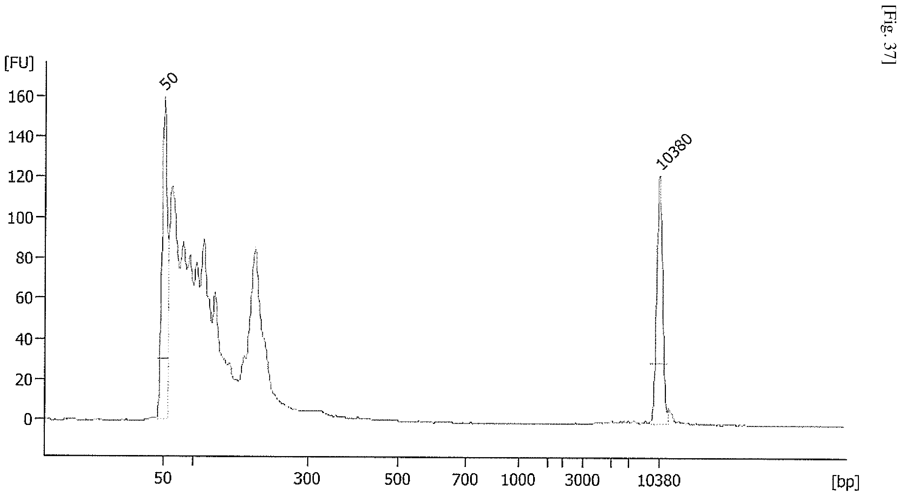

[fig.37]Fig. 37 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 300 microM.

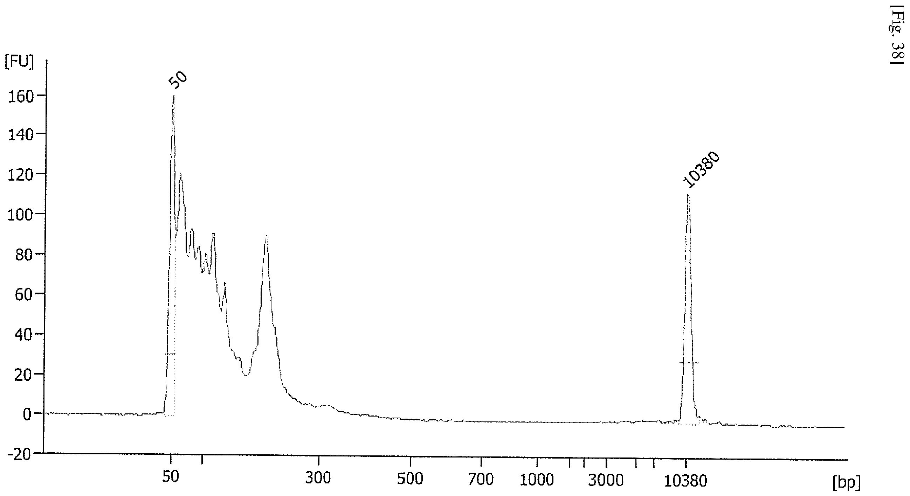

[fig.38]Fig. 38 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 300 microM.

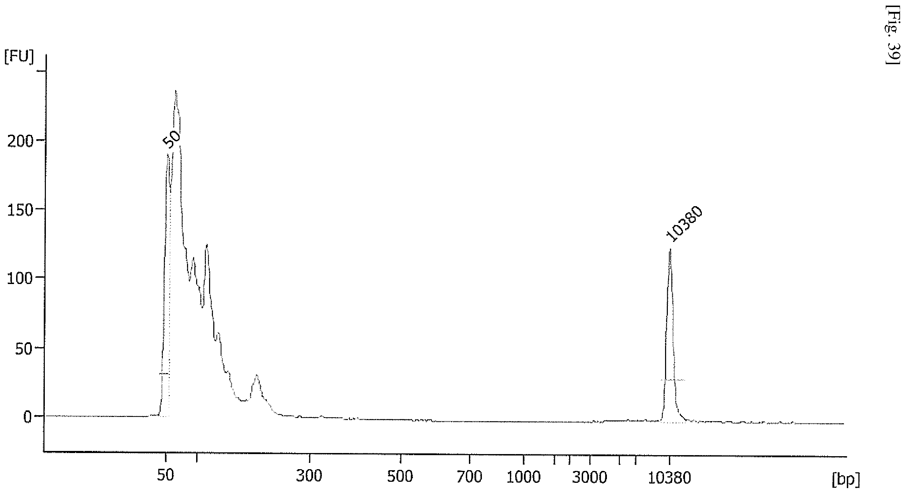

[fig.39]Fig. 39 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 400 microM.

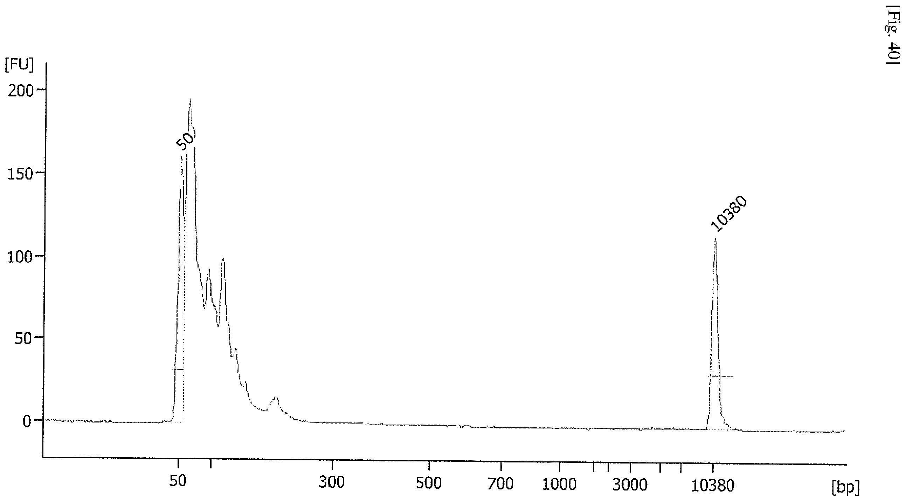

[fig.40]Fig. 40 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 400 microM.

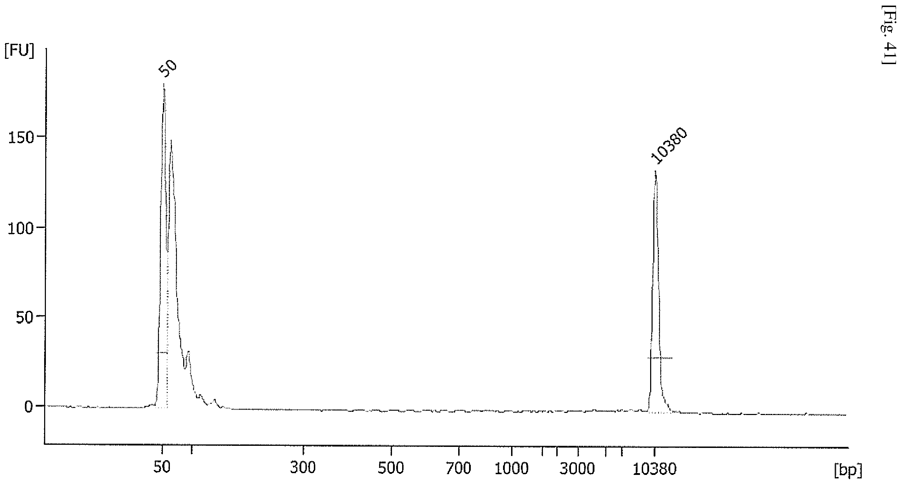

[fig.41]Fig. 41 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer at a concentration of 500 microM.

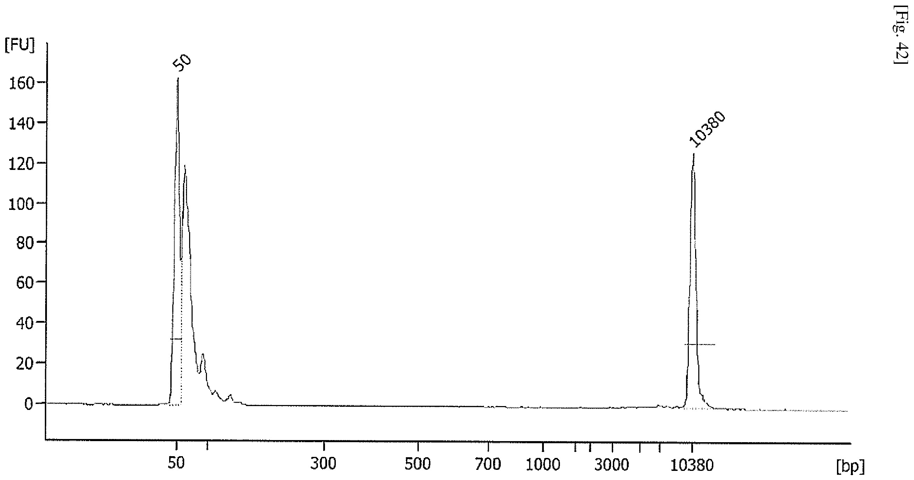

[fig.42]Fig. 42 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer at a concentration of 500 microM.

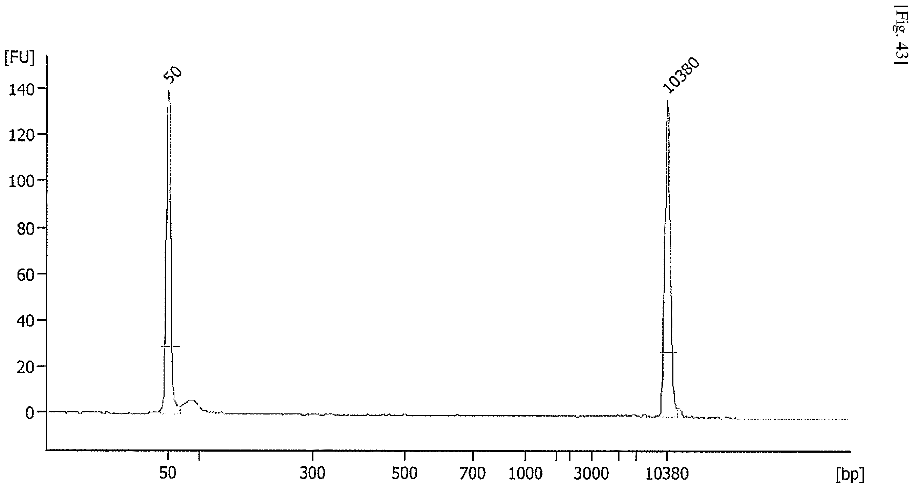

[fig.43]Fig. 43 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 600 microM.

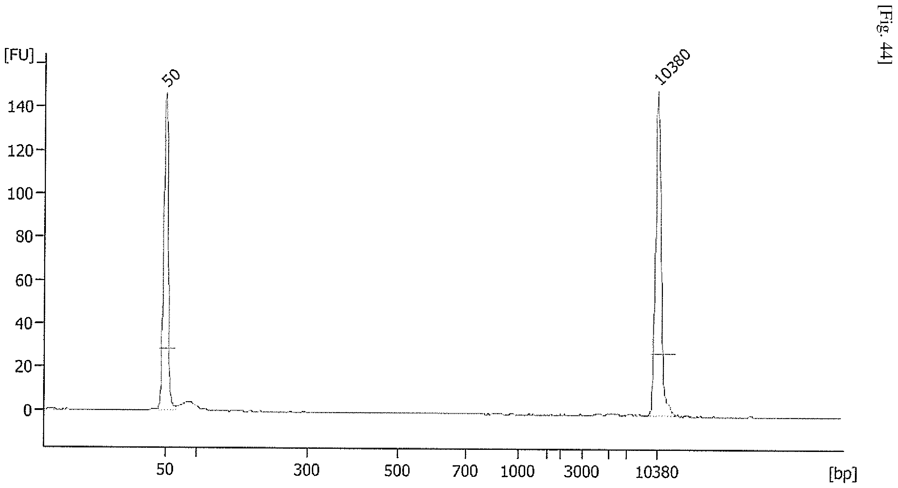

[fig.44]Fig. 44 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 700 microM.

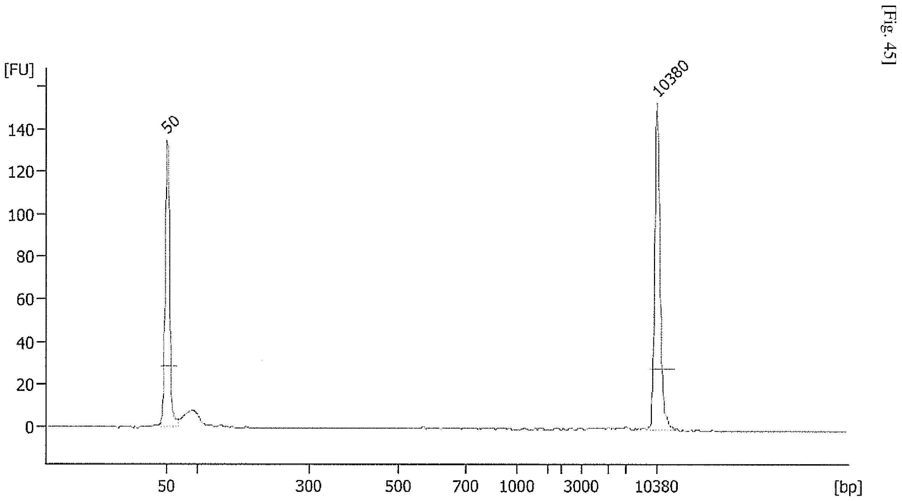

[fig.45]Fig. 45 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 800 microM.

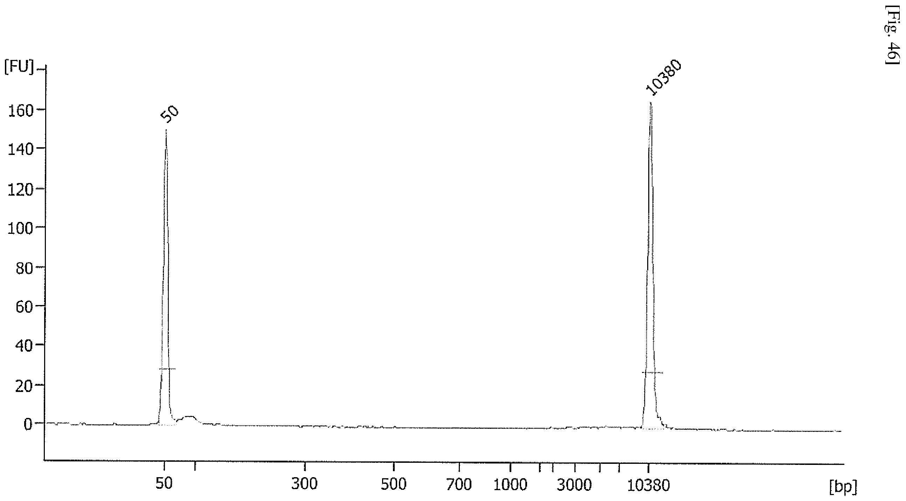

[fig.46]Fig. 46 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 900 microM.

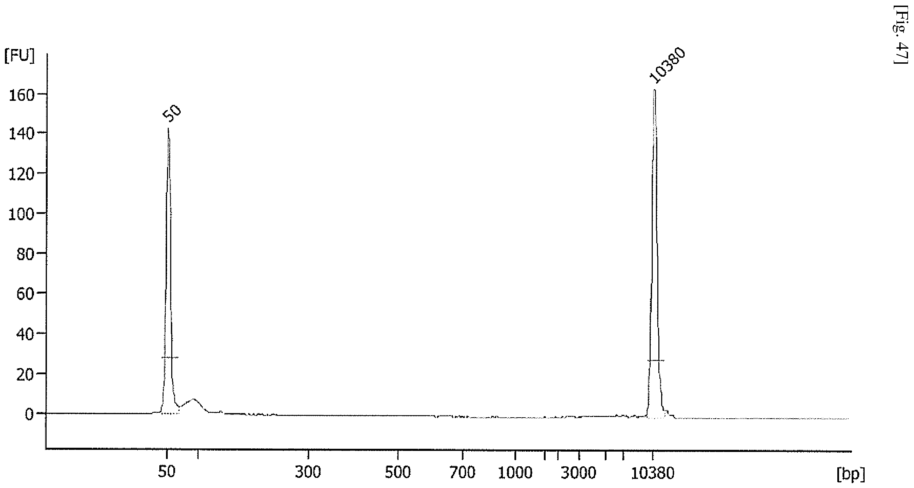

[fig.47]Fig. 47 shows a characteristic diagram demonstrating a correlation between an amplified fragment length and a fluorescence unit (FU) in which the amplified fragment length is determined based on an electrophoretic pattern of the DNA library amplified using DNA of the sugarcane variety NiF8 as a template and a random primer at a concentration of 1000 microM.

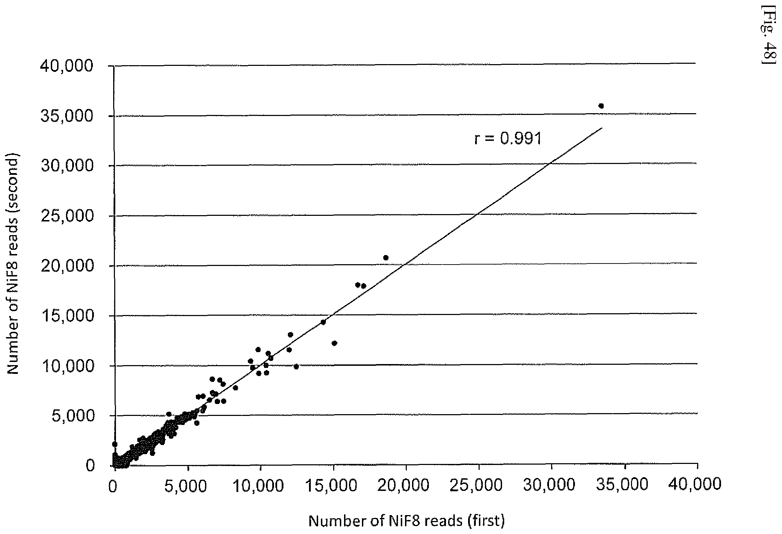

[fig.48]Fig. 48 shows a characteristic diagram demonstrating the results of MiSeq analysis of a DNA library amplified using DNA of the sugarcane variety NiF8 as a template and a random primer.

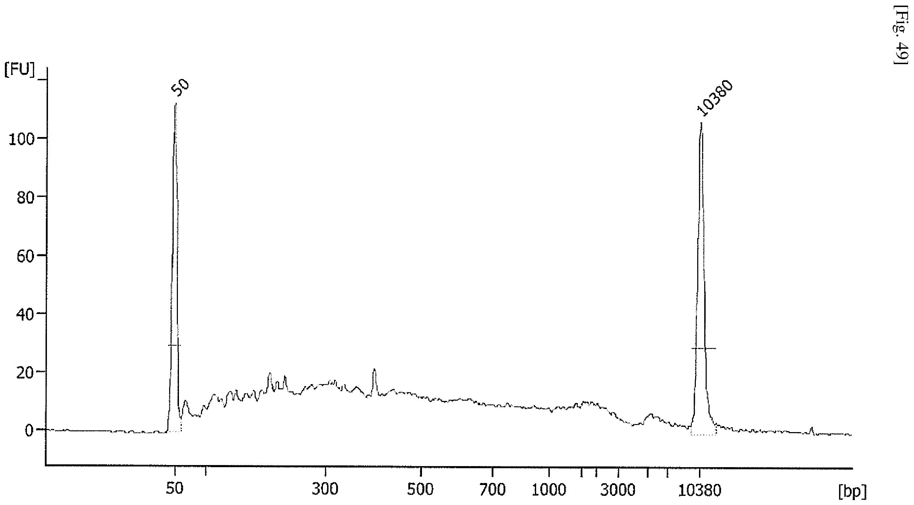

[fig.49]Fig. 49 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the rice variety Nipponbare as a template and a random primer.

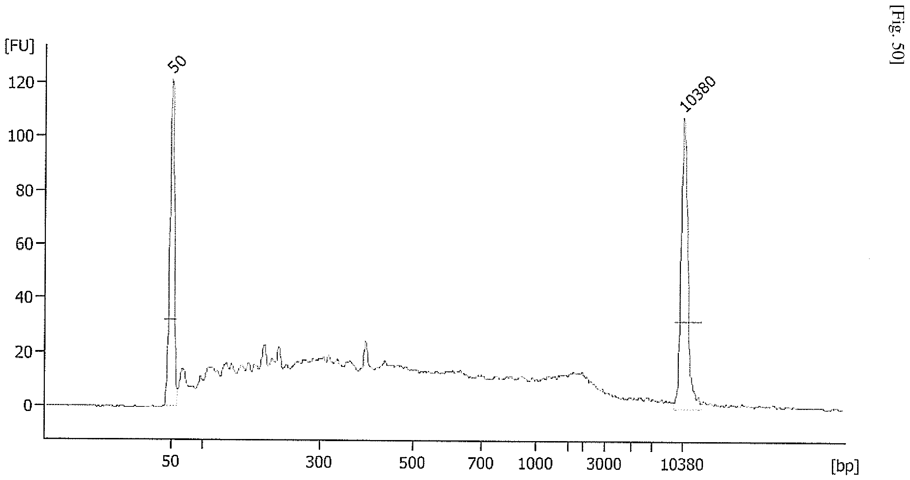

[fig.50]Fig. 50 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the rice variety Nipponbare as a template and a random primer.

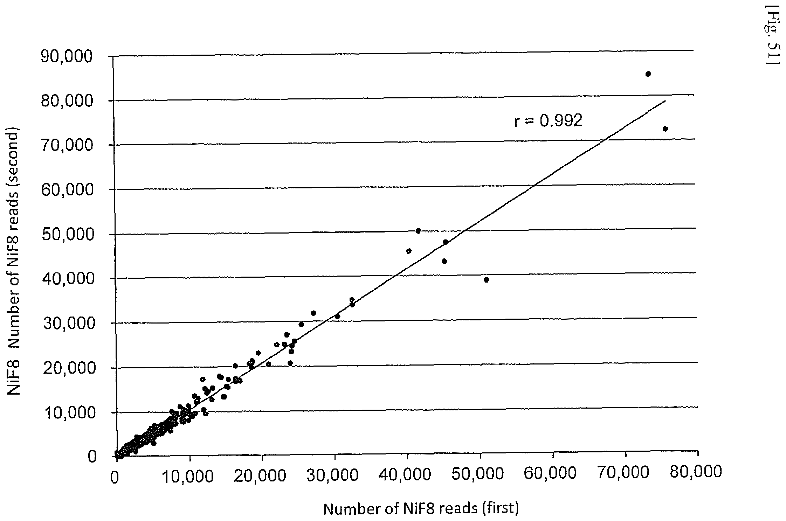

[fig.51]Fig. 51 shows a characteristic diagram demonstrating the results of MiSeq analysis of a DNA library amplified using DNA of the rice variety Nipponbare as a template and a random primer.

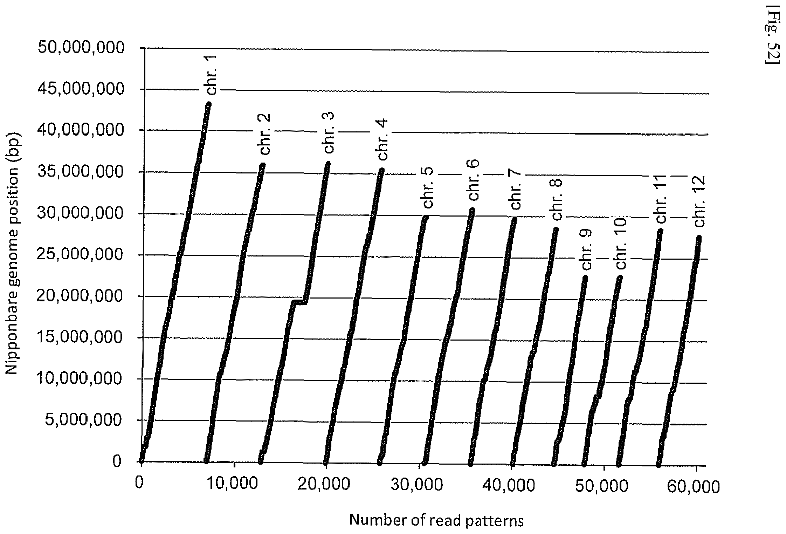

[fig.52]Fig. 52 shows a characteristic diagram demonstrating positions of MiSeq read patterns in the genome information of the rice variety Nipponbare.

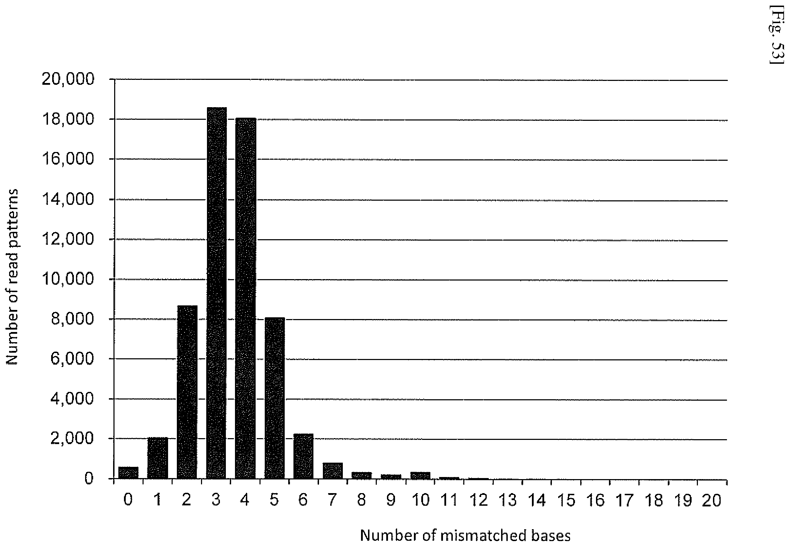

[fig.53]Fig. 53 shows a characteristic diagram demonstrating the frequency distribution of the number of mismatched nucleotides between the random primer and the rice genome.

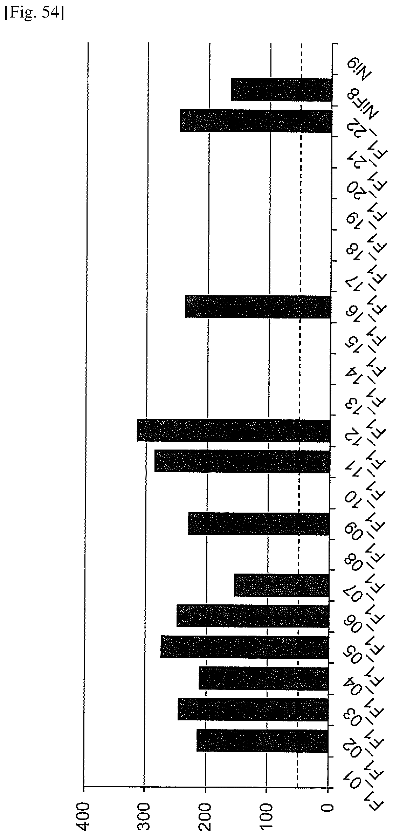

[fig.54]Fig. 54 shows a characteristic diagram demonstrating the number of reads of the sugarcane varieties NiF8 and Ni9 and hybrid progeny lines thereof at the marker N80521152.

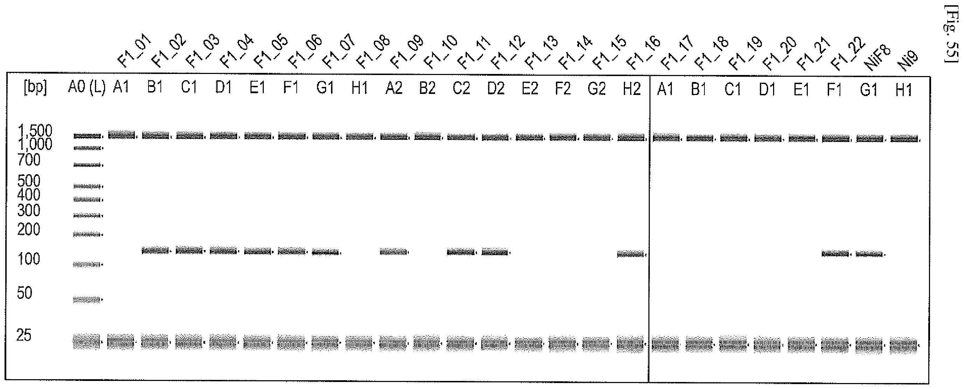

[fig.55]Fig. 55 shows a photograph demonstrating electrophoretic patterns of the sugarcane varieties NiF8 and Ni9 and hybrid progeny lines thereof at the PCR marker N80521152.

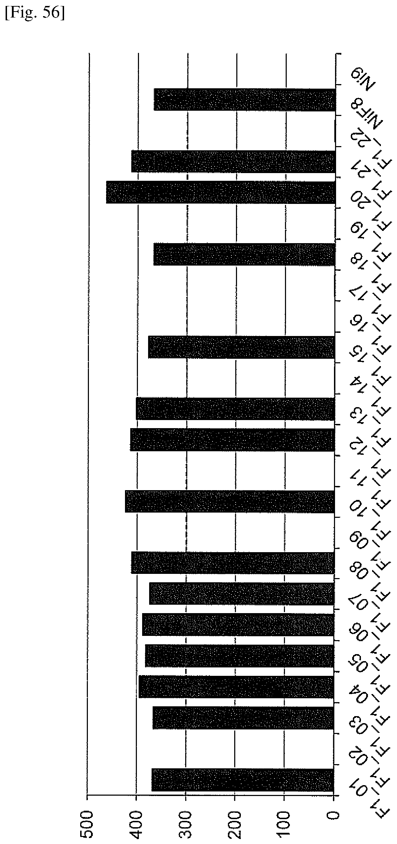

[fig.56]Fig. 56 shows a characteristic diagram demonstrating the number of reads of the sugarcane varieties NiF8 and Ni9 and hybrid progeny lines thereof at the marker N80997192.

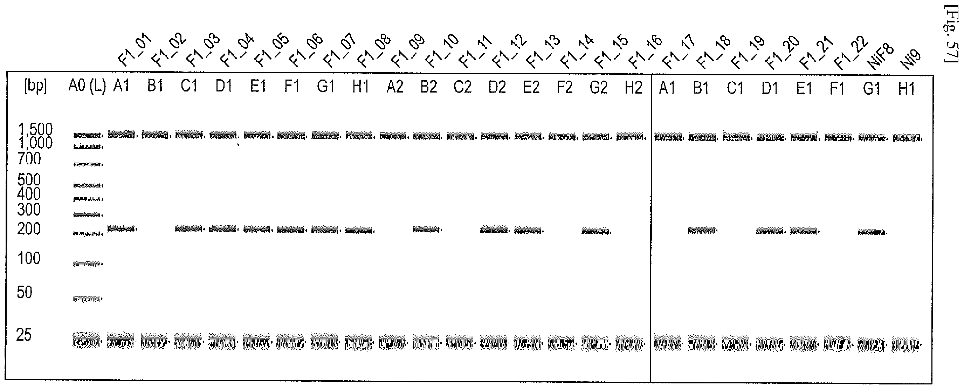

[fig.57]Fig. 57 shows a photograph demonstrating electrophoretic patterns of the sugarcane varieties NiF8 and Ni9 and hybrid progeny lines thereof at the PCR marker N80997192.

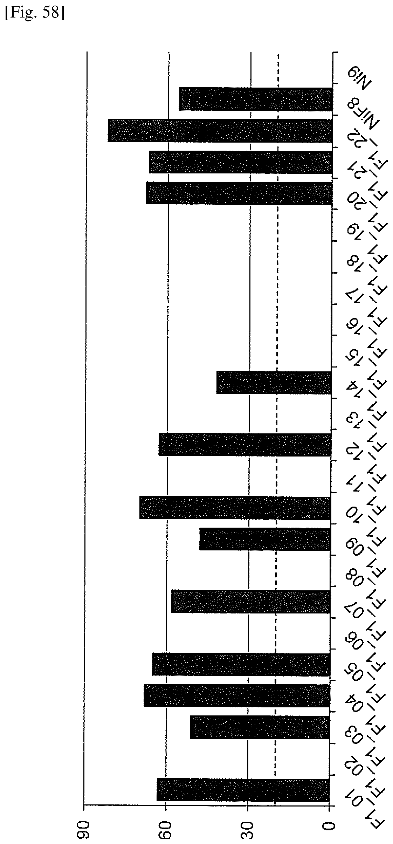

[fig.58]Fig. 58 shows a characteristic diagram demonstrating the number of reads of the sugarcane varieties NiF8 and Ni9 and hybrid progeny lines thereof at the marker N80533142.

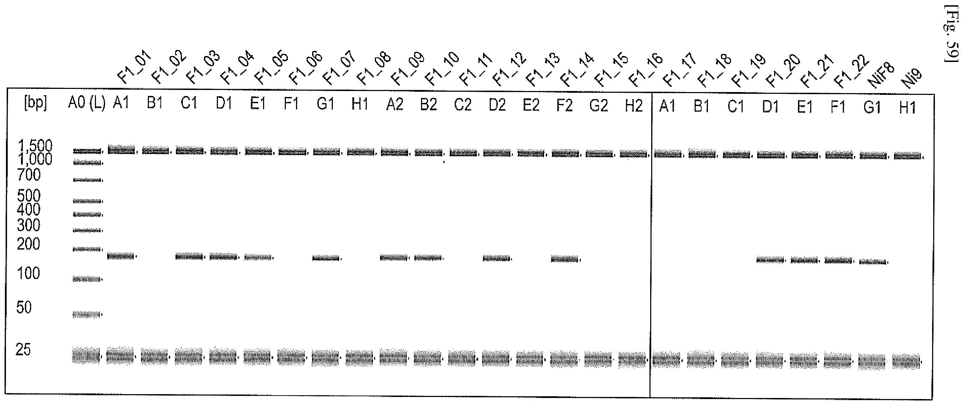

[fig.59]Fig. 59 shows a photograph demonstrating electrophoretic patterns of the sugarcane varieties NiF8 and Ni9 and hybrid progeny lines thereof at the PCR marker N80533142.

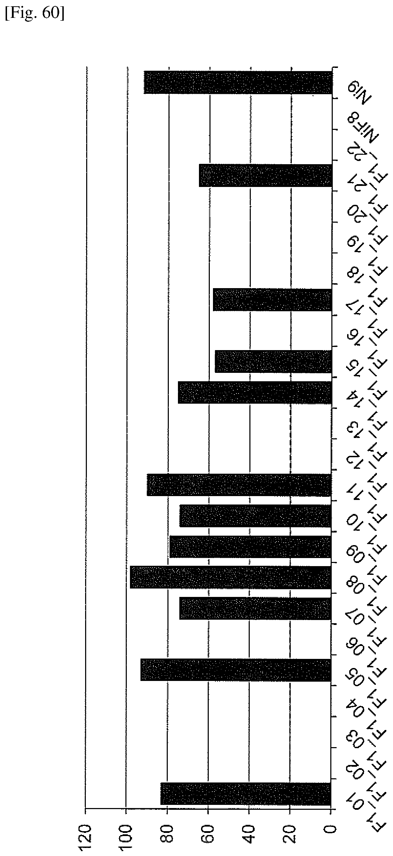

[fig.60]Fig. 60 shows a characteristic diagram demonstrating the number of reads of the sugarcane varieties NiF8 and Ni9 and hybrid progeny lines thereof at the marker N91552391.

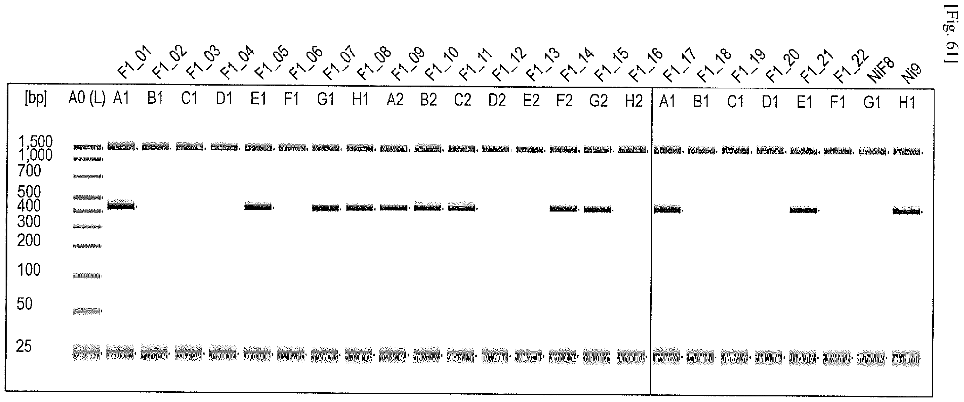

[fig.61]Fig. 61 shows a photograph demonstrating electrophoretic patterns of the sugarcane varieties NiF8 and Ni9 and hybrid progeny lines thereof at the PCR marker N91552391.

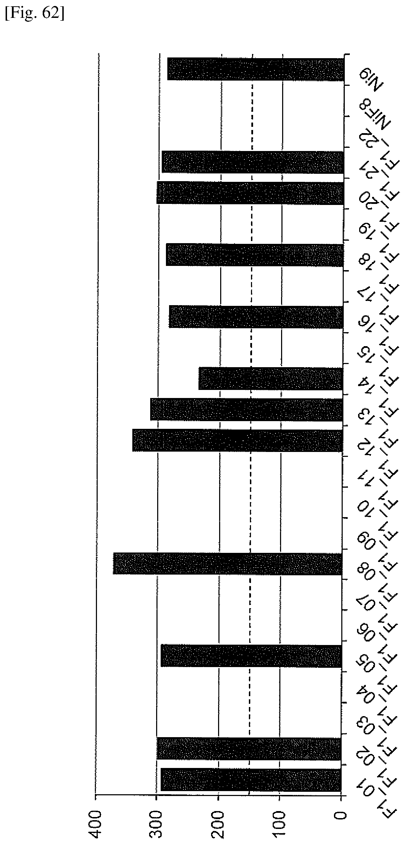

[fig.62]Fig. 62 shows a characteristic diagram demonstrating the number of reads of the sugarcane varieties NiF8 and Ni9 and hybrid progeny lines thereof at the marker N91653962.

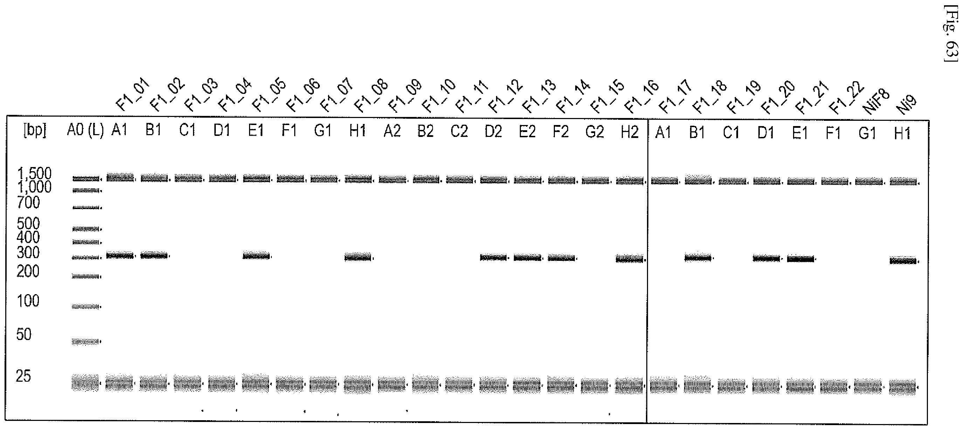

[fig.63]Fig. 63 shows a photograph demonstrating electrophoretic patterns of the sugarcane varieties NiF8 and Ni9 and hybrid progeny lines thereof at the PCR marker N91653962.

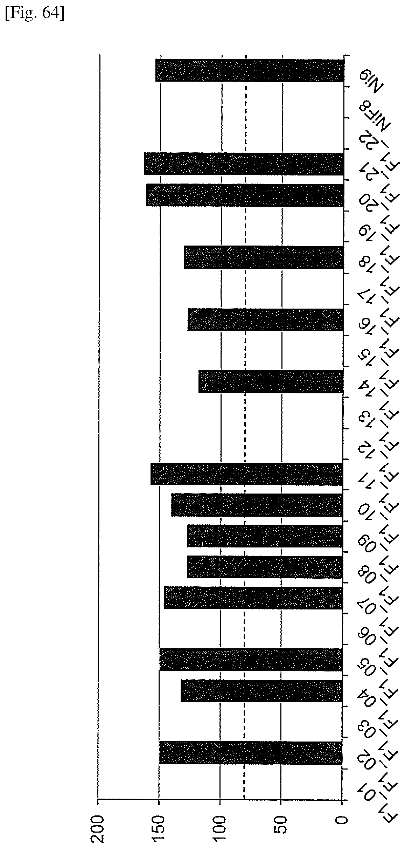

[fig.64]Fig. 64 shows a characteristic diagram demonstrating the number of reads of the sugarcane varieties NiF8 and Ni9 and hybrid progeny lines thereof at the marker N91124801.

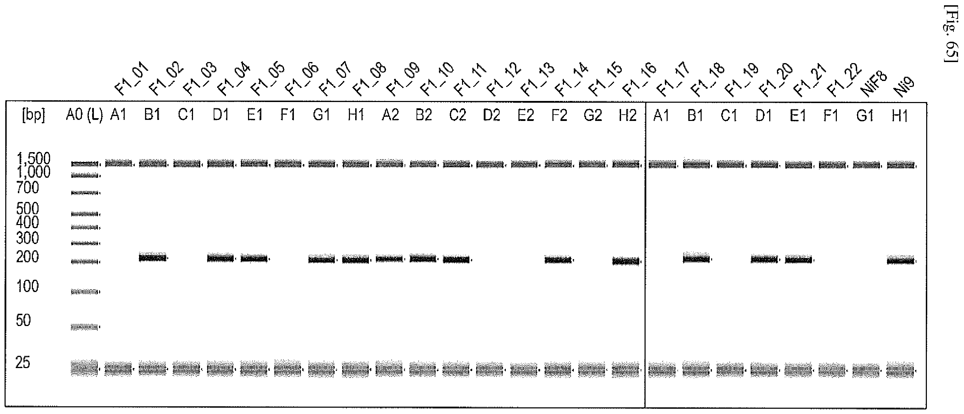

[fig.65]Fig. 65 shows a photograph demonstrating electrophoretic patterns of the sugarcane varieties NiF8 and Ni9 and hybrid progeny lines thereof at the PCR marker N91124801.

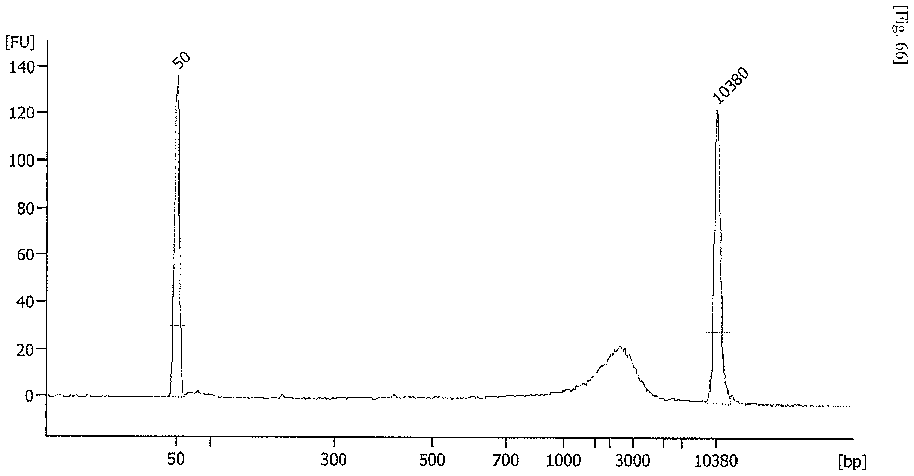

[fig.66]Fig. 66 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 9 bases.

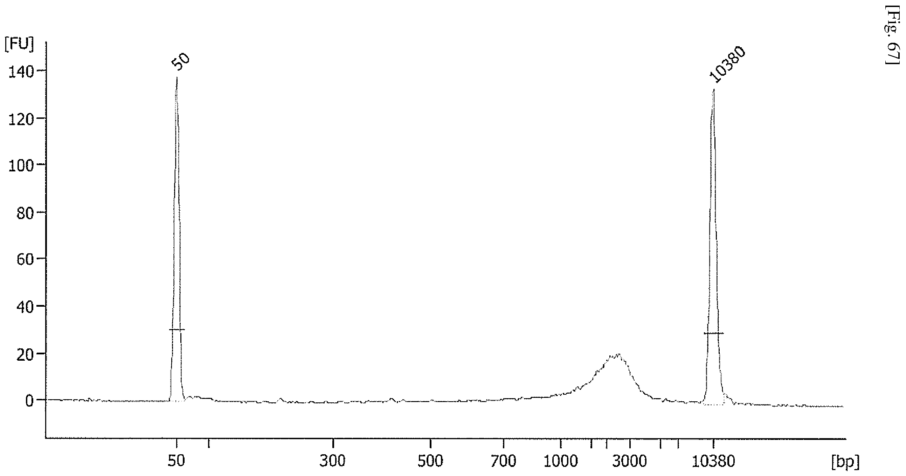

[fig.67]Fig. 67 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 9 bases.

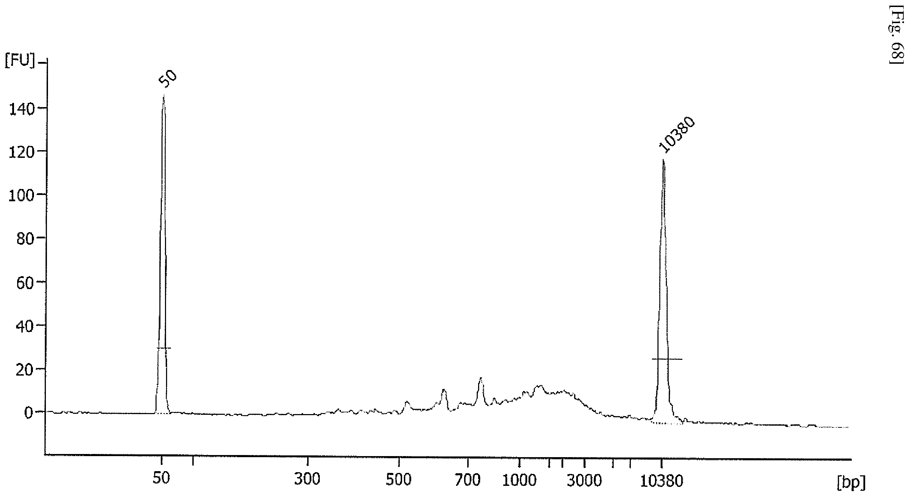

[fig.68]Fig. 68 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 10 bases.

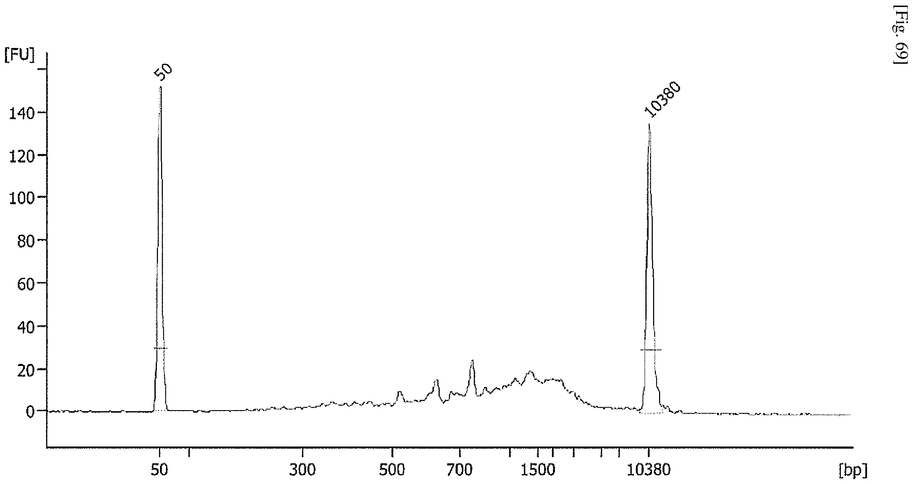

[fig.69]Fig. 69 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 10 bases.

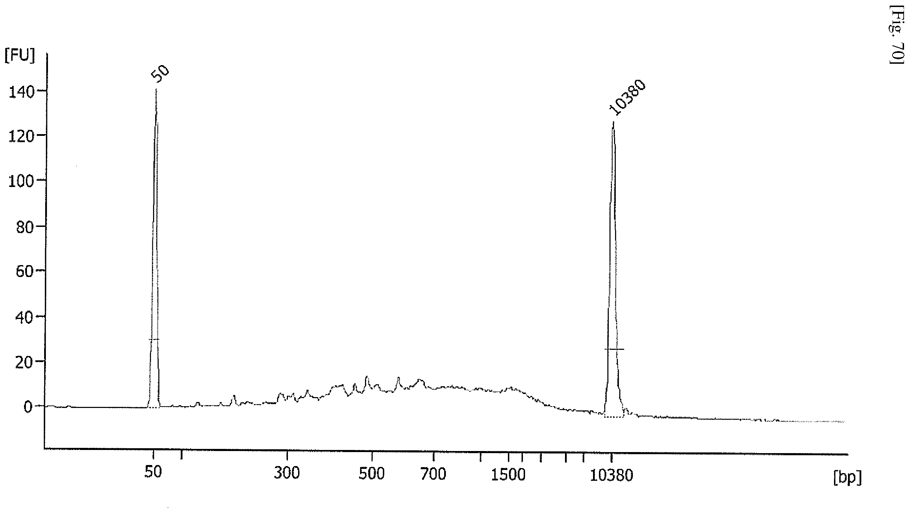

[fig.70]Fig. 70 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 11 bases.

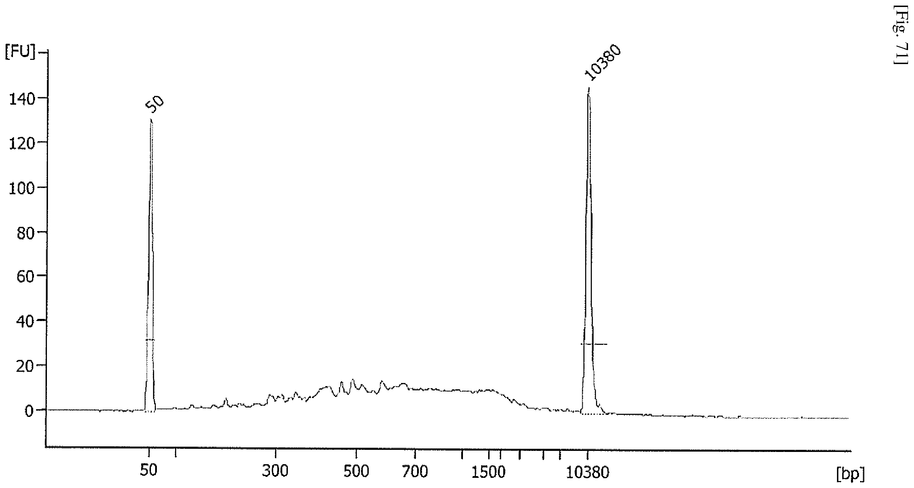

[fig.71]Fig. 71 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 11 bases.

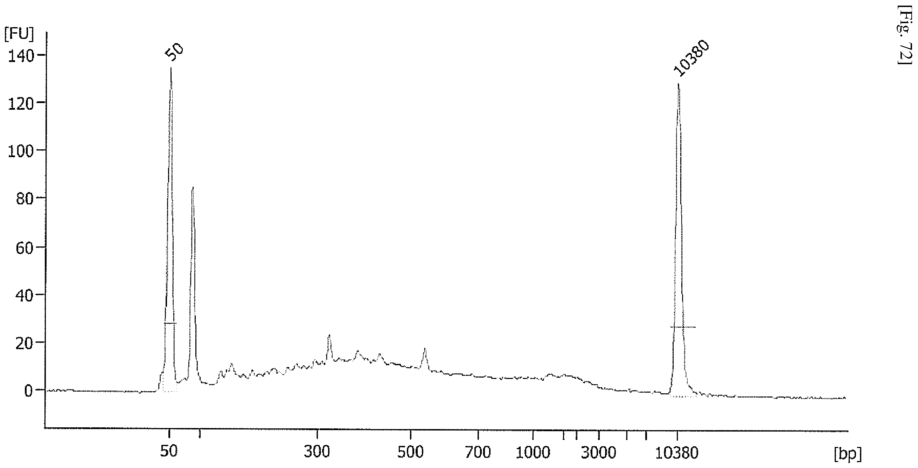

[fig.72]Fig. 72 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 12 bases.

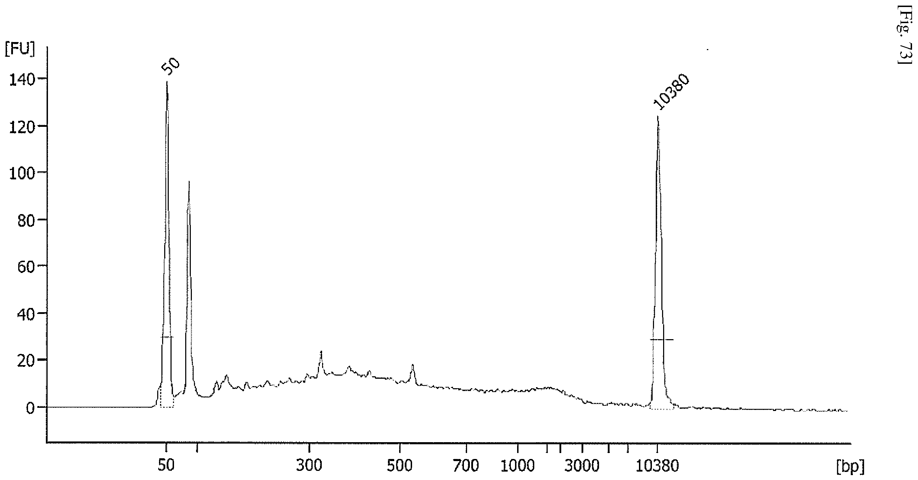

[fig.73]Fig. 73 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 12 bases.

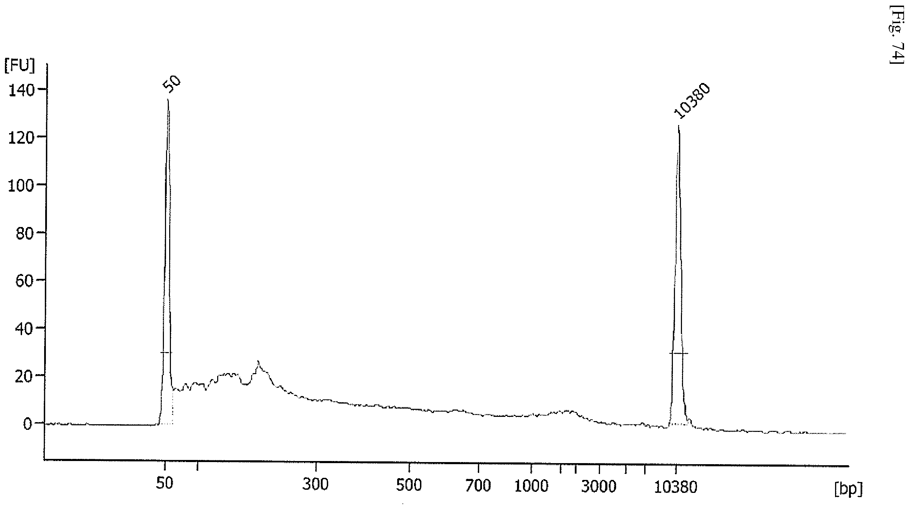

[fig.74]Fig. 74 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 14 bases.

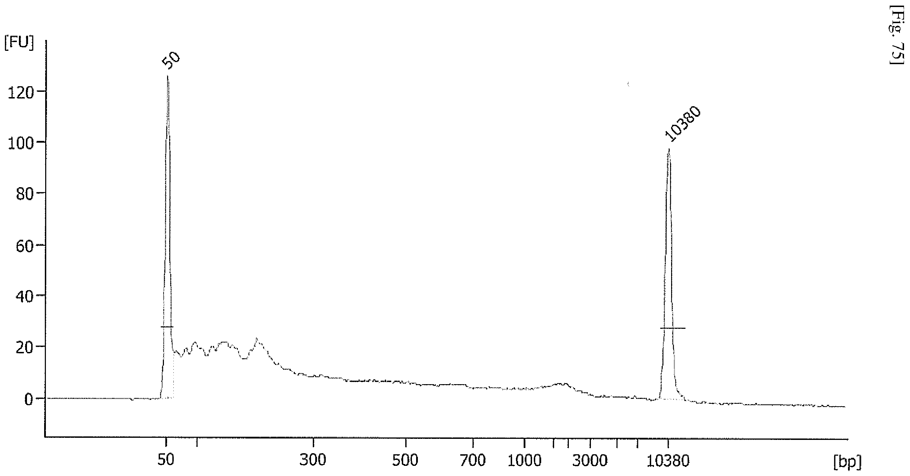

[fig.75]Fig. 75 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 14 bases.

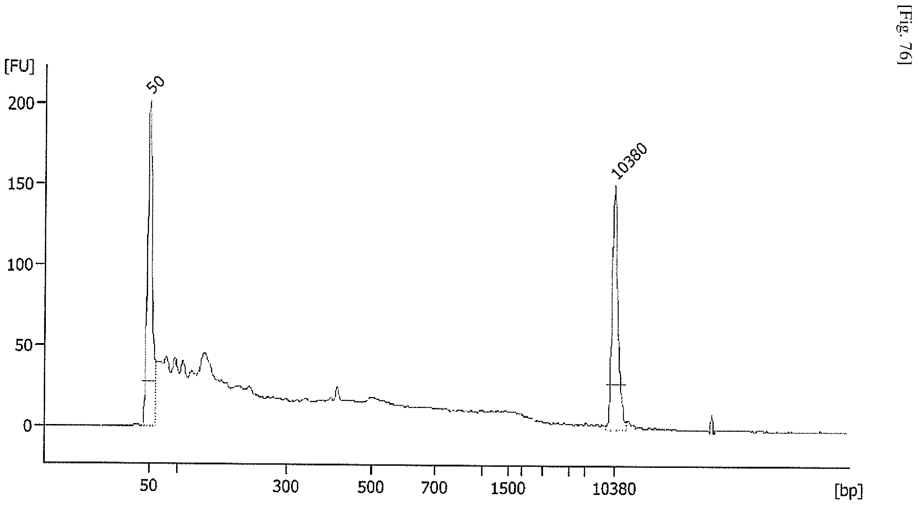

[fig.76]Fig. 76 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 16 bases.

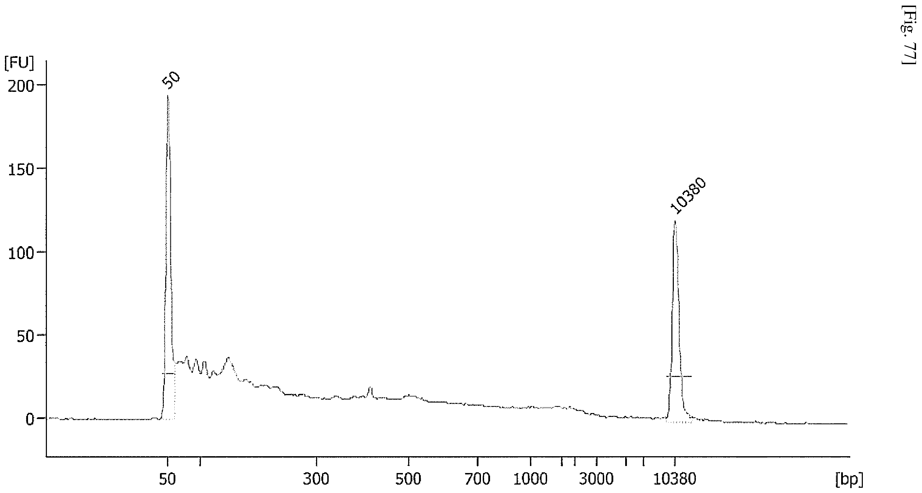

[fig.77]Fig. 77 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 16 bases.

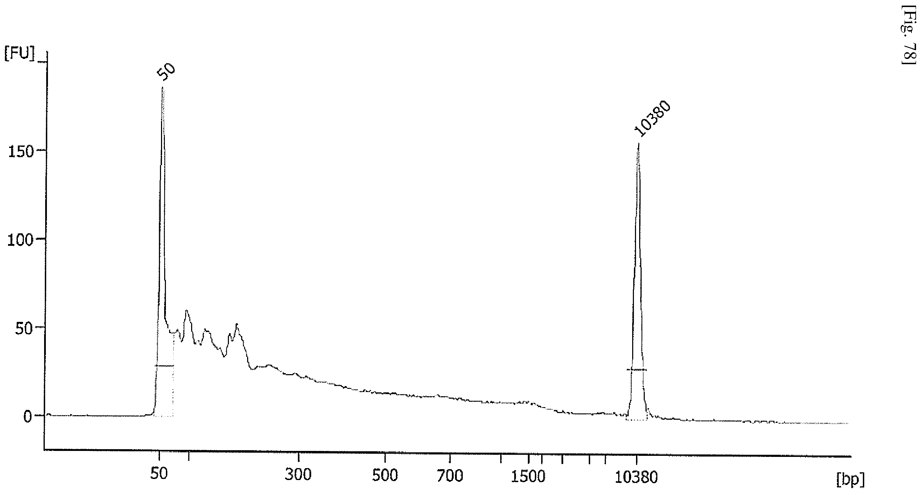

[fig.78]Fig. 78 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 18 bases.

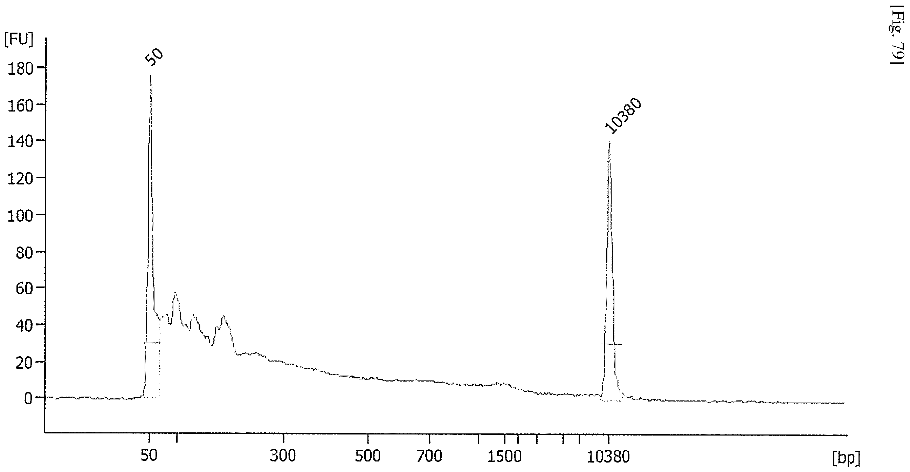

[fig.79]Fig. 79 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 18 bases.

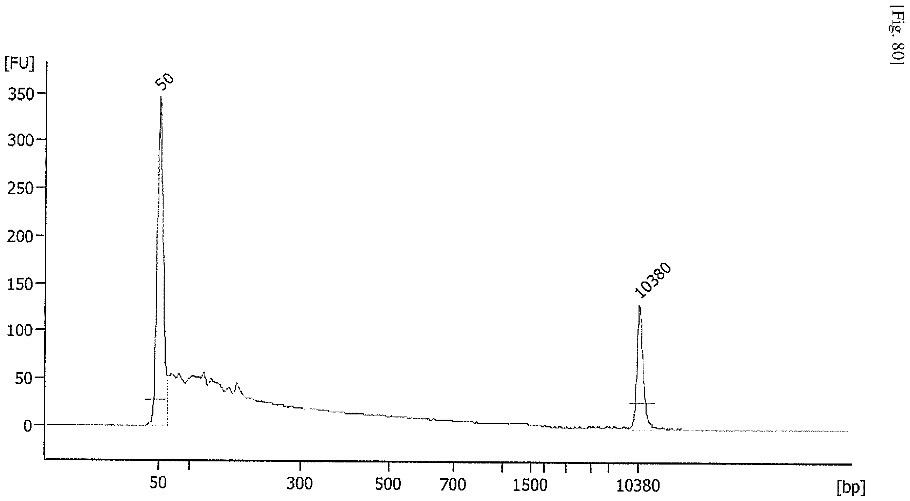

[fig.80]Fig. 80 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer comprising 20 bases.

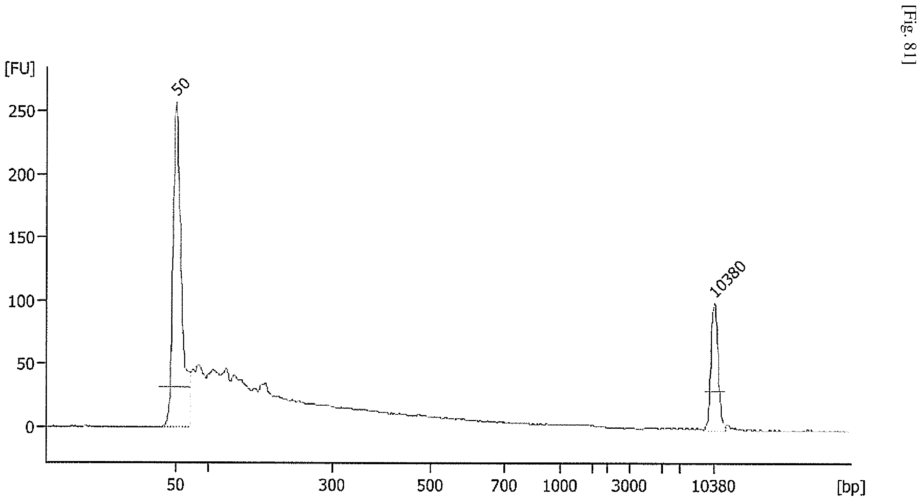

[fig.81]Fig. 81 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer comprising 20 bases.

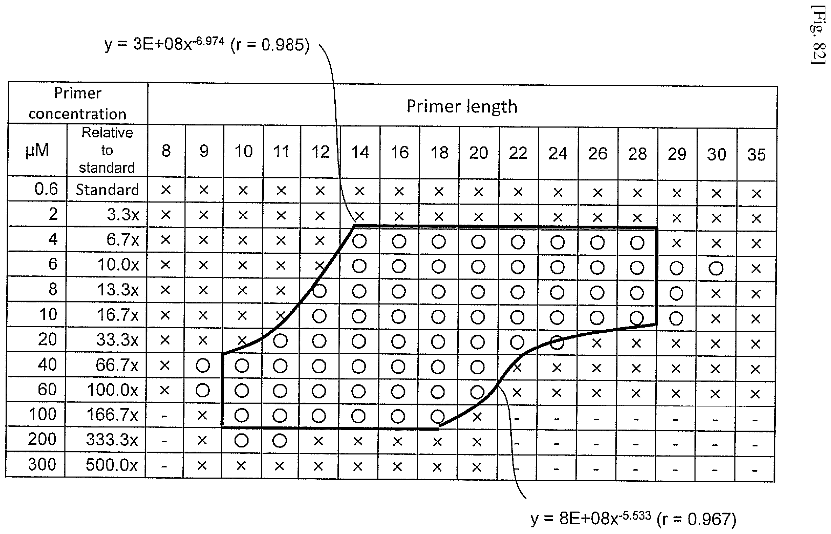

[fig.82]Fig. 82 shows a characteristic diagram demonstrating the results of investigating the reproducibility of the DNA library amplified using DNA of the sugarcane variety NiF8 as a template and random primers each comprising 8 to 35 bases used at a concentration of 0.6 to 300 microM.

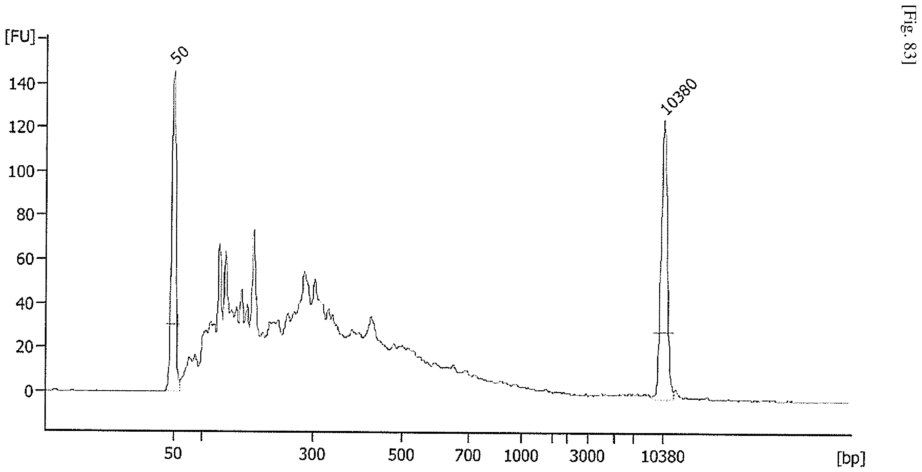

[fig.83]Fig. 83 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a single type of random primer.

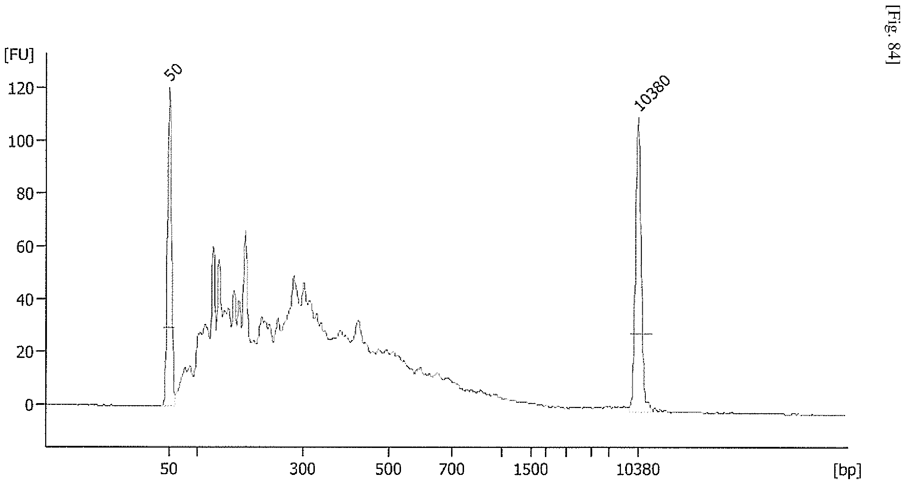

[fig.84]Fig. 84 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a single type of random primer.

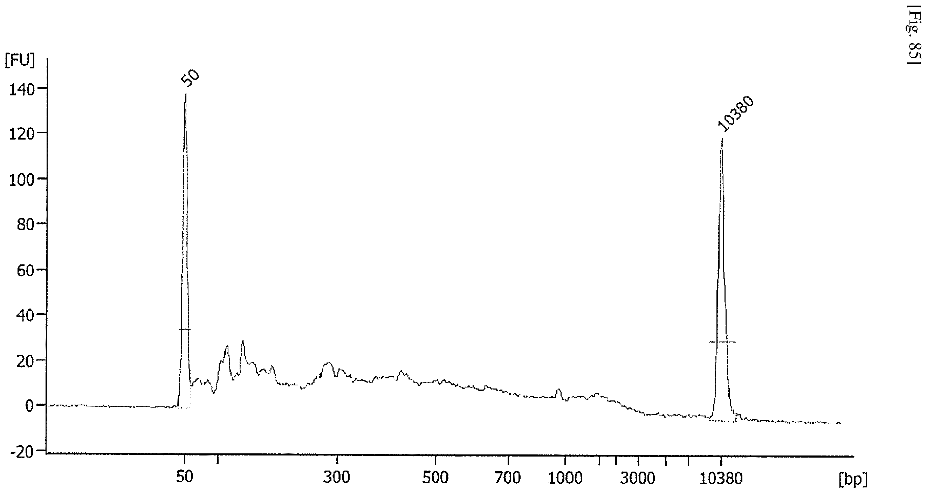

[fig.85]Fig. 85 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 2 types of random primers.

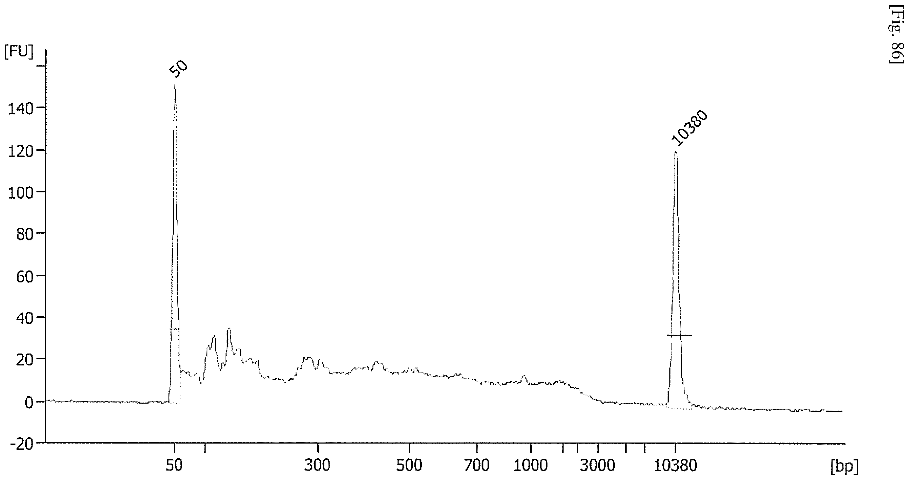

[fig.86]Fig. 86 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 2 types of random primers.

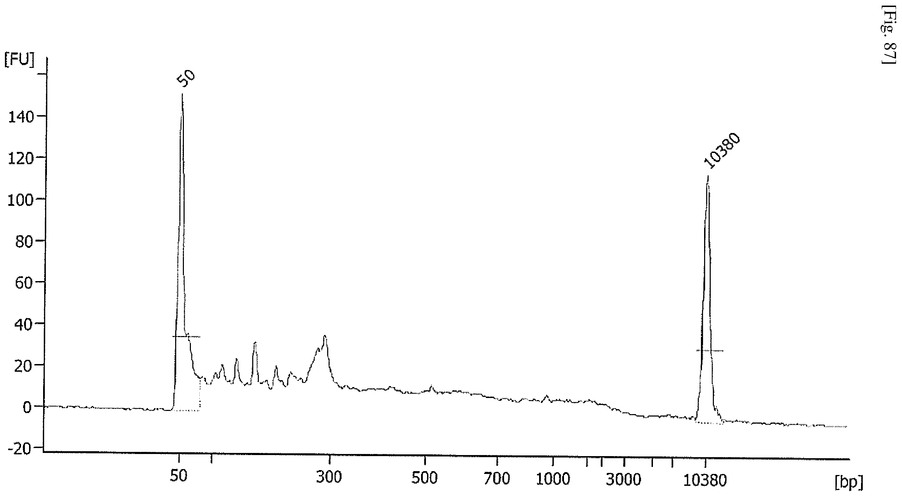

[fig.87]Fig. 87 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 3 types of random primers.

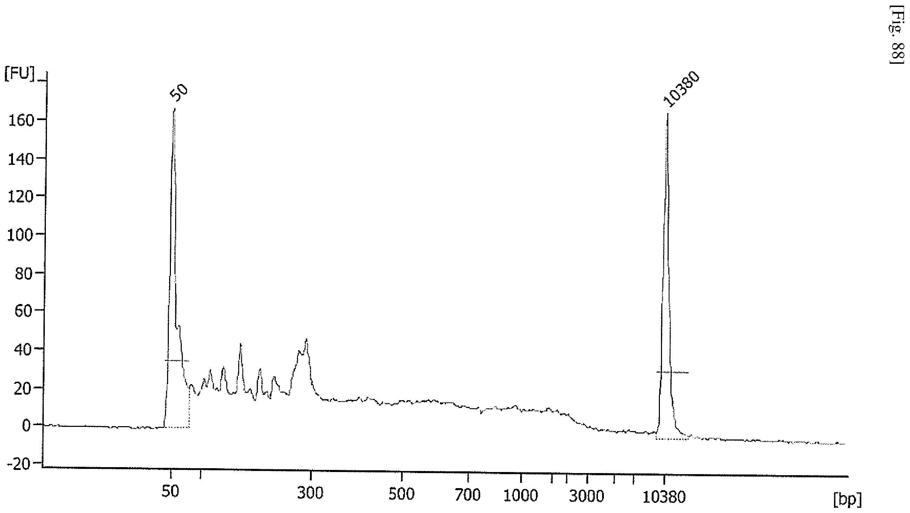

[fig.88]Fig. 88 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 3 types of random primers.

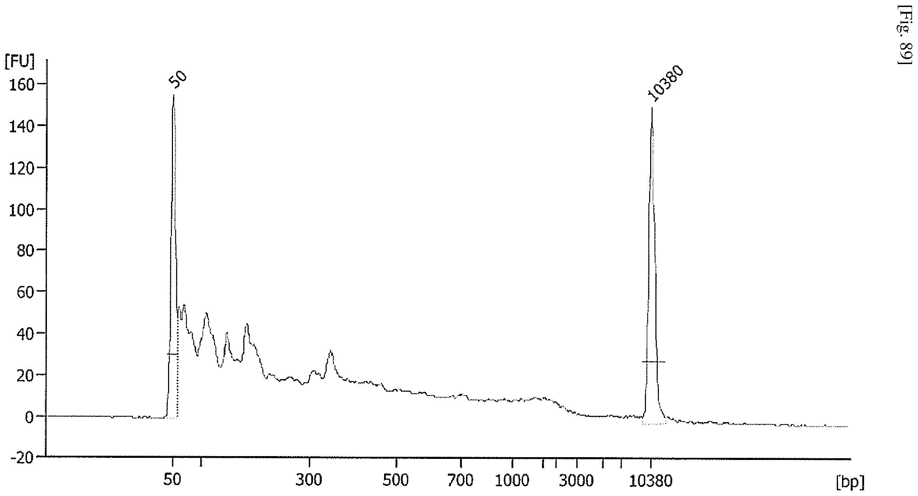

[fig.89]Fig. 89 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 12 types of random primers.

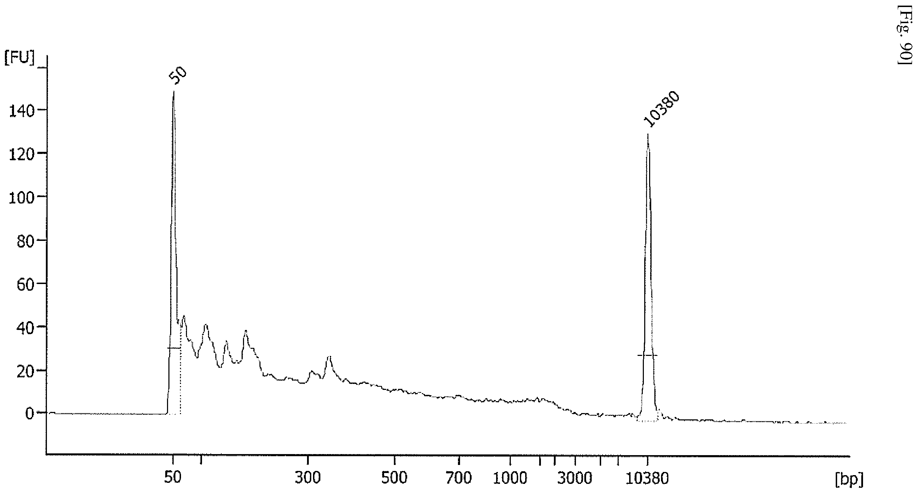

[fig.90]Fig. 90 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 12 types of random primers.

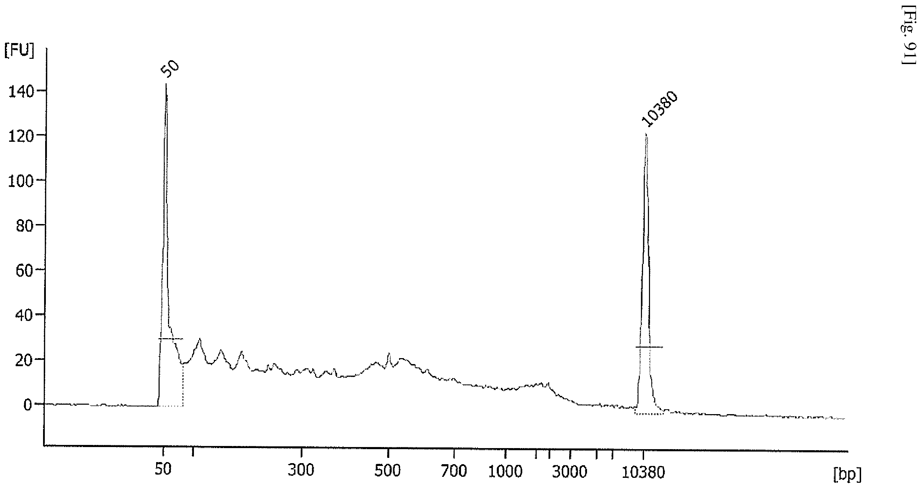

[fig.91]Fig. 91 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 24 types of random primers.

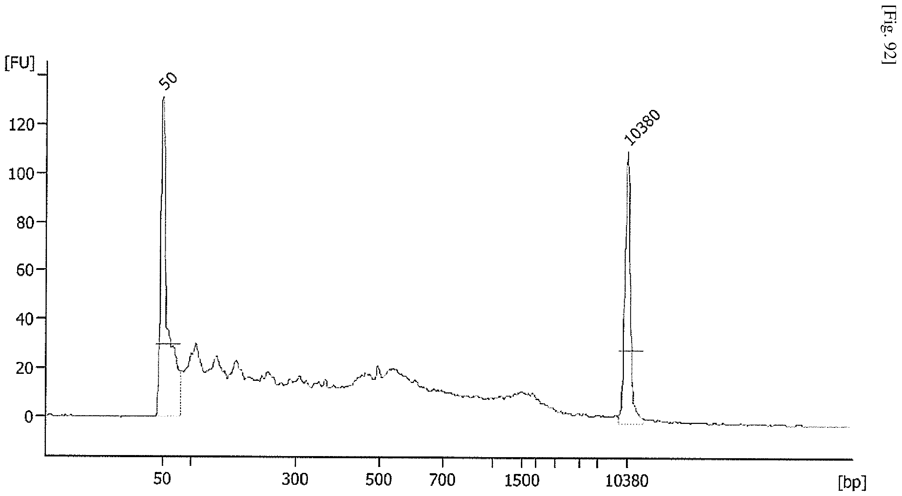

[fig.92]Fig. 92 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 24 types of random primers.

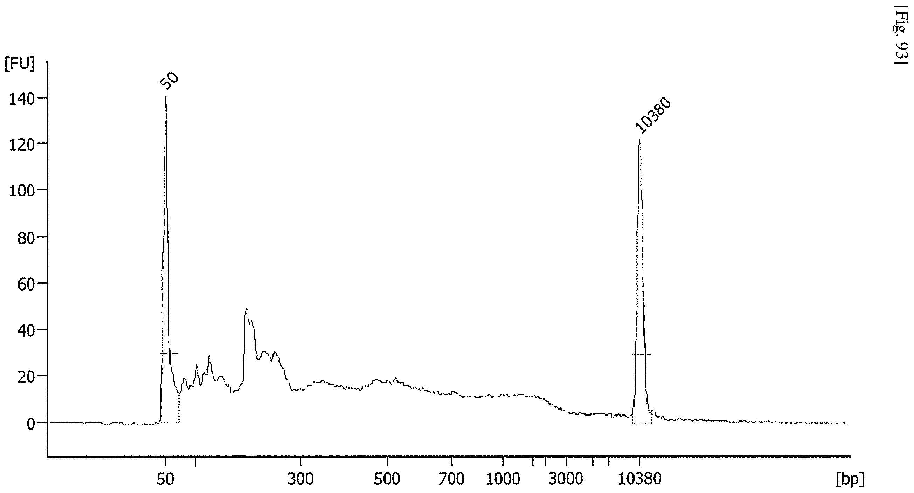

[fig.93]Fig. 93 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and 48 types of random primers.

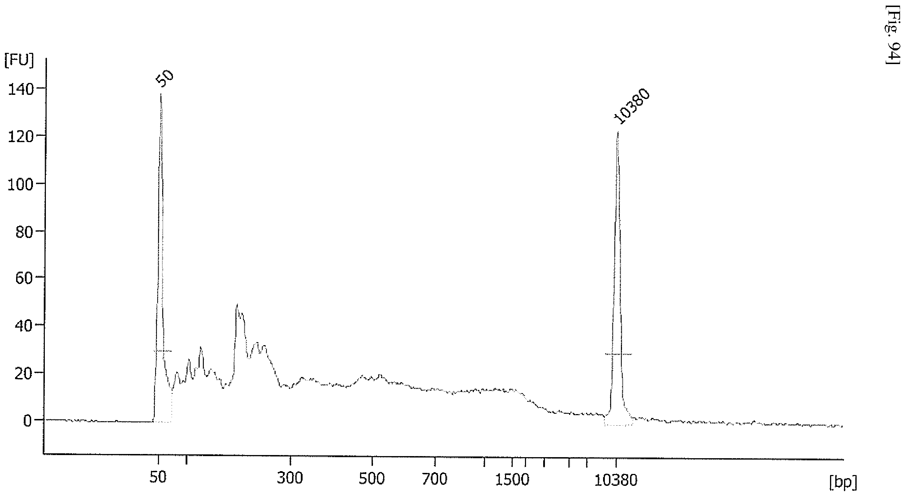

[fig.94]Fig. 94 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and 48 types of random primers.

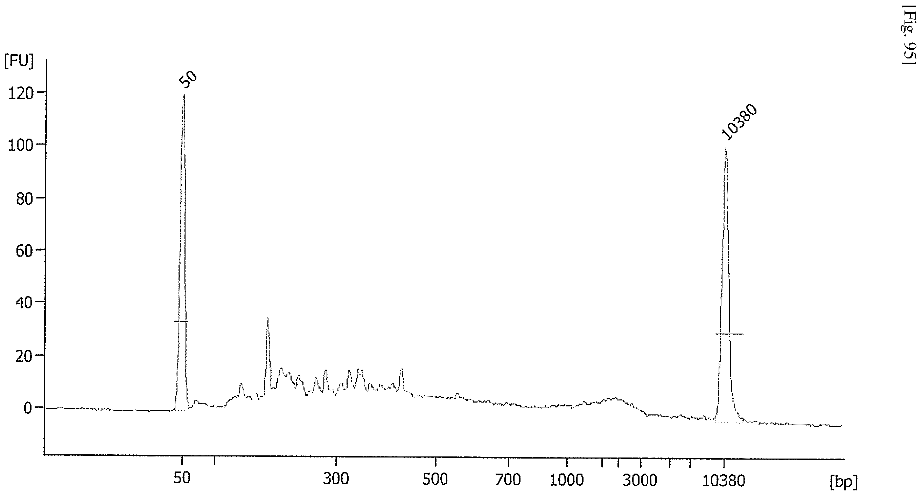

[fig.95]Fig. 95 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer B comprising 10 nucleotides.

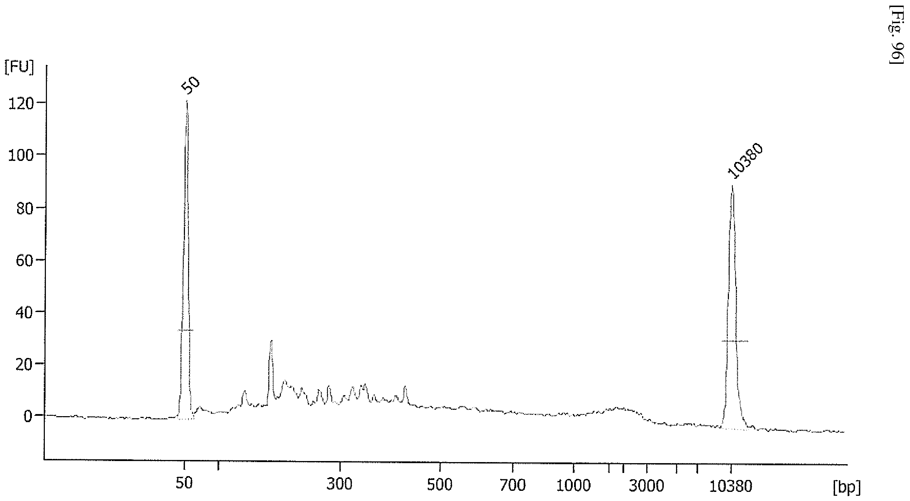

[fig.96]Fig. 96 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer B comprising 10 nucleotides.

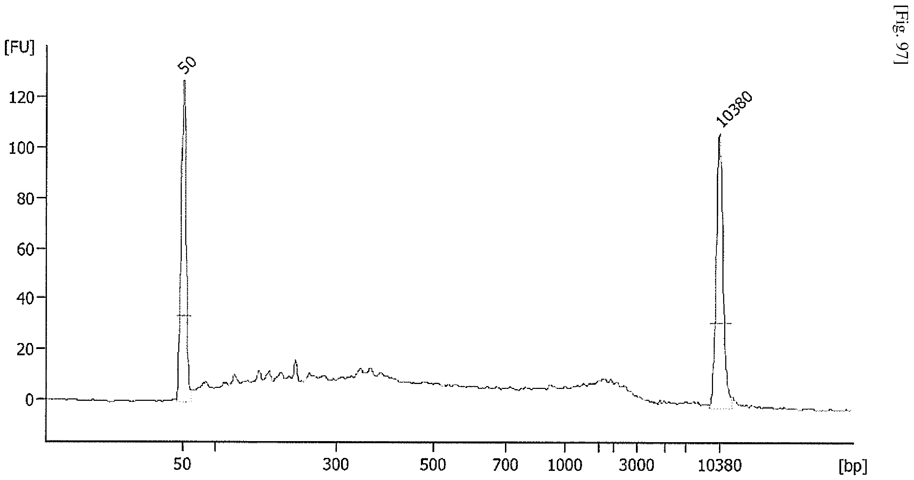

[fig.97]Fig. 97 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer C comprising 10 nucleotides.

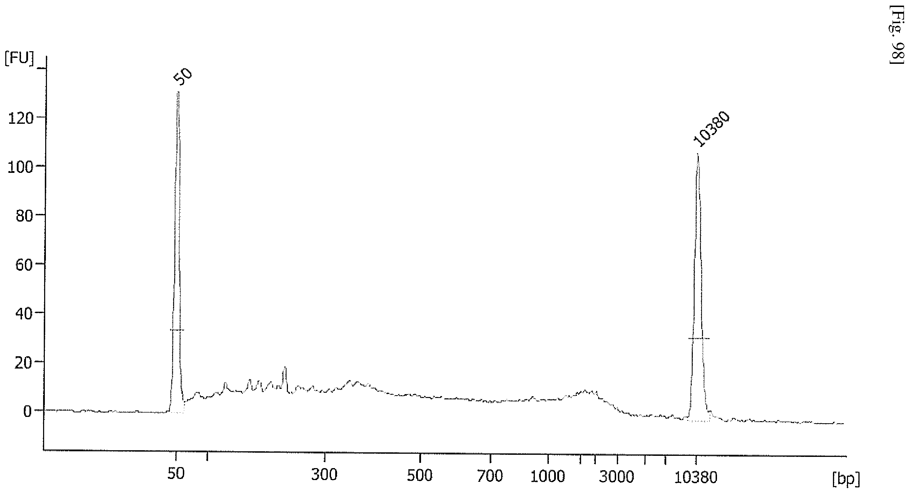

[fig.98]Fig. 98 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer C comprising 10 nucleotides.

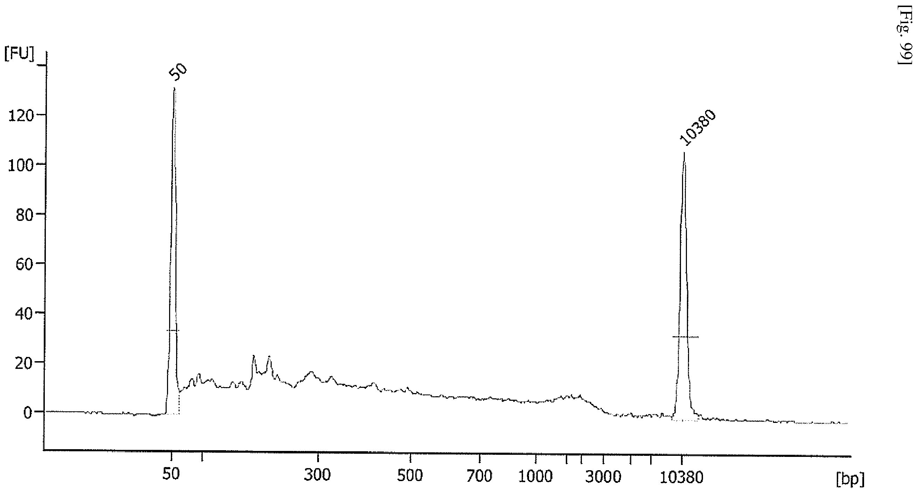

[fig.99]Fig. 99 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer D comprising 10 nucleotides.

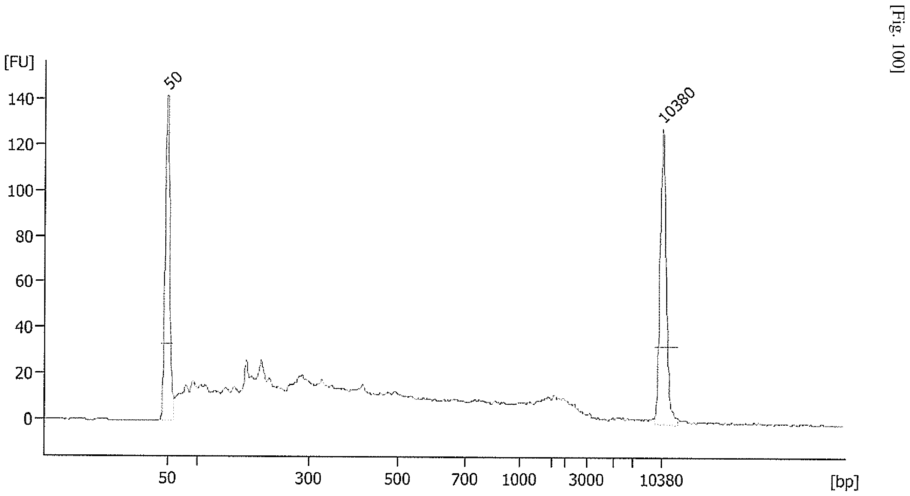

[fig.100]Fig. 100 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer D comprising 10 nucleotides.

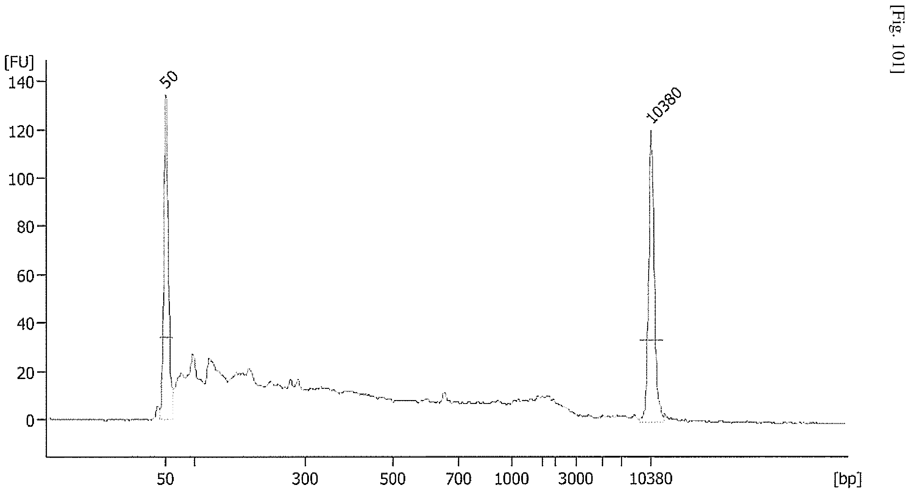

[fig.101]Fig. 101 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer E comprising 10 nucleotides.

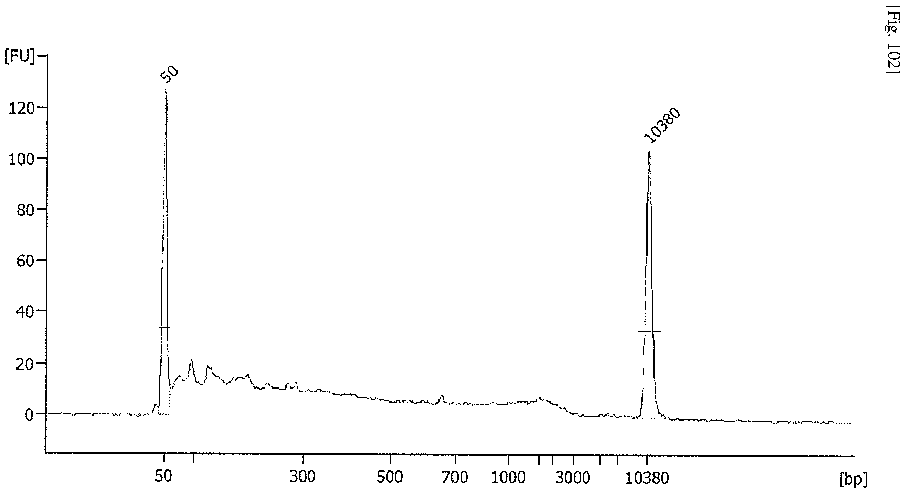

[fig.102]Fig. 102 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer E comprising 10 nucleotides.

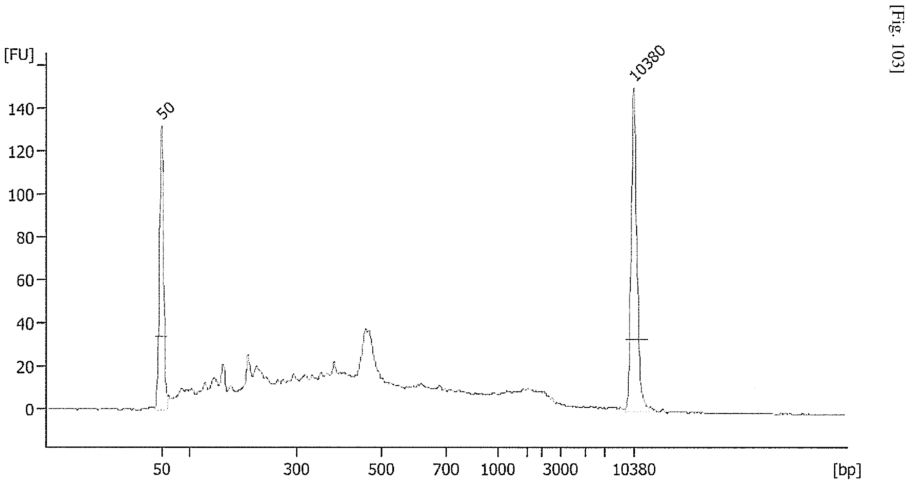

[fig.103]Fig. 103 shows a characteristic diagram demonstrating a correlation between an amplified fragment length and a fluorescence unit (FU) in which the amplified fragment length is determined based on an electrophoretic pattern (appeared for the first time) of the DNA library amplified using DNA of the sugarcane variety NiF8 as a template and a random primer F comprising 10 nucleotides.

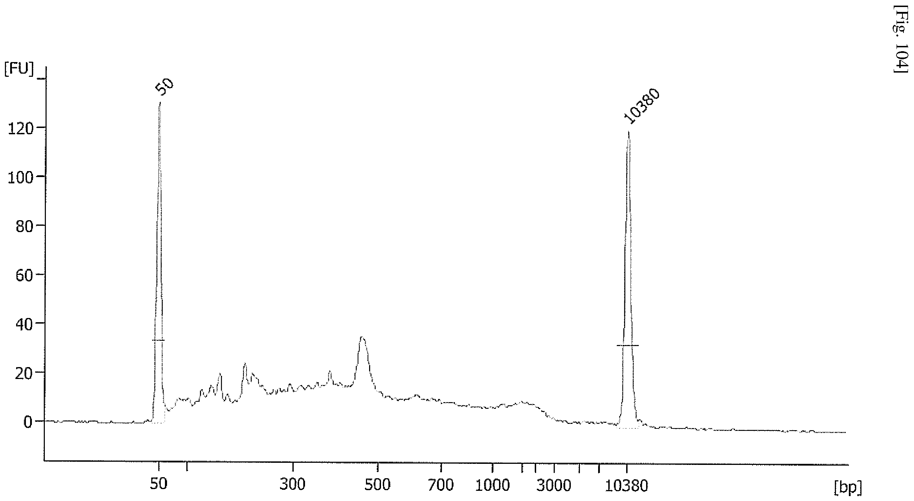

[fig.104]Fig. 104 shows a characteristic diagram demonstrating a correlation between an amplified fragment length and a fluorescence unit (FU) in which the amplified fragment length is determined based on an electrophoretic pattern (appeared for the second time) of the DNA library amplified using DNA of the sugarcane variety NiF8 as a template and a random primer F comprising 10 nucleotides.

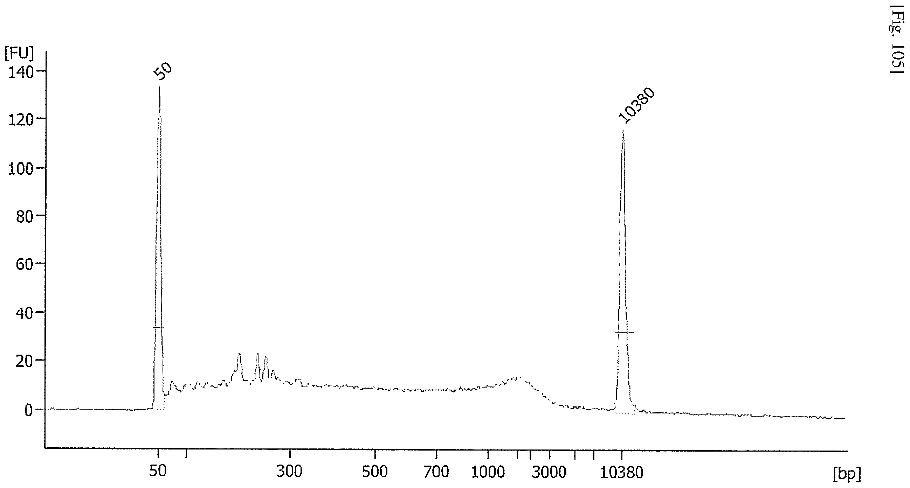

[fig.105]Fig. 105 shows a characteristic diagram demonstrating a correlation between an amplified fragment length and a fluorescence unit (FU) in which the amplified fragment length is determined based on an electrophoretic pattern (appeared for the first time) of the DNA library amplified using human genomic DNA as a template and a random primer A comprising 10 nucleotides.

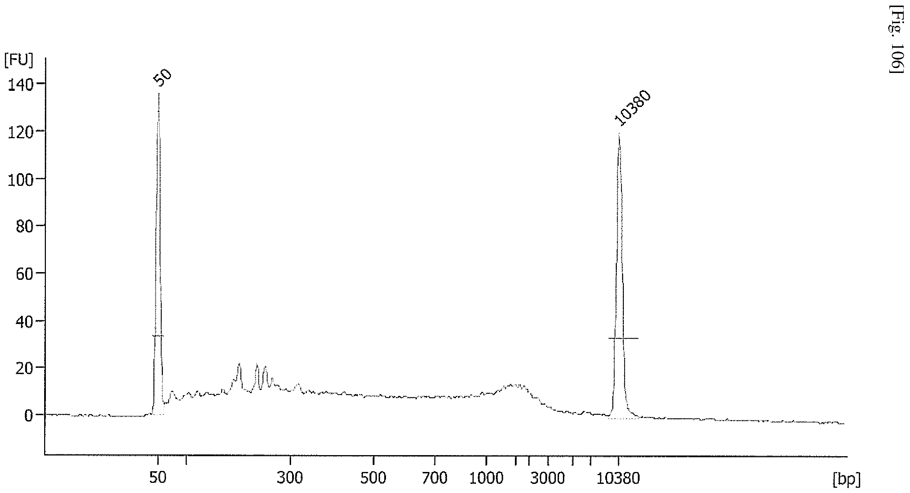

[fig. 106]Fig. 106 shows a characteristic diagram demonstrating a correlation between an amplified fragment length and a fluorescence unit (FU) in which the amplified fragment length is determined based on an electrophoretic pattern (appeared for the second time) of the DNA library amplified using human genomic DNA as a template and a random primer A comprising 10 nucleotides.

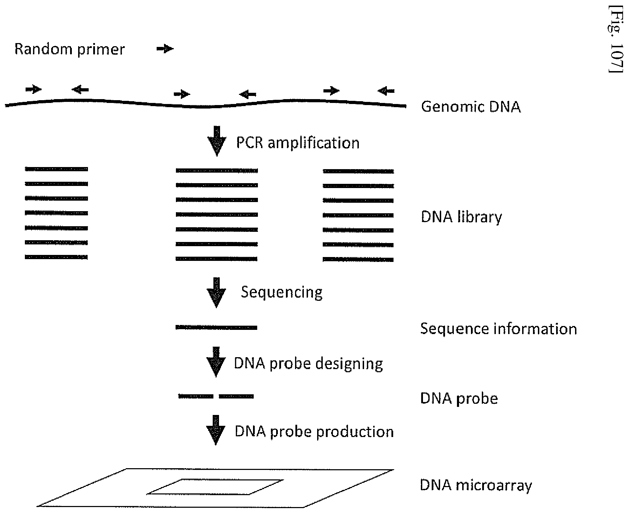

[fig. 107]Fig. 107 shows a flow chart demonstrating a process for producing a DNA microarray with the application of the method for producing a DNA probe according to the present invention.

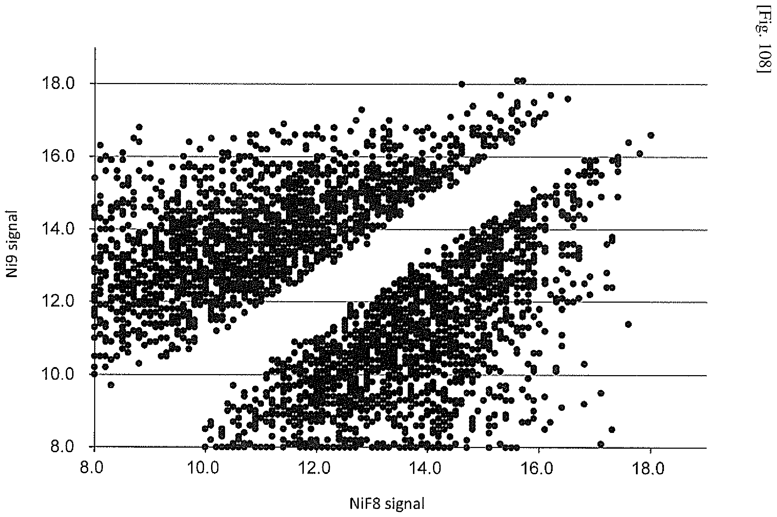

[fig.108]Fig. 108 shows a characteristic diagram demonstrating the results of assaying signals obtained from a DNA probe concerning the DNA library amplified using genomic DNAs of NiF8 and Ni9 as templates and a random primer at a high concentration.

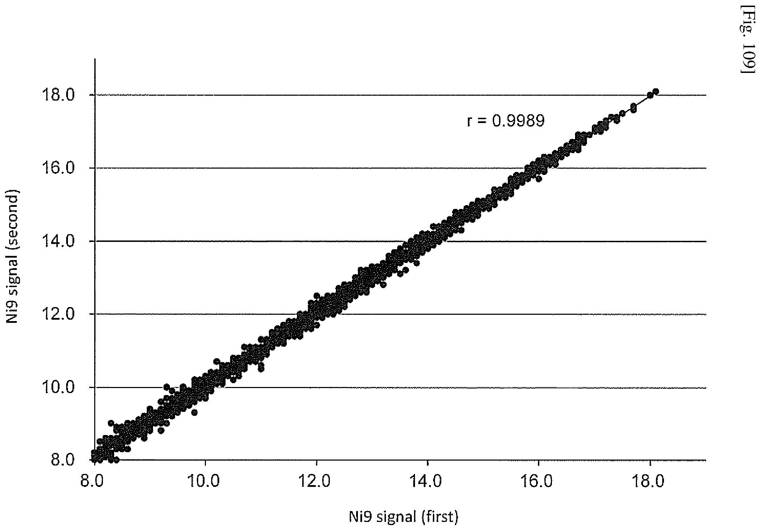

[fig.109]Fig. 109 shows a characteristic diagram demonstrating the results of comparison of signals obtained through repeated measurements concerning the DNA library amplified using genomic DNA of Ni9 as a template and a random primer at a high concentration.

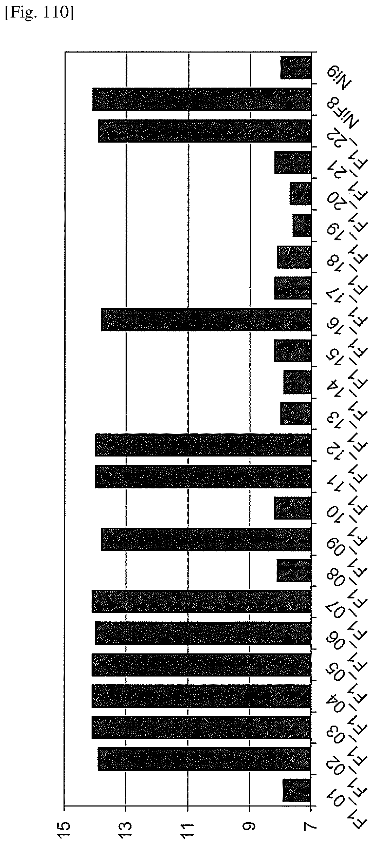

[fig. 110]Fig. 110 shows a characteristic diagram demonstrating the results of assaying signal levels obtained from the DNA probe reacting with the marker N80521152.

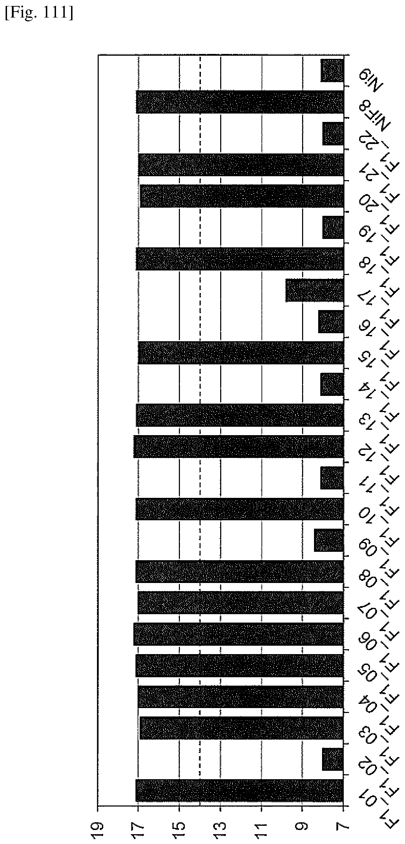

[fig.111]Fig. 111 shows a characteristic diagram demonstrating the results of assaying signal levels obtained from the DNA probe reacting with the marker N80997192.

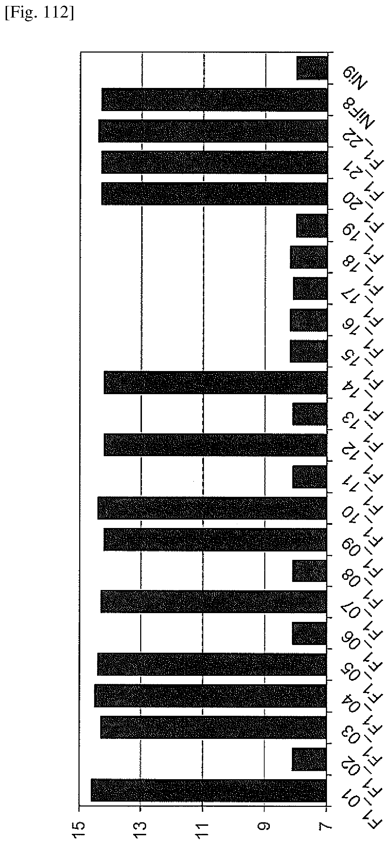

[fig.112]Fig. 112 shows a characteristic diagram demonstrating the results of assaying signal levels obtained from the DNA probe reacting with the marker N80533142.

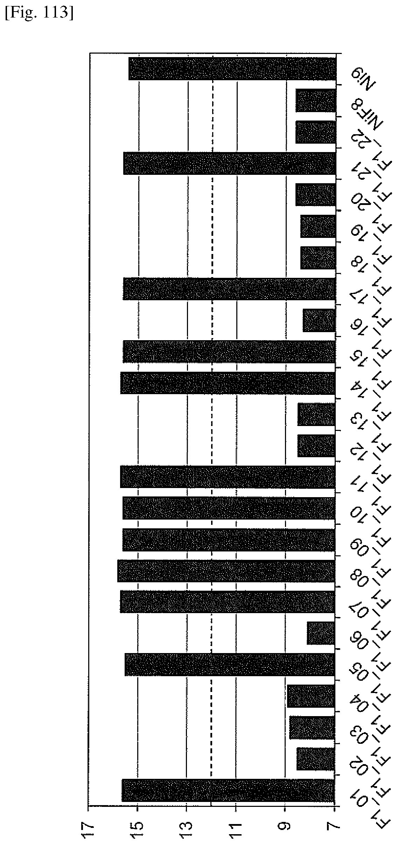

[fig.113]Fig. 113 shows a characteristic diagram demonstrating the results of assaying signal levels obtained from the DNA probe reacting with the marker N91552391.

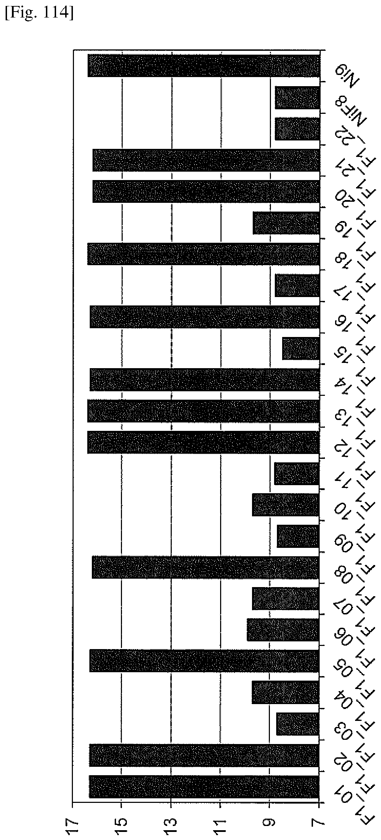

[fig.114]Fig. 114 shows a characteristic diagram demonstrating the results of assaying signal levels obtained from the DNA probe reacting with the marker N91653962.

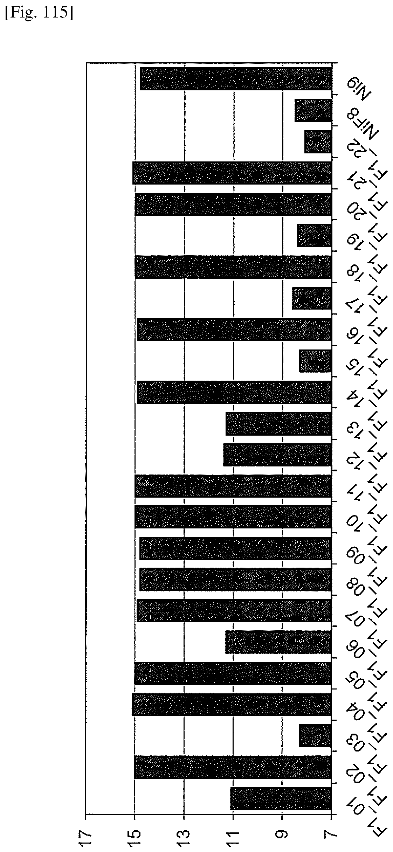

[fig.115]Fig. 115 shows a characteristic diagram demonstrating the results of assaying signal levels obtained from the DNA probe reacting with the marker N91124801.

### Description of Embodiments

Hereafter, the present invention is described in detail.

According to the method for producing a DNA probe of the present invention, a nucleic acid amplification reaction is carried out in a reaction solution, which is prepared to contain a primer having an arbitrary nucleotide sequence (hereafter, referred to as a "random primer")at a high concentration, and a nucleotide sequence of a DNA probe used for detecting an amplified nucleic acid fragment (i.e., a DNA fragment) is designed based on the nucleotide sequence of such DNA fragment. By conducting a nucleic acid amplification reaction in a reaction solution containing a random primer at a high concentration, a DNA fragment of interest can be amplified with excellent reproducibility. Hereafter, the obtained DNA fragment is referred to as a "DNA library."

When a reaction solution contains a random primer at a high concentration, such concentration is higher than the concentration of a primer used in a general nucleic acid amplification reaction. When producing a DNA library, specifically, a random primer is used at a higher concentration than a primer used in a general nucleic acid amplification reaction. As a template contained in a reaction solution, genomic DNA prepared from a target organism for which a DNA library is to be produced can be used. A target organism species is not particularly limited, and a target organism species can be, for example, an animal including a human, a plant, a microorganism, or a virus. That is, a DNA library can be produced from any organism species.

When producing a DNA library, the concentration of a random primer may be prescribed as described above. Thus, a nucleic acid fragment (or nucleic acid fragments) can be amplified with high reproducibility. The term "reproducibility" used herein refers to an extent of concordance among nucleic acid fragments amplified by a plurality of nucleic acid amplification reactions carried out with the use of the same template and the same random primer. That is, the term "high reproducibility (or the expression "reproducibility is high")" refers to a high extent of concordance among nucleic acid fragments amplified by a plurality of nucleic acid amplification reactions carried out with the use of the same template and the same random primer.

The extent of reproducibility can be evaluated by, for example, conducting a plurality of nucleic acid amplification reactions with the use of the same template and the same random primer, calculating the Spearman's rank correlation coefficient for the data of the nucleotide sequences of the resulting amplified fragments, and evaluating the extent of reproducibility on the basis of such coefficient. The Spearman's rank correlation coefficient is generally represented by the symbol ρ (rho). When ρ (rho) is greater than 0.9, for example, the reproducibility of the amplification reaction of interest can be evaluated to be sufficient.

### Random primer

A sequence constituting a random primer that can be used for producing a DNA library is not particularly limited. A random primer comprising nucleotides having 9 to 30 bases can be used. In particular, a random primer may be composed of any nucleotide sequence comprising 9 to 30 bases, a nucleotide type (i.e., a sequence type) is not particularly limited, and a random primer may be composed of 1 or more types of nucleotide sequences, preferably 1 to 10,000 types of nucleotide sequences, more preferably 1 to 1,000 types of nucleotide sequences, further preferably 1 to 100 types of nucleotide sequences, and most preferably 1 to 96 types of nucleotide sequences. With the use of nucleotides (or a group of nucleotides) within the range mentioned above for a random primer, an amplified nucleic acid fragment can be obtained with higher reproducibility. When a random primer comprises a plurality of nucleotide sequences, it is not necessary that all nucleotide sequences comprise the same number of bases (9 to 30 nucleotides). A random primer may comprise a plurality of nucleotide sequences composed of a different number of bases.

When designing a plurality of types of nucleotide sequences for a random primer, 30% or more, preferably 50% or more, more preferably 70% or more, and further preferably 90% or more of the entire such sequences exhibit 70% or less, preferably 60% or less, more preferably 50% or less, and most preferably 40% or less identity. By designing a plurality of types of nucleotides for a random primer exhibiting the identity within such range, an amplified fragment can be obtained over the entire genomic DNA of the target organism species. Thus, uniformity of the amplified fragment can be enhanced.

A nucleotide sequence constituting a random primer is preferably designed to have a G-C content of 5% to 95%, more preferably 10% to 90%, further preferably 15% to 80%, and most preferably 20% to 70%. With the use of an aggregate of nucleotides having the G-C content within the aforementioned range as a random primer, amplified nucleic acid fragments can be obtained with higher reproducibility. G-C content is the percentage of guanine and cytosine contained in the whole nucleotide chain.

In particular, a nucleotide sequence used as a random primer is preferably designed to comprise continuous bases accounting for 80% or less, more preferably 70% or less, further preferably 60% or less, and most preferably 50% or less of the full-length sequence. Alternatively, the number of continuous bases in a nucleotide sequence used as a random primer is preferably 8 or less, more preferably 7 or less, further preferably 6 or less, and most preferably 5 or less. With the use of an aggregate of nucleotides comprising the number of continuous bases within the aforementioned range as a random primer, amplified nucleic acid fragments can be obtained with higher reproducibility.

In addition, it is preferable that a nucleotide sequence used as a random primer be designed to not comprise a complementary region of 6 or more, more preferably 5 or more, and further preferably 4 or more bases in a molecule. Thus, double strand formation occurring in a molecule can be prevented, and amplified nucleic acid fragments can be obtained with higher reproducibility.

When a plurality of types of nucleotide sequences are designed as random primers, in particular, it is preferable that a plurality of nucleotide sequences be designed to not comprise complementary regions of 6 or more, more preferably 5 or more, and further preferably 4 or more bases among a plurality of types of nucleotide sequences. Thus, double strand formation occurring between nucleotide sequences can be prevented, and amplified nucleic acid fragments can be obtained with higher reproducibility.

When a plurality of nucleotide sequences are designed as random primers, in addition, it is preferable that such sequences be designed to not comprise complementary regions of 6 or more, more preferably 5 or more, and further preferably 5 or more bases at the 3' terminus. Thus, double strand formation occurring between nucleotide sequences can be prevented, and amplified nucleic acid fragments can be obtained with higher reproducibility.

The terms "complementary regions" and "complementary sequences" refer to, for example, regions and sequences exhibiting 80% to 100% identity to each other (e.g., regions and sequences each comprising 5 bases in which 4 or 5 bases are complementary to each other) or regions and sequences exhibiting 90% to 100% identity to each other (e.g., regions and sequences each comprising 5 bases in which 5 bases are complementary to each other).

Further, a nucleotide sequence used as a random primer is preferably designed to have a Tm value suitable for thermal cycling conditions (in particular, an annealing temperature) of a nucleic acid amplification reaction. A Tm value can be calculated by a conventional method, such as the nearest neighbor base pair approach, the Wallace method, and the GC% method, although a method of calculation is not particularly limited thereto. Specifically, a nucleotide sequence used as a random primer is preferably designed to have a Tm value of 10 to 85 degrees C, more preferably 12 to 75 degrees C, further preferably 14 to 70 degrees C, and most preferably 16 to 65 degrees C. By designing a random primer to have a Tm value within the aforementioned range, amplified nucleic acid fragments can be obtained with higher reproducibility under given thermal cycling conditions (in particular, at a given annealing temperature) of the nucleic acid amplification reaction.

When a plurality of nucleotide sequences are designed as random primers, in addition, a variation for Tm among a plurality of nucleotide sequences is preferably 50 degrees C or less, more preferably 45 degrees C or less, further preferably 40 degrees C or less, and most preferably 35 degrees C or less. By designing random primers while adjusting a variation for Tm among a plurality of nucleotide sequences within the range mentioned above, amplified nucleic acid fragments can be obtained with higher reproducibility under given thermal cycling conditions (in particular, at a given annealing temperature) of the nucleic acid amplification reaction.

### Nucleic acid amplification reaction

When producing a DNA library, many DNA fragments are obtained via the nucleic acid amplification reaction carried out with the use of random primers and genomic DNA as a template described above. At the time of the nucleic acid amplification reaction, in particular, the concentration of random primes in a reaction solution is prescribed higher than the concentration of primers in a conventional nucleic acid amplification reaction. Thus, many DNA fragments can be obtained with the use of genomic DNA as a template while achieving high reproducibility. Such many DNA fragments can be used for a DNA library that can be used for genotyping and other purposes.

A nucleic acid amplification reaction is aimed at synthesis of amplified fragments in a reaction solution containing genomic DNA as a template, the random primers, DNA polymerase, deoxynucleoside triphosphates as a substrate (i.e., dNTP, which is a mixture of dATP, dCTP, dTTP, and dGTP), and a buffer under the given thermal cycling conditions. It is necessary that a nucleic acid amplification reaction be carried out in a reaction solution containing Mg²⁺ at a given concentration. In the reaction solution of the composition described above, the buffer contains MgCl₂. When the buffer does not contain MgCl₂, the reaction solution of the composition described above further contains MgCl₂.

In a nucleic acid amplification reaction, in particular, it is preferable that the concentration of random primers be adequately determined in accordance with the base lengths of the random primers. When a plurality of types of nucleotide sequences having different numbers of bases are used as random primers, the number of bases constituting the random primers may be the average of such plurality of nucleotide sequences (the average may be a simple average or the weight average taking the amount of nucleotides into account).

Specifically, a nucleic acid amplification reaction is carried out with the use of a random primer comprising 9 to 30 bases at a concentration of 4 to 200 microM, and preferably at 4 to 100 microM. Under such conditions, many amplified fragments, and, in particular, many amplified fragments comprising 100 to 500 bases, can be obtained via a nucleic acid amplification reaction while achieving high reproducibility.

When a random primer comprises 9 to 10 bases, more specifically, the concentration of such random primer is 40 to 60 microM. When a random primer comprises 10 to 14 bases, it is that the concentration of such random primer satisfy the conditions defined by an in equation: y >3E + 08x^{-6.974} and be 100 microM or less, provided that the base length of the random primer is represented by "y" and the concentration of the random primer is represented by "x." When a random primer comprises 14 to 18 bases, the concentration of such random primer is 4 to 100 microM. When a random primer comprises 18 to 28 bases, it is that the concentration of such random primer be 4 microM or more and satisfy the conditions defined by an in equation: y <8E + 08x^{-5.533}. When a random primer comprises 28 to 29 bases, the concentration of such random primer is 6 to 10 microM. By designating the random primer concentration in accordance with the number of bases constituting the random primer as described above, many amplified fragments can be obtained with more certainty while achieving high reproducibility.

As described in the examples below, the in equations: y >3E + 08x^{-6.974} and y <8E +08x^{-5.533},are developed to be able to represent the concentration of a random primer at which many DNA fragments comprising 100 to 500 bases can be obtained with high reproducibility as a result of thorough inspection of the correlation between random primer length and random primer concentration.

While the amount of genomic DNA serving as a template in a nucleic acid amplification reaction is not particularly limited, it is preferably 0.1 to 1000 ng, more preferably 1 to 500 ng, further preferably 5 to 200 ng, and most preferably 10 to 100 ng, when the amount of the reaction solution is 50 microliters. By designating the amount of genomic DNA as a template within such range, many amplified fragments can be obtained without inhibiting the amplification reaction from a random primer, while achieving high reproducibility.

Genomic DNA can be prepared in accordance with a conventional technique without particular limitation. With the use of a commercialized kit, also, genomic DNA can be easily prepared from a target organism species. Genomic DNA extracted from an organism in accordance with a conventional technique or with the use of a commercialized kit may be used without further processing, genomic DNA extracted from an organism and then purified may be used, or genomic DNA subjected to restriction enzyme treatment or ultrasonic treatment may be used.

DNA polymerase used in a nucleic acid amplification reaction is not particularly limited, and an enzyme having DNA polymerase activity under thermal cycling conditions for a nucleic acid amplification reaction can be used. Specifically, heat-stable DNA polymerase used for a general nucleic acid amplification reaction can be used. Examples of DNA polymerases include thermophilic bacteria-derived DNA polymerase, such as Taq DNA polymerase, and hyperthermophilic archaea-derived DNA polymerase, such as KOD DNA polymerase and Pfu DNA polymerase. In a nucleic acid amplification reaction, it is particularly preferable that Pfu DNA polymerase be used as DNA polymerase in combination with the random primer described above. With the use of such DNA polymerase, many amplified fragments can be obtained with more certainty while achieving high reproducibility.

In a nucleic acid amplification reaction, the concentration of deoxynucleoside triphosphate as a substrate (i.e., dNTP, which is a mixture of dATP, dCTP, dTTP, and dGTP) is not particularly limited, and it can be 5 microM to 0.6 mM, preferably 10 microM to 0.4 mM, and more preferably 20 microM to 0.2 mM. By designating the concentration of dNTP serving as a substrate within such range, errors caused by incorrect incorporation by DNA polymerase can be prevented, and many amplified fragments can be obtained while achieving high reproducibility.

A buffer used in a nucleic acid amplification reaction is not particularly limited. For example, a solution comprising MgCl₂ as described above, Tris-HCl (pH 8.3), and KCl can be used. The concentration of Mg²⁺ is not particularly limited. For example, it can be 0.1 to 4.0 mM, preferably 0.2 to 3.0 mM, more preferably 0.3 to 2.0 mM, and further preferably 0.5 to 1.5 mM. By designating the concentration of Mg²⁺ in the reaction solution within such range, many amplified fragments can be obtained while achieving high reproducibility.

Thermal cycling conditions of a nucleic acid amplification reaction are not particularly limited, and a common thermal cycle can be adopted. A specific example of a thermal cycle comprises a first step of thermal denaturation in which genomic DNA as a template is dissociated into single strands, a cycle comprising thermal denaturation, annealing, and extension repeated a plurality of times (e.g., 20 to 40 times), a step of extension for a given period of time according to need, and the final step of storage.

Thermal denaturation can be performed at, for example, 93 to 99 degrees C, preferably 95 to 98 degrees C, and more preferably 97 to 98 degrees C. Annealing can be performed at, for example, 30 to 70 degrees C, preferably 35 to 68 degrees C, and more preferably 37 to 65 degrees C, although it varies depending on a Tm value of the random primer. Extension can be performed at, for example, 70 to 76 degrees C, preferably 71 to 75 degrees C, and more preferably 72 to 74 degrees C. Storage can be performed at, for example, 4 degrees C.

The first step of thermal denaturation can be performed within the temperature range described above for a period of, for example, 5 seconds to 10 minutes, preferably 10 seconds to 5 minutes, and more preferably 30 seconds to 2 minutes. In the cycle comprising "thermal denaturation, annealing, and extension," thermal denaturation can be carried out within the temperature range described above for a period of, for example, 2 seconds to 5 minutes, preferably 5 seconds to 2 minutes, and more preferably 10 seconds to 1 minute. In the cycle comprising "thermal denaturation, annealing, and extension," annealing can be carried out within the temperature range described above for a period of, for example, 1 second to 3 minutes, preferably 3 seconds to 2 minutes, and more preferably 5 seconds to 1 minute. In the cycle comprising "thermal denaturation, annealing, and extension," extension can be carried out within the temperature range described above for a period of, for example, 1 second to 3 minutes, preferably 3 seconds to 2 minutes, and more preferably 5 seconds to 1 minute.

When producing a DNA library, amplified fragments may be obtained by a nucleic acid amplification reaction that employs a hot start method. The hot start method is intended to prevent mis-priming or non-specific amplification caused by primer-dimer formation prior the cycle comprising "thermal denaturation, annealing, and extension." The hot start method involves the use of an enzyme in which DNA polymerase activity has been suppressed by binding an anti-DNA polymerase antibody thereto or chemical modification thereof. Thus, DNA polymerase activity can be suppressed and a non-specific reaction prior to the thermal cycle can be prevented. According to the hot start method, a temperature is set high in the first thermal cycle, DNA polymerase activity is thus recovered, and the subsequent nucleic acid amplification reaction is then allowed to proceed.

As described above, many amplified fragments can be obtained with the use of genomic DNA as a template and a random primer by conducting a nucleic acid amplification reaction with the use of a random primer comprising 9 to 30 bases and prescribing the concentration thereof to 4 to 200 microM in a reaction solution. With the use of the random primer comprising 9 to 30 bases by prescribing the concentration thereof to 4 to 200 microM in a reaction solution, a nucleic acid amplification reaction can be performed with very high reproducibility. According to the nucleic acid amplification reaction, specifically, many amplified fragments can be obtained while achieving very high reproducibility. Accordingly, such many amplified fragments can be used for a DNA library in genetic analysis targeting genomic DNA.

By performing a nucleic acid amplification reaction with the use of the random primer comprising 9 to 30 bases and prescribing the concentration thereof in a reaction solution to 4 to 200 microM, in particular, many amplified fragments comprising about 100 to 500 bases can be obtained with the use of genomic DNA as a template. Such many amplified fragments comprising about 100 to 500 bases are suitable for mass analysis of nucleotide sequences with the use of, for example, a next-generation sequencer, and highly accurate sequence information can thus be obtained. According to the present invention, accordingly, a DNA library, including DNA fragments comprising about 100 to 500 bases, can be produced.

By performing a nucleic acid amplification reaction with the use of the random primer comprising 9 to 30 bases and prescribing the concentration thereof to 4 to 200 microM in a reaction solution, in particular, the entire genomic DNA can be uniformly amplified. In other words, amplified DNA fragments are not obtained from a particular region of genomic DNA by the nucleic acid amplification reaction with the use of such random primer, but amplified fragments are obtained from the entire genome. According to the present invention, specifically, a DNA library can be produced uniformly across the entire genome.

### DNA probe

In the present invention, the term "DNA probe" refers to a DNA fragment that has a nucleotide sequence complementary to the target DNA fragment and is able to hybridize to such DNA fragment. A DNA probe that is applicable to a so-called oligonucleotide microarray is particularly preferable. An oligonucleotide microarray is a microarray in which oligonucleotides comprising nucleotide sequences of interest are synthesized on a support and the synthesized oligonucleotides are used as DNA probes. The synthesized oligonucleotides serving as DNA probes comprise, for example, 20 to 100 bases, preferably 30 to 90 bases, and more preferably 50 to 60 bases.

The DNA probes designed in accordance with the present invention may be applied to a microarray comprising the synthesized oligonucleotides with the base length described above immobilized on a support, as with the case of the so-called Stanford-type microarray. Specifically, the DNA probes designed in accordance with the present invention can be applied to any microarrays according to conventional techniques. Thus, the DNA probes designed in accordance with the present invention can be applied to a microarray comprising a flat substrate, such as a glass or silicone substrate, as a support and a bead array comprising a microbead support.

According to the method for producing a DNA probe of the present invention, a nucleotide sequence of a DNA probe is designed to detect a DNA fragment (a DNA library) on the basis of the nucleotide sequence of the DNA fragment. Specifically, the nucleotide sequence of the DNA fragment (the DNA library) produced in the manner described above is first determined, and a nucleotide sequence of a DNA probe is designed based on the determined nucleotide sequence. A method for determining a nucleotide sequence of a DNA fragment is not particularly limited. For example, a DNA sequencer in accordance with the Sanger method or a next-generation sequencer can be used. While a next-generation sequencer is not particularly limited, such sequencer is also referred to as a second-generation sequencer, and such sequencer is an apparatus for nucleotide sequencing that is capable of simultaneous determination of nucleotide sequences of several tens of millions of DNA fragments. A sequencing principle of the next-generation sequencer is not particularly limited. For example, sequencing can be carried out in accordance with the method in which target DNA is amplified on flow cells and sequencing is carried out while conducting synthesis with the use of bridge PCR method and sequencing-by-synthesis method, or in accordance with emulsion PCR method and the method of Pyrosequencing in which sequencing is carried out by assaying the amount of pyrophosphoric acids released at the time of and DNA synthesis. More specific examples of next-generation sequencers include MiniSeq, MiSeq, NextSeq, HiSeq, and HiSeq X Series (Illumina) and Roche 454 GS FLX sequencers (Roche).

Subsequently, a DNA probe is designed to comprise, a nucleotide sequence complementary to the nucleotide sequence of the DNA fragment (the DNA library) described above. More specifically, a region or a plurality of regions of the base lengths shorter than those of the DNA fragment (the DNA library) and covering at least a part of the DNA fragment (the DNA library) is/are identified, and the identified one or more regions are designed as probes for detecting the DNA fragment (the DNA library).

When a plurality of regions are designed for a particular DNA fragment, such DNA fragment is to be detected with the use of a plurality of DNA probes. A region may be designed for a particular DNA fragment, and two or more regions may be designed for another DNA fragment. Specifically, a different number of regions; that is, DNA probes, may be designed for each DNA fragment. When a plurality of DNA probes are to be designed for a DNA fragment, parts of such plurality of DNA probes may overlap with each other, or such plurality of DNA probes may be designed with intervals comprising several bases.

The number of bases constituting a DNA probe to be designed in the manner described above is not particularly limited. Such DNA probe can comprise 20 to 100 bases, preferably 30 to 90 bases, more preferably 40 to 80 bases, and most preferably 50 to 60 bases.

It is particularly preferable that a plurality of regions be designed, in such a manner that the entire region of a genomic DNA fragment, the nucleotide sequence of which had been determined, would be covered with a plurality of regions. In such a case, a plurality of probes can react with a genomic DNA fragment obtained from genomic DNA derived from a particular organism species via restriction enzyme treatment, and such genomic DNA fragment can be detected with the use of such plurality of probes.

A Tm value of a DNA probe is not particularly limited, and it can be 60 to 95 degrees C, preferably 70 to 90 degrees C, more preferably 75 to 85 degrees C, and most preferably 78 to 82degrees C.

When preparing DNA fragments from genomic DNAs with the use of random primers as described above, DNA fragments are obtained from a plurality of different genomic DNAs, and nucleotide sequences of these DNA fragments with different origins can be determined independently from each other. By comparing the determined nucleotide sequences, regions having different nucleotide sequences among the genomic DNAs can be identified. According to the method for producing a DNA probe of the present invention, DNA probes can be designed to comprise regions having different nucleotide sequences among the genomic DNAs thus identified. Specifically, a DNA probe may be designed to comprise a region of a particular genomic DNA that is different from another genomic DNA, and another DNA probe may be designed to comprise a region of the other genomic DNA that is different from the aforementioned particular genomic DNA. With the use of a pair of DNA probes thus designed, a specific type of genomic DNA to be analyzed can be identified.

The nucleotide sequence of the DNA fragment amplified from genomic DNA with the use of a random primer may be compared with a known nucleotide sequence, and a DNA probe may be designed to comprise a region different from such known nucleotide sequence. A known nucleotide sequence can be obtained from a variety of conventional databases. While any databases can be used without particular limitation, the DDBJ database provided by the DNA Data Bank of Japan, the EMBL database provided by the European Bioinformatics Institute, the Genbank database provided by the National Center for Biotechnology Information, the KEGG database provided by the Kyoto Encyclopedia of Genes and Genomes, or a combined database comprising such various databases can be adequately used.

### Apparatus for DNA analysis (not covered by the present claims)

The apparatus for DNA analysis (not covered by the present claims) comprises the DNA probes designed in the manner described above immobilized on a support. An apparatus for DNA analysis comprising DNA probes immobilized on a support is occasionally referred to as a "DNA microarray." Specifically, the apparatus for DNA analysis (not covered by the present claims) is not limited to a so-called DNA chip comprising DNA probes immobilized on a support (i.e., a DNA microarray in a narrow sense), and apparatuses composed to be capable of utilization of DNA probes designed in the manner described above on a support are not covered by the present claims.

For example, a DNA microarray comprising DNA probes designed in the manner described above can be produced in accordance with a conventional technique. A DNA microarray can be produced by, for example, synthesizing an oligonucleotide comprising a nucleotide sequence of the DNA probe designed in the manner described above on a support based on such nucleotide sequence. A method for oligonucleotide synthesis is not particularly limited, and any conventional technique can be employed. For example, oligonucleotide synthesis can be performed on a support by photolithography in combination with chemical synthesis via light application. Alternatively, an oligonucleotide comprising a linker molecule having a high affinity with a support surface added to its terminus may be separately synthesized on the basis of the nucleotide sequence of the DNA probe designed in the manner described above, and the resulting oligonucleotide may then be immobilized on a support surface at a particular position. A DNA microarray can also be produced by spotting the DNA probe designed in the manner described above on a support with the use of a pin-type arrayer or a nozzle-type arrayer.

The DNA microarray thus produced (i.e., the apparatus for DNA analysis) comprises a DNA probe comprising a nucleotide sequence complementary to a DNA fragment amplified from genomic DNA derived from a particular type of organism with the use of a random primer at a high concentration. Specifically, the DNA microarray thus produced is intended to detect a DNA fragment amplified from genomic DNA with the use of a random primer at a high concentration with the use of a DNA probe.

A DNA microarray may be any of a microarray using a flat substrate made of glass or silicone as a support, a bead array comprising a microbead support, and a three-dimensional microarray comprising a probe immobilized on an inner wall of a hollow fiber.

### Method of genomic DNA analysis

With the use of the DNA probe produced in the manner described above, analysis of genomic DNA, such as genotyping, can be performed. The DNA probe described above is equivalent to the DNA library produced with the use of a random primer at a high concentration. Such DNA library has very high reproducibility, the size of which is suitable for a next-generation sequencer, and it is uniform across the entire genome. Accordingly, the DNA library can be used as a DNA marker (it is also referred to as a genetic marker or a gene marker). The term "DNA marker" refers to a region in the genome serving as a marker associated with genetic traits. A DNA marker can be used for, for example, breeding comprising a step of selection with the use of genotype identification, linkage maps, gene mapping, or a marker, back crossing using a marker, quantitative trait locus mapping, bulked segregant analysis, variety identification, or discontinuous imbalance mapping.

Specifically, a DNA marker can be detected with the use of the DNA probe produced in the manner described above, and breeding comprising a step of selection with the use of genotype identification, linkage maps, gene mapping, and a marker, back crossing with the use of a marker, quantitative trait locus mapping, bulked segregant analysis, variety identification, or discontinuous imbalance mapping can be carried out.

More specifically, an example of a method for genomic DNA analysis involving the use of the DNA probe comprises bringing the DNA probe produced in the manner described above into contact with a DNA fragment derived from genomic DNA of the target of analysis. Such DNA fragment may be prepared with the use of the random primer that was used for producing the DNA library. Alternatively, a pair of primers that specifically amplify the DNA marker of interest may be designed on the basis of the nucleotide sequence of interest, and a DNA fragment may be prepared via a nucleic acid amplification reaction with the use of the pair of designed primers.

Subsequently, hybridization occurring between the DNA probe and the DNA fragment is detected in accordance with a conventional technique. For example, a label is added to the amplified DNA fragment, and hybridization of interest can be thus detected on the basis of the label. Any conventional substance may be used as a label. Examples of labels that can be used include a fluorescent molecule, a pigment molecule, and a radioactive molecule. A labeled nucleotide may be used in the step of DNA fragment amplification.

When a DNA microarray comprising a DNA probe is used, for example, a labeled DNA fragment is brought into contact with the DNA microarray under given conditions, and a DNA probe immobilized on the DNA microarray is allowed to hybridize to a labeled genomic DNA fragment. In this case, a probe hybridizes to a part of the DNA fragment, and it is preferable that hybridization be carried out under highly stringent conditions, so that hybridization does not occur in the presence of mismatch of a base, but it occurs only when the bases completely match. Under such highly stringent conditions, a slight change in single nucleotide polymorphism can be detected.

The stringency conditions can be adjusted in terms of reaction temperatures and salt concentrations. At a higher temperature, specifically, higher stringency conditions can be achieved. At a lower salt concentration, higher stringency conditions can be achieved. When a probe comprising 50 to 75 bases is used, for example, higher stringency conditions can be achieved by conducting hybridization at 40 to 44 degrees C with 0.21 SDS and 6× SSC.

Hybridization occurring between a DNA probe and a labeled DNA fragment can be detected based on a label. After the hybridization reaction between the labeled DNA fragment and the DNA probe, specifically, an unreacted DNA fragment or the like is washed, and a label of the DNA fragment that had specifically hybridized to the DNA probe is then observed. When a label is a fluorescent substance, for example, the fluorescent wavelength is detected. When a label is a pigment molecule, the pigment wavelength is detected. More specifically, an apparatus used for general DNA microarray analysis, such as a fluorescence detector or image analyzer, can be used.

In particular, DNA fragments amplified using genomic DNA as a template and a random primer at a high concentration can be detected with the use of such DNA probe. When a DNA probe comprising regions that are different among a plurality of different genomic DNAs is used, the genomic DNA as the target of analysis can be analyzed in accordance with the DNA probe to which a DNA fragment derived from the genomic DNA as the target of analysis had hybridized. For example, a DNA probe reacting with a DNA marker comprising differences in nucleotide sequences among genomic DNAs of relative species may be used, so that the species of the genomic DNA as the target of analysis can be identified.

### Examples

Hereafter, the present invention is described in greater detail with reference to the following examples, although the technical scope of the present invention is not limited to these examples.

### Example 1

### 1. Flow chart

In this example, a DNA library was prepared via PCR using genomic DNAs extracted from various types of organism species as templates and various sets of random primers in accordance with the flow chart shown in Fig. 1. With the use of the prepared DNA library, also, sequence analysis was performed with the use of a so-called next-generation sequencer, and the genotype was analyzed based on the read data.

### 2. Materials

In this example, genomic DNAs were extracted from the sugarcane varieties NiF8 and Ni9, 22 hybrid progeny lines thereof, and the rice variety Nipponbare using the DNeasy Plant Mini kit (QIAGEN), and the extracted genomic DNAs were purified. The purified genomic DNAs were used as NiF8-derived genomic DNA, Ni9-derived genomic DNA, 22 hybrid sugarcane progeny-derived genomic DNAs, and Nipponbare-derived genomic DNA, respectively. In Example 1, human genomic DNA was purchased from TakaraBio and used as human-derived genomic DNA.

### 3. Method

### 3.1 Correlation between PCR condition and DNA fragment size

### 3.1.1 Random primer designing

In order to design random primers, GC content was set between 20% and 70%, and the number of continuous bases was adjusted to 5 or fewer. Sequence length was set at 16 levels (i.e., 8, 9, 10, 11, 12, 14, 16, 18, 20, 22, 24, 26, 28, 29, 30, and 35). For each sequence length, 96 types of nucleotide sequences were designed, and 96 sets of random primers were prepared. Concerning 10-base primers, 6 sets of random primers each comprising 96 types of random primers were designed (these 6 sets are referred to as 10-base primer A to 10-base primer F, respectively). In this example, specifically, 21 different sets of random primers were prepared.

Tables 1 to 21 show nucleotide sequences of random primers contained in such 21 different sets of random primers.

**[Table 1-1]**

| Table 1 List of random primers (10-base primers A) | | |
|---|---|---|
| No | Primer sequence | SEQ ID NO: |
| 1 | AGACGTCGTT | 1 |
| 2 | GAGGCGATAT | 2 |
| 3 | GTGCGAACGT | 3 |
| 4 | TTATACTGCC | 4 |
| 5 | CAAGTTCGCA | 5 |
| 6 | ACAAGGTAGT | 6 |
| 7 | ACACAGCGAC | 7 |
| 8 | TTACCGATGT | 8 |
| 9 | CACAGAGTCG | 9 |
| 10 | TTCAGCGCGT | 10 |
| 11 | AGGACCGTGA | 11 |
| 12 | GTCTGTTCGC | 12 |
| 13 | ACCTGTCCAC | 13 |
| 14 | CCGCAATGAC | 14 |
| 15 | CTGCCGATCA | 15 |
| 16 | TACACGGAGC | 16 |
| 17 | CCGCATTCAT | 17 |
| 18 | GACTCTAGAC | 18 |
| 19 | GGAGAACTTA | 19 |
| 20 | TCCGGTATGC | 20 |
| 21 | GGTCAGGAGT | 21 |
| 22 | ACATTGGCAG | 22 |
| 23 | CGTAGACTGC | 23 |
| 24 | AGACTGTACT | 24 |
| 25 | TAGACGCAGT | 25 |
| 26 | CCGATAATCT | 26 |
| 27 | GAGAGCTAGT | 27 |
| 28 | GTACCGCGTT | 28 |
| 29 | GACTTGCGCA | 29 |
| 30 | CGTGATTGCG | 30 |
| 31 | ATCGTCTCTG | 31 |
| 32 | CGTAGCTACG | 32 |
| 33 | GCCGAATAGT | 33 |
| 34 | GTACCTAGGC | 34 |
| 35 | GCTTACATGA | 35 |
| 36 | TCCACGTAGT | 36 |
| 37 | AGAGGCCATC | 37 |
| 38 | CGGTGATGCT | 38 |
| 39 | CACTGTGCTT | 39 |
| 40 | CATGATGGCT | 40 |
| 41 | GCCACACATG | 41 |
| 42 | CACACACTGT | 42 |
| 43 | CAGAATCATA | 43 |
| 44 | ATCGTCTACG | 44 |
| 45 | CGAGCAATAC | 45 |
| 46 | ACAAGCGCAC | 46 |
| 47 | GCTTAGATGT | 47 |
| 48 | TGCATTCTGG | 48 |
| 49 | TGTCGGACCA | 49 |
| 50 | AGGCACTCGT | 50 |
| 51 | CTGCATGTGA | 51 |
| 52 | ACCACGCCTA | 52 |
| 53 | GAGGTCGTAC | 53 |
| 54 | AATACTCTGT | 54 |
| 55 | TGCCAACTGA | 55 |
| 56 | CCTGTTCGGT | 56 |
| 57 | GTAGAGAGTT | 57 |
| 58 | TACAGCGTAA | 58 |
| 59 | TGACGTGATG | 59 |
| 60 | AGACGTCGGT | 60 |
| 61 | CGCTAGGTTC | 61 |
| 62 | GCCTTATAGC | 62 |
| 63 | CCTTCGATCT | 63 |
| 64 | AGGCAACGTG | 64 |

**[Table 1-2]**

| No | Primer sequence | SEQ ID NO: |
|---|---|---|
| 65 | TGAGCGGTGT | 65 |
| 66 | GTGTCGAACG | 66 |
| 67 | CGATGTTGCG | 67 |
| 68 | AACAAGACAC | 68 |
| 69 | GATGCTGGTT | 69 |
| 70 | ACCGGTAGTC | 70 |
| 71 | GTGACTAGCA | 71 |
| 72 | AGCCTATATT | 72 |
| 73 | TCGTGAGCTT | 73 |
| 74 | ACACTATGGC | 74 |
| 75 | GACTCTGTCG | 75 |
| 76 | TCGATGATGC | 76 |
| 77 | CTTGGACACT | 77 |
| 78 | GGCTGATCGT | 78 |
| 79 | ACTCACAGGC | 79 |
| 80 | ATGTGCGTAC | 80 |
| 81 | CACCATCGAT | 81 |
| 82 | AGCCATTAAC | 82 |
| 83 | AATCGACTGT | 83 |
| 84 | AATACTAGCG | 84 |
| 85 | TCGTCACTGA | 85 |
| 86 | CAGGCTCTTA | 86 |
| 87 | GGTCGGTGAT | 87 |
| 88 | CATTAGGCGT | 88 |
| 89 | ACTCGCGAGT | 89 |
| 90 | TTCCGAATAA | 90 |
| 91 | TGAGCATCGT | 91 |
| 92 | GCCACGTAAC | 92 |
| 93 | GAACTACATG | 93 |
| 94 | TCGTGAGGAC | 94 |
| 95 | GCGGCCTTAA | 95 |
| 96 | GCTAAGGACC | 96 |

**[Table 2-1]**

| Table 2 List of random primers (10-base primers B) | | |
|---|---|---|
| No | Primer sequence | SEQ ID NO |
| 1 | ATAGCCATTA | 97 |
| 2 | CAGTAATCAT | 98 |
| 3 | ACTCCTTAAT | 99 |
| 4 | TCGAACATTA | 100 |
| 5 | ATTATGAGGT | 101 |
| 6 | AATCTTAGAG | 102 |
| 7 | TTAGATGATG | 103 |
| 8 | TACATATCTG | 104 |
| 9 | TCCTTAATCA | 105 |
| 10 | GTTGAGATTA | 106 |
| 11 | TGTTAACGTA | 107 |
| 12 | CATACAGTAA | 108 |
| 13 | CTTATACGAA | 109 |
| 14 | AGATCTATGT | 110 |
| 15 | AAGACTTAGT | 111 |
| 16 | TGCGCAATAA | 112 |
| 17 | TTGGCCATAT | 113 |
| 18 | TATTACGAGG | 114 |
| 19 | TTATGATCGC | 115 |
| 20 | AACTTAGGAG | 116 |
| 21 | TCACAATCGT | 117 |
| 22 | GAGTATATGG | 118 |
| 23 | ATCAGGACAA | 119 |
| 24 | GTACTGATAG | 120 |
| 25 | CTTATACTCG | 121 |
| 26 | TAACGGACTA | 122 |
| 27 | GCGTTGTATA | 123 |
| 28 | CTTAAGTGCT | 124 |
| 29 | ATACGACTGT | 125 |
| 30 | ACTGTTATCG | 126 |
| 31 | AATCTTGACG | 127 |
| 32 | ACATCACCTT | 128 |
| 33 | GGTATAGTAC | 129 |
| 34 | CTAATCCACA | 130 |
| 35 | GCACCTTATT | 131 |
| 36 | ATTGACGGTA | 132 |
| 37 | GACATATGGT | 133 |
| 38 | GATAGTCGTA | 134 |
| 39 | CAATTATCGC | 135 |
| 40 | CTTAGGTGAT | 136 |
| 41 | CATACTACTG | 137 |
| 42 | TAACGCGAAT | 138 |
| 43 | CAAGTTACGA | 139 |
| 44 | AATCTCAAGG | 140 |
| 45 | GCAATCATCA | 141 |
| 46 | TGTAACGTTC | 142 |
| 47 | TATCGTTGGT | 143 |
| 48 | CGCTTAAGAT | 144 |
| 49 | TTAGAACTGG | 145 |
| 50 | GTCATAACGT | 146 |
| 51 | AGAGCAGTAT | 147 |
| 52 | CAACATCACT | 148 |
| 53 | CAGAAGCTTA | 149 |
| 54 | AACTAACGTG | 150 |
| 55 | TTATACCGCT | 151 |
| 56 | GAATTCGAGA | 152 |
| 57 | TTACGTAACC | 153 |
| 58 | GCATGGTTAA | 154 |
| 59 | GCACCTAATT | 155 |
| 60 | TGTAGGTTGT | 156 |
| 61 | CCATCTGGAA | 157 |
| 62 | TTCGCGTTGA | 158 |
| 63 | AACCGAGGTT | 159 |
| 64 | GTACGCTGTT | 160 |

**[Table 2-2]**

| No | Primer sequence | SEQ ID NO |
|---|---|---|
| 65 | AGTATCCTGG | 161 |
| 66 | GGTTGTACAG | 162 |
| 67 | ACGTACACCA | 163 |
| 68 | TGTCGAGCAA | 164 |
| 69 | GTCGTGTTAC | 165 |
| 70 | GTGCAATAGG | 166 |
| 71 | ACTCGATGCT | 167 |
| 72 | GAATCGCGTA | 168 |
| 73 | CGGTCATTGT | 169 |
| 74 | ATCAGGCGAT | 170 |
| 75 | GTAAGATGCG | 171 |
| 76 | GGTCTCTTGA | 172 |
| 77 | TCCTCGCTAA | 173 |
| 78 | CTGCGTGATA | 174 |
| 79 | CATACTCGTC | 175 |
| 80 | ATCTGAGCTC | 176 |
| 81 | ACGGATAGTG | 177 |
| 82 | ACTGCAATGC | 178 |
| 83 | TAACGACGTG | 179 |
| 84 | TAGACTGTCG | 180 |
| 85 | CAGCACTTCA | 181 |
| 86 | AACATTCGCC | 182 |
| 87 | ACTAGTGCGT | 183 |
| 88 | ACGCTGTTCT | 184 |
| 89 | CGTCGAATGC | 185 |
| 90 | CTCTGACGGT | 186 |
| 91 | GTCGCCATGT | 187 |
| 92 | GGTCCACGTT | 188 |
| 93 | CGAGCGACTT | 189 |
| 94 | TTGACGCGTG | 190 |
| 95 | CTGAGAGCCT | 191 |
| 96 | CGCGCTAACT | 192 |

**[Table 3-1]**

| Table 3 List of random primers (10-base primers C) | | |
|---|---|---|
| No | Primer sequence | SEQ ID NO |
| 1 | GGTCGTCAAG | 193 |
| 2 | AGGTTGACCA | 194 |
| 3 | TAACGGCAAC | 195 |
| 4 | GAGGCTGGAT | 196 |
| 5 | GTGCACACCT | 197 |
| 6 | TGAGGACCAG | 198 |
| 7 | TACTTGCGAG | 199 |
| 8 | AACTGTGAGA | 200 |
| 9 | CTCCATCAAC | 201 |
| 10 | CGGACTGTTA | 202 |
| 11 | TAGGACAGTC | 203 |
| 12 | AGAGGACACA | 204 |
| 13 | ACATTCGCGG | 205 |
| 14 | GCTTACTGCA | 206 |
| 15 | CAATACGTAA | 207 |
| 16 | AGACTTGCGC | 208 |
| 17 | GAGCGGTGTT | 209 |
| 18 | CGTGAGAGGT | 210 |
| 19 | AATCCGTCAG | 211 |
| 20 | ATACGTACCG | 212 |
| 21 | AACTGATTCC | 213 |
| 22 | CTGAGCGTAC | 214 |
| 23 | GTCGGATTCG | 215 |
| 24 | GCCGACCATA | 216 |
| 25 | GCAGAACTAA | 217 |
| 26 | CTAACGACCG | 218 |
| 27 | GCTGGACCAT | 219 |
| 28 | GACGCGGTTA | 220 |
| 29 | AGTGGTGAGC | 221 |
| 30 | CAGGCAGTCA | 222 |
| 31 | TCTGACGTCA | 223 |
| 32 | TACATGACGT | 224 |
| 33 | TGAGGCAACC | 225 |
| 34 | CAACTGCAGT | 226 |
| 35 | CGGAGATACG | 227 |
| 36 | CTTCGCAAGT | 228 |
| 37 | CTGGCATACG | 229 |
| 38 | TAACGTTCGC | 230 |
| 39 | CCGGCGTTAA | 231 |
| 40 | ACAAGACGCC | 232 |
| 41 | CCATTAGACT | 233 |
| 42 | GTCTGTGACA | 234 |
| 43 | GGCATTGGAC | 235 |
| 44 | TCTTCGCACG | 236 |
| 45 | TAGCCTGTGC | 237 |
| 46 | CACTGACCTA | 238 |
| 47 | CCGCACGATT | 239 |
| 48 | ATAGCACACG | 240 |
| 49 | GCACGTCATA | 241 |
| 50 | AAGCCGTTGG | 242 |
| 51 | CGGACCGTTA | 243 |
| 52 | TACACAGCGT | 244 |
| 53 | CGGACTTCAG | 245 |
| 54 | TAGAACGTCA | 246 |
| 55 | GGCATTGGAG | 247 |
| 56 | GGCACTCGTT | 248 |
| 57 | GTACCGTTAA | 249 |
| 58 | AATACGTGTC | 250 |
| 59 | CCATTGACGT | 251 |
| 60 | CGTGAATCGC | 252 |
| 61 | ATCAACGCGG | 253 |
| 62 | CGCCAAGGTA | 254 |
| 63 | AGAAGACGCC | 255 |
| 64 | CCGCATAGTC | 256 |

**[Table 3-2]**

| No | Primer sequence | SEQ ID NO |
|---|---|---|
| 65 | CTTATATGTG | 257 |
| 66 | GGTCTCATCG | 258 |
| 67 | CCACCATGTC | 259 |
| 68 | ACGAATGTGT | 260 |
| 69 | GGTAGTAACA | 261 |
| 70 | GCCACTTAAT | 262 |
| 71 | ATATTGCGCC | 263 |
| 72 | GACCAATAGT | 264 |
| 73 | AACAACACGG | 265 |
| 74 | ATAGCCGATG | 266 |
| 75 | CGAGAGCATA | 267 |
| 76 | CGAGACATGA | 268 |
| 77 | CGCCAAGTTA | 269 |
| 78 | TTATAATCGC | 270 |
| 79 | TAGAAGTGCA | 271 |
| 80 | GGAGGCATGT | 272 |
| 81 | GCCACTTCGA | 273 |
| 82 | TCCACGGTAC | 274 |
| 83 | CAACTATGCA | 275 |
| 84 | CAAGGAGGAC | 276 |
| 85 | GAGGTACCTA | 277 |
| 86 | GAGCGCATAA | 278 |
| 87 | TCGTCACGTG | 279 |
| 88 | AACTGTGACA | 280 |
| 89 | TCCACGTGAG | 281 |
| 90 | ACACTGCTCT | 282 |
| 91 | TACGGTGAGC | 283 |
| 92 | CGGACTAAGT | 284 |
| 93 | AAGCCACGTT | 285 |
| 94 | CAATTACTCG | 286 |
| 95 | TCTGGCCATA | 287 |
| 96 | TCAGGCTAGT | 288 |

**[Table 4-1]**

| Table 4 List of random primers (10-base primers D) | | |
|---|---|---|
| No | Primer sequence | SEQ ID NO |
| 1 | TTGACCCGGA | 289 |
| 2 | TTTTTATGGT | 290 |
| 3 | ATGTGGTGCG | 291 |
| 4 | AAGGCGCTAG | 292 |
| 5 | TCCAACTTTG | 293 |
| 6 | CCATCCCATC | 294 |
| 7 | CAATACGAGG | 295 |
| 8 | GAGTGTTACC | 296 |
| 9 | GCCTCCTGTA | 297 |
| 10 | CGAAGGTTGC | 298 |
| 11 | GAGGTGCTAT | 299 |
| 12 | TAGGATAATT | 300 |
| 13 | CGTTGTCCTC | 301 |
| 14 | TGAGACCAGC | 302 |
| 15 | TGCCCAAGCT | 303 |
| 16 | TACTGAATCG | 304 |
| 17 | TTACATAGTC | 305 |
| 18 | ACAAAGGAAA | 306 |
| 19 | CTCGCTTGGG | 307 |
| 20 | CCTTGCGTCA | 308 |
| 21 | TAATTCCGAA | 309 |
| 22 | GTGAGCTTGA | 310 |
| 23 | ATGCCGATTC | 311 |
| 24 | GCTTGGGCTT | 312 |
| 25 | ACAAAGCGCC | 313 |
| 26 | GAAAGCTCTA | 314 |
| 27 | TACCGACCGT | 315 |
| 28 | TCGAAGAGAC | 316 |
| 29 | GTCGCTTACG | 317 |
| 30 | GGGCTCTCCA | 318 |
| 31 | GCGCCCTTGT | 319 |
| 32 | GGCAATAGGC | 320 |
| 33 | CAAGTCAGGA | 321 |
| 34 | GGGTCGCAAT | 322 |
| 35 | CAGCAACCTA | 323 |
| 36 | TTCCCGCCAC | 324 |
| 37 | TGTGCATTTT | 325 |
| 38 | ATCAACGACG | 326 |
| 39 | GTGACGTCCA | 327 |
| 40 | CGATCTAGTC | 328 |
| 41 | TTACATCCTG | 329 |
| 42 | AGCCTTCAAT | 330 |
| 43 | TCCATCCGAT | 331 |
| 44 | GACTGGGTCT | 332 |
| 45 | TTCGGTGGAG | 333 |
| 46 | GACCAGCACA | 334 |
| 47 | CATTAACGGA | 335 |
| 48 | TTTTTCTTGA | 336 |
| 49 | CATTGCACTG | 337 |
| 50 | TGCGGCGATC | 338 |
| 51 | ATATTGCGGT | 339 |
| 52 | GACGTCGCTC | 340 |
| 53 | TCGCTTATCG | 341 |
| 54 | GCGCAGACAC | 342 |
| 55 | CATGTATTGT | 343 |
| 56 | TCTATAACCT | 344 |
| 57 | GTGGAGACAA | 345 |
| 58 | CGAAGATTAT | 346 |
| 59 | TAGCAACTGC | 347 |
| 60 | ATAATCGGTA | 348 |
| 61 | CAGGATGGGT | 349 |
| 62 | GACGATTCCC | 350 |
| 63 | CACGCCTTAC | 351 |
| 64 | AGTTGGTTCC | 352 |

**[Table 4-2]**

| No | Primer sequence | SEQ ID NO |
|---|---|---|
| 65 | TCTTATCAGG | 353 |
| 66 | CGAGAAGTTC | 354 |
| 67 | GTGGTAGAAT | 355 |
| 68 | TAGGCTTGTG | 356 |
| 69 | ATGCGTTACG | 357 |
| 70 | ACTACCGAGG | 358 |
| 71 | CGAGTTGGTG | 359 |
| 72 | GGACGATCAA | 360 |
| 73 | AACAGTATGC | 361 |
| 74 | TTGGCTGATC | 362 |
| 75 | AGGATTGGAA | 363 |
| 76 | CATATGGAGA | 364 |
| 77 | CTGCAGGTTT | 365 |
| 78 | CTCTCTTTTT | 366 |
| 79 | AGTAGGGGTC | 367 |
| 80 | ACACCGCAAG | 368 |
| 81 | GAAGCGGGAG | 369 |
| 82 | GATACGGACT | 370 |
| 83 | TACGACGTGT | 371 |
| 84 | GTGCCTCCTT | 372 |
| 85 | GGTGACTGAT | 373 |
| 86 | ATATCTTACG | 374 |
| 87 | AATCATACGG | 375 |
| 88 | CTCTTGGGAC | 376 |
| 89 | GACGACAAAT | 377 |
| 90 | GTTGCGAGGT | 378 |
| 91 | AAACCGCACC | 379 |
| 92 | GCTAACACGT | 380 |
| 93 | ATCATGAGGG | 381 |
| 94 | GATTCACGTA | 382 |
| 95 | TCTCGAAAAG | 383 |
| 96 | CTCGTAACCA | 384 |

**[Table 5-1]**

| Table 5 List of random primers (10-base primers E) | | |
|---|---|---|
| No | Primer sequence | SEQ ID NO |
| 1 | GTTACACACG | 385 |
| 2 | CGTGAAGGGT | 386 |
| 3 | ACGAGCATCT | 387 |
| 4 | ACGAGGGATT | 388 |
| 5 | GCAACGTCGG | 389 |
| 6 | CACGGCTAGG | 390 |
| 7 | CGTGACTCTC | 391 |
| 8 | TCTAGACGCA | 392 |
| 9 | CTGCGCACAT | 393 |
| 10 | ATGCTTGACA | 394 |
| 11 | TTTGTCGACA | 395 |
| 12 | ACGTGTCAGC | 396 |
| 13 | GAAAACATTA | 397 |
| 14 | ACATTAACGG | 398 |
| 15 | GTACAGGTCC | 399 |
| 16 | CTATGTGTAC | 400 |
| 17 | GCGTACATTA | 401 |
| 18 | GATTTGTGGC | 402 |
| 19 | TCGCGCGCTA | 403 |
| 20 | ACAAGGGCGA | 404 |
| 21 | AACGCGCGAT | 405 |
| 22 | CGTAAATGCG | 406 |
| 23 | TAGGCACTAC | 407 |
| 24 | GCGAGGATCG | 408 |
| 25 | CACGTTTACT | 409 |
| 26 | TACCACCACG | 410 |
| 27 | TTAACAGGAC | 411 |
| 28 | GCTGTATAAC | 412 |
| 29 | GTTGCTGGCA | 413 |
| 30 | AGTGTGGCCA | 414 |
| 31 | CTGCGGTTGT | 415 |
| 32 | TAGATCAGCG | 416 |
| 33 | TTCCGGTTAT | 417 |
| 34 | GATAAACTGT | 418 |
| 35 | TACAGTTGCC | 419 |
| 36 | CGATGGCGAA | 420 |
| 37 | CCGACGTCAG | 421 |
| 38 | TATGGTGCAA | 422 |
| 39 | GACGACAGTC | 423 |
| 40 | GTCACCGTCC | 424 |
| 41 | GGTTTTAACA | 425 |
| 42 | GAGGACAGTA | 426 |
| 43 | GTTACCTAAG | 427 |
| 44 | ATCACGTGTT | 428 |
| 45 | TAAGGCCTGG | 429 |
| 46 | TGTTCGTAGC | 430 |
| 47 | TGAGGACGTG | 431 |
| 48 | GTGCTGTGTA | 432 |
| 49 | GAGGGTACGC | 433 |
| 50 | CCGTGATTGT | 434 |
| 51 | AAAATCGCCT | 435 |
| 52 | CGATCGCAGT | 436 |
| 53 | ACGCAATAAG | 437 |
| 54 | AAGGTGCATC | 438 |
| 55 | CGCGTAGATA | 439 |
| 56 | CGAGCAGTGC | 440 |
| 57 | ATACGTGACG | 441 |
| 58 | AGATTGCGCG | 442 |
| 59 | ACGTGATGCC | 443 |
| 60 | GTACGCATCG | 444 |
| 61 | TCCCGACTTA | 445 |
| 62 | GTTTTTACAC | 446 |
| 63 | CCTGAGCGTG | 447 |
| 64 | CGGCATTGTA | 448 |

**[Table 5-2]**

| No | Primer sequence | SEQ ID NO |
|---|---|---|
| 65 | TAGAGTGCGT | 449 |
| 66 | ATGGCCAGAC | 450 |
| 67 | CTTAGCATGC | 451 |
| 68 | ACAACACCTG | 452 |
| 69 | AGTGACTATC | 453 |
| 70 | CATGCTACAC | 454 |
| 71 | AAAGCGGGCG | 455 |
| 72 | AGATCGCCGT | 456 |
| 73 | CGTAGATATT | 457 |
| 74 | AATGGCAGAC | 458 |
| 75 | GTATAACGTG | 459 |
| 76 | ATGTGCGTCA | 460 |
| 77 | CCTGCCAACT | 461 |
| 78 | TTTATAACTC | 462 |
| 79 | ACGGTTACGC | 463 |
| 80 | TAGCCTCTTG | 464 |
| 81 | TCGCGAAGTT | 465 |
| 82 | GTCTACAACC | 466 |
| 83 | GTCTACTGCG | 467 |
| 84 | GTTGCGTCTC | 468 |
| 85 | GGGCCGCTAA | 469 |
| 86 | GTACGTCGGA | 470 |
| 87 | AGCGAGAGAC | 471 |
| 88 | TGGCTACGGT | 472 |
| 89 | AGGCATCACG | 473 |
| 90 | TAGCTCCTCG | 474 |
| 91 | GGCTAGTCAG | 475 |
| 92 | CTCACTTTAT | 476 |
| 93 | ACGGCCACGT | 477 |
| 94 | AGCGTATATC | 478 |
| 95 | GACACGTCTA | 479 |
| 96 | GCCAGCGTAC | 480 |

**[Table 6-1]**

| Table 6 List of random primers (10-base primers F) | | |
|---|---|---|
| No | Primer sequence | SEQ ID NO |
| 1 | AACATTAGCG | 481 |
| 2 | AGTGTGCTAT | 482 |
| 3 | CACGAGCGTT | 483 |
| 4 | GTAACGCCTA | 484 |
| 5 | CACATAGTAC | 485 |
| 6 | CGCGATATCG | 486 |
| 7 | CGTTCTGTGC | 487 |
| 8 | CTGATCGCAT | 488 |
| 9 | TGGCGTGAGA | 489 |
| 10 | TTGCCAGGCT | 490 |
| 11 | GTTATACACA | 491 |
| 12 | AGTGCCAACT | 492 |
| 13 | TCACGTAGCA | 493 |
| 14 | TAATTCAGCG | 494 |
| 15 | AAGTATCGTC | 495 |
| 16 | CACAGTTACT | 496 |
| 17 | CCTTACCGTG | 497 |
| 18 | ACGGTGTCGT | 498 |
| 19 | CGCGTAAGAC | 499 |
| 20 | TTCGCACCAG | 500 |
| 21 | CACGAACAGA | 501 |
| 22 | GTTGGACATT | 502 |
| 23 | GGTGCTTAAG | 503 |
| 24 | TCGGTCTCGT | 504 |
| 25 | TCTAGTACGC | 505 |
| 26 | TTAGGCCGAG | 506 |
| 27 | CGTCAAGAGC | 507 |
| 28 | ACATGTCTAC | 508 |
| 29 | ATCGTTACGT | 509 |
| 30 | ACGGATCGTT | 510 |
| 31 | AATCTTGGCG | 511 |
| 32 | AGTATCTGGT | 512 |
| 33 | CAACCGACGT | 513 |
| 34 | TGGTAACGCG | 514 |
| 35 | GTGCAGACAT | 515 |
| 36 | GTCTAGTTGC | 516 |
| 37 | CAATTCGACG | 517 |
| 38 | CTTAGCACCT | 518 |
| 39 | TAATGTCGCA | 519 |
| 40 | CAATCGGTAC | 520 |
| 41 | AGCACGCATT | 521 |
| 42 | AGGTCCTCGT | 522 |
| 43 | TTGTGCCTGC | 523 |
| 44 | ACCGCCTGTA | 524 |
| 45 | GTACGTCAGG | 525 |
| 46 | GCACACAACT | 526 |
| 47 | TGAGCACTTA | 527 |
| 48 | GTGCCGCATA | 528 |
| 49 | ATGTTTTCGC | 529 |
| 50 | ACACTTAGGT | 530 |
| 51 | CGTGCCGTGA | 531 |
| 52 | TTACTAATCA | 532 |
| 53 | GTGGCAGGTA | 533 |
| 54 | GCGCGATATG | 534 |
| 55 | GAACGACGTT | 535 |
| 56 | ATCAGGAGTG | 536 |
| 57 | GCCAGTAAGT | 537 |
| 58 | GCAAGAAGCA | 538 |
| 59 | AACTCCGCCA | 539 |
| 60 | ACTTGAGCCT | 540 |
| 61 | CGTGATCGTG | 541 |
| 62 | AATTAGCGAA | 542 |
| 63 | ACTTCCTTAG | 543 |
| 64 | TGTGCTGATA | 544 |

**[Table 6-2]**

| No | Primer sequence | SEQ ID NO |
|---|---|---|
| 65 | AGGCGGCTGA | 545 |
| 66 | CGTTTAGAGC | 546 |
| 67 | ACGCGTCTAA | 547 |
| 68 | GCGAATGTAC | 548 |
| 69 | CGTGATCCAA | 549 |
| 70 | CAACCAGATG | 550 |
| 71 | ACCATTAACC | 551 |
| 72 | CGATTCACGT | 552 |
| 73 | CTAGAACCTG | 553 |
| 74 | CCTAACGACA | 554 |
| 75 | GACGTGCATG | 555 |
| 76 | ATGTAACCTT | 556 |
| 77 | GATACAGTCG | 557 |
| 78 | CGTATGTCTC | 558 |
| 79 | AGATTATCGA | 559 |
| 80 | ATACTGGTAA | 560 |
| 81 | GTTGAGTAGC | 561 |
| 82 | ACCATTATCA | 562 |
| 83 | CACACTTCAG | 563 |
| 84 | GACTAGCGGT | 564 |
| 85 | AATTGTCGAG | 565 |
| 86 | CTAAGGACGT | 566 |
| 87 | ATTACGATGA | 567 |
| 88 | ATTGAAGACT | 568 |
| 89 | GCTTGTACGT | 569 |
| 90 | CCTACGTCAC | 570 |
| 91 | CACAACTTAG | 571 |
| 92 | GCGGTTCATC | 572 |
| 93 | GTACTCATCT | 573 |
| 94 | GTGCATCAGT | 574 |
| 95 | TCACATCCTA | 575 |
| 96 | CACGCGCTAT | 576 |

**[Table 7-1]**

| Table 7 List of random primers (8-base primers) | | |
|---|---|---|
| No | Primer sequence | SEQ ID NO |
| 1 | CTATCTTG | 577 |
| 2 | AAGTGCGT | 578 |
| 3 | ACATGCGA | 579 |
| 4 | ACCAATGG | 580 |
| 5 | TGCGTTGA | 581 |
| 6 | GACATGTC | 582 |
| 7 | TTGTGCGT | 583 |
| 8 | ACATCGCA | 584 |
| 9 | GAAGACGA | 585 |
| 10 | TCGATAGA | 586 |
| 11 | TCTTGCAA | 587 |
| 12 | AGCAAGTT | 588 |
| 13 | TTCATGGA | 589 |
| 14 | TCAATTCG | 590 |
| 15 | CGGTATGT | 591 |
| 16 | ACCACTAC | 592 |
| 17 | TCGCTTAT | 593 |
| 18 | TCTCGACT | 594 |
| 19 | GAATCGGT | 595 |
| 20 | GTTACAAG | 596 |
| 21 | CTGTGTAG | 597 |
| 22 | TGGTAGAA | 598 |
| 23 | ATACTGCG | 599 |
| 24 | AACTCGTC | 600 |
| 25 | ATATGTGC | 601 |
| 26 | AAGTTGCG | 602 |
| 27 | GATCATGT | 603 |
| 28 | TTGTTGCT | 604 |
| 29 | CCTCTTAG | 605 |
| 30 | TCACAGCT | 606 |
| 31 | AGATTGAC | 607 |
| 32 | AGCCTGAT | 608 |
| 33 | CGTCAAGT | 609 |
| 34 | AAGTAGAC | 610 |
| 35 | TCAGACAA | 611 |
| 36 | TCCTTGAC | 612 |
| 37 | GTAGCTGT | 613 |
| 38 | CGTCGTAA | 614 |
| 39 | CCAATGGA | 615 |
| 40 | TTGAGAGA | 616 |
| 41 | ACAACACC | 617 |
| 42 | TCTAGTAC | 618 |
| 43 | GAGGAAGT | 619 |
| 44 | GCGTATTG | 620 |
| 45 | AAGTAGCT | 621 |
| 46 | TGAACCTT | 622 |
| 47 | TGTGTTAC | 623 |
| 48 | TAACCTGA | 624 |
| 49 | GCTATTCC | 625 |
| 50 | GTTAGATG | 626 |
| 51 | CAGGATAA | 627 |
| 52 | ACCGTAGT | 628 |
| 53 | CCGTGTAT | 629 |
| 54 | TCCACTCT | 630 |
| 55 | TAGCTCAT | 631 |
| 56 | CGCTAATA | 632 |
| 57 | TACCTCTG | 633 |
| 58 | TGCACTAC | 634 |
| 59 | CTTGGAAG | 635 |
| 60 | AATGCACG | 636 |
| 61 | CACTGTTA | 637 |
| 62 | TCGACTAG | 638 |
| 63 | CTAGGTTA | 639 |
| 64 | GCAGATGT | 640 |

**[Table 7-2]**

| No | Primer sequence | SEQ ID NO |
|---|---|---|
| 65 | AGTTCAGA | 641 |
| 66 | CTCCATCA | 642 |
| 67 | TGGTTACG | 643 |
| 68 | ACGTAGCA | 644 |
| 69 | CTCTTCCA | 645 |
| 70 | CGTCAGAT | 646 |
| 71 | TGGATCAT | 647 |
| 72 | ATATCGAC | 648 |
| 73 | TTGTGGAG | 649 |
| 74 | TTAGAGCA | 650 |
| 75 | TAACTACC | 651 |
| 76 | CTATGAGG | 652 |
| 77 | CTTCTCAC | 653 |
| 78 | CGTTCTCT | 654 |
| 79 | GTCACTAT | 655 |
| 80 | TCGTTAGC | 656 |
| 81 | ATCGTGTA | 657 |
| 82 | GAGAGCAA | 658 |
| 83 | AGACGCAA | 659 |
| 84 | TCCAGTTA | 660 |
| 85 | AATGCCAC | 661 |
| 86 | ATCACGTG | 662 |
| 87 | ACTGTGCA | 663 |
| 88 | TCACTGCA | 664 |
| 89 | GCATCCAA | 665 |
| 90 | AGCACTAT | 666 |
| 91 | CGAAGGAT | 667 |
| 92 | CCTTGTGT | 668 |
| 93 | TGCGGATA | 669 |
| 94 | AGGAATGG | 670 |
| 95 | ATCGTAAC | 671 |
| 96 | GAATGTCT | 672 |

**[Table 8-1]**

| Table 8 List of random primers (9-base primers) | | |
|---|---|---|
| No | Primer sequence | SEQ ID NO |
| 1 | TTGCTACAT | 673 |
| 2 | TAACGTATG | 674 |
| 3 | CAGTATGTA | 675 |
| 4 | TCAATAACG | 676 |
| 5 | CACACTTAT | 677 |
| 6 | GACTGTAAT | 678 |
| 7 | TATACACTG | 679 |
| 8 | ACTGCATTA | 680 |
| 9 | ACATTAAGC | 681 |
| 10 | CATATTACG | 682 |
| 11 | ATATCTACG | 683 |
| 12 | AGTAACTGT | 684 |
| 13 | ATGACGTTA | 685 |
| 14 | ATTATGCGA | 686 |
| 15 | AGTATACAC | 687 |
| 16 | TTAGCGTTA | 688 |
| 17 | TATGACACT | 689 |
| 18 | ATTAACGCT | 690 |
| 19 | TAGGACAAT | 691 |
| 20 | AAGACGTTA | 692 |
| 21 | TATAAGCGT | 693 |
| 22 | ATACCTGGC | 694 |
| 23 | CTCGAGATC | 695 |
| 24 | ATGGTGAGG | 696 |
| 25 | ATGTCGACG | 697 |
| 26 | GACGTCTGA | 698 |
| 27 | TACACTGCG | 699 |
| 28 | ATCGTCAGG | 700 |
| 29 | TGCACGTAC | 701 |
| 30 | GTCGTGCAT | 702 |
| 31 | GAGTGTTAC | 703 |
| 32 | AGACTGTAC | 704 |
| 33 | TGCGACTTA | 705 |
| 34 | TGTCCGTAA | 706 |
| 35 | GTAATCGAG | 707 |
| 36 | GTACCTTAG | 708 |
| 37 | ATCACGTGT | 709 |
| 38 | ACTTAGCGT | 710 |
| 39 | GTAATCGTG | 711 |
| 40 | ATGCCGTTA | 712 |
| 41 | ATAACGTGC | 713 |
| 42 | CTACGTTGT | 714 |
| 43 | TATGACGCA | 715 |
| 44 | CCGATAACA | 716 |
| 45 | ATGCGCATA | 717 |
| 46 | GATAAGCGT | 718 |
| 47 | ATATCTGCG | 719 |
| 48 | ACTTAGACG | 720 |
| 49 | ATCACCGTA | 721 |
| 50 | TAAGACACG | 722 |
| 51 | AATGCCGTA | 723 |
| 52 | AATCACGTG | 724 |
| 53 | TCGTTAGTC | 725 |
| 54 | CATCATGTC | 726 |
| 55 | TAAGACGGT | 727 |
| 56 | TGCATAGTG | 728 |
| 57 | GAGCGTTAT | 729 |
| 58 | TGCCTTACA | 730 |
| 59 | TTCGCGTTA | 731 |
| 60 | GTGTTAACG | 732 |
| 61 | GACACTGAA | 733 |
| 62 | CTGTTATCG | 734 |
| 63 | GGTCGTTAT | 735 |
| 64 | CGAGAGTAT | 736 |

**[Table 8-2]**

| No | Primer sequence | SEQ ID NO |
|---|---|---|
| 65 | ATACAGTCC | 737 |
| 66 | AATTCACGC | 738 |
| 67 | TATGTGCAC | 739 |
| 68 | GATGACGTA | 740 |
| 69 | GATGCGATA | 741 |
| 70 | GAGCGATTA | 742 |
| 71 | TGTCACAGA | 743 |
| 72 | TACTAACCG | 744 |
| 73 | CATAACGAG | 745 |
| 74 | CGTATACCT | 746 |
| 75 | TATCACGTG | 747 |
| 76 | GAACGTTAC | 748 |
| 77 | GTCGTATAC | 749 |
| 78 | ATGTCGACA | 750 |
| 79 | ATACAGCAC | 751 |
| 80 | TACTTACGC | 752 |
| 81 | AACTACGGT | 753 |
| 82 | TAGAACGGT | 754 |
| 83 | GAATGTCAC | 755 |
| 84 | TGTACGTCT | 756 |
| 85 | AACATTGCG | 757 |
| 86 | TTGAACGCT | 758 |
| 87 | AATCAGGAC | 759 |
| 88 | ATTCGCACA | 760 |
| 89 | CCATGTACT | 761 |
| 90 | TGTCCTGTT | 762 |
| 91 | TAATTGCGC | 763 |
| 92 | GATAGTGTG | 764 |
| 93 | ATAGACGCA | 765 |
| 94 | TGTACCGTT | 766 |
| 95 | ATTGTCGCA | 767 |
| 96 | GTCACGTAA | 768 |

**[Table 9-1]**

| Table 9 List of random primers (11-base primers) | | |
|---|---|---|
| No | Primer sequence | SEQ ID NO |
| 1 | TTACACTATGC | 769 |
| 2 | GCGATAGTCGT | 770 |
| 3 | CTATTCACAGT | 771 |
| 4 | AGAGTCACTGT | 772 |
| 5 | AGAGTCGAAGC | 773 |
| 6 | CTGAATATGTG | 774 |
| 7 | ACTCCACAGGA | 775 |
| 8 | ATCCTCGTAAG | 776 |
| 9 | TACCATCGCCT | 777 |
| 10 | AACGCCTATAA | 778 |
| 11 | CTGTCGAACTT | 779 |
| 12 | TCAGATGTCCG | 780 |
| 13 | CTGCTTATCGT | 781 |
| 14 | ACATTCGCACA | 782 |
| 15 | CCTTAATGCAT | 783 |
| 16 | GGCTAGCTACT | 784 |
| 17 | TTCCAGTTGGC | 785 |
| 18 | GAGTCACAAGG | 786 |
| 19 | CAGAAGGTTCA | 787 |
| 20 | TCAACGTGCAG | 788 |
| 21 | CAAGCTTACTA | 789 |
| 22 | AGAACTCGTTG | 790 |
| 23 | CCGATACAGAG | 791 |
| 24 | GTACGCTGATC | 792 |
| 25 | TCCTCAGTGAA | 793 |
| 26 | GAGCCAACATT | 794 |
| 27 | GAGATCGATGG | 795 |
| 28 | ATCGTCAGCTG | 796 |
| 29 | GAAGCACACGT | 797 |
| 30 | ATCACGCAACC | 798 |
| 31 | TCGAATAGTCG | 799 |
| 32 | TATTACCGTCT | 800 |
| 33 | CAGTCACGACA | 801 |
| 34 | TTACTCGACGT | 802 |
| 35 | GCAATGTTGAA | 803 |
| 36 | GACACGAGCAA | 804 |
| 37 | CGAGATTACAA | 805 |
| 38 | TACCGACTACA | 806 |
| 39 | ACCGTTGCCAT | 807 |
| 40 | ATGTAATCGCC | 808 |
| 41 | AAGCCTGATGT | 809 |
| 42 | AAGTAACGTGG | 810 |
| 43 | GTAGAGGTTGG | 811 |
| 44 | CTCTTGCCTCA | 812 |
| 45 | ATCGTGAAGTG | 813 |
| 46 | ACCAGCACTAT | 814 |
| 47 | CACCAGAATGT | 815 |
| 48 | GAGTGAACAAC | 816 |
| 49 | TAACGTTACGC | 817 |
| 50 | CTTGGATCTTG | 818 |
| 51 | GTTCCAACGTT | 819 |
| 52 | CAAGGACCGTA | 820 |
| 53 | GACTTCACGCA | 821 |
| 54 | CACACTACTGG | 822 |
| 55 | TCAGATGAATC | 823 |
| 56 | TATGGATCTGG | 824 |
| 57 | TCTTAGGTGTG | 825 |
| 58 | TGTCAGCGTCA | 826 |
| 59 | GTCTAGGACAG . | 827 |
| 60 | GCCTCTTCATA | 828 |
| 61 | AGAAGTGTTAC | 829 |
| 62 | CATGAGGCTTG | 830 |
| 63 | TGGATTGCTCA | 831 |
| 64 | ATCTACCTAAG | 832 |

**[Table 9-2]**

| No | Primer sequence | SEQ ID NO |
|---|---|---|
| 65 | ATGAGCAGTGA | 833 |
| 66 | CCAGGAGATAC | 834 |
| 67 | CCGTTATACTT | 835 |
| 68 | CTCAGTACAAG | 836 |
| 69 | GGTGATCGTAG | 837 |
| 70 | CGAACGAGACA | 838 |
| 71 | ACTACGAGCTT | 839 |
| 72 | TTGCCACAGCA | 840 |
| 73 | GTCAACTCTAC | 841 |
| 74 | TGGACTGTGTC | 842 |
| 75 | GGAATGGACTT | 843 |
| 76 | CGAGAACATAA | 844 |
| 77 | ACCTGGTCAGT | 845 |
| 78 | CGAACGAGACA | 846 |
| 79 | AGTCTAGCCAT | 847 |
| 80 | AGGCCTAGATG | 848 |
| 81 | GGTGCGTTAGT | 849 |
| 82 | ATTGTGTCCGA | 850 |
| 83 | GCAGACATTAA | 851 |
| 84 | ATTGGCTCATG | 852 |
| 85 | GAGGTTACATG | 853 |
| 86 | CCTATAGGACC | 854 |
| 87 | TTAGACGGTCT | 855 |
| 88 | GATTGACGCAC | 856 |
| 89 | AAGACACCTCG | 857 |
| 90 | TCGAATAATCG | 858 |
| 91 | TCTATGTCGGA | 859 |
| 92 | TCGCATGAACC | 860 |
| 93 | TGTTATGTCTC | 861 |
| 94 | TGGATCCTACA | 862 |
| 95 | ATCGTTCAGCC | 863 |
| 96 | TACCGCAAGCA | 864 |

**[Table 10-1]**

| Table 10 List of random primers (12-base primers) | | |
|---|---|---|
| No | Primer sequence | SEQ ID NO |
| 1 | GCTGTTGAACCG | 865 |
| 2 | ATACTCCGAGAT | 866 |
| 3 | CTTAAGGAGCGC | 867 |
| 4 | TATACTACAAGC | 868 |
| 5 | TAGTGGTCGTCA | 869 |
| 6 | GTGCTTCAGGAG | 870 |
| 7 | GACGCATACCTC | 871 |
| 8 | CCTACCTGTGGA | 872 |
| 9 | GCGGTCACATAT | 873 |
| 10 | CTGCATTCACGA | 874 |
| 11 | TGGATCCTTCAT | 875 |
| 12 | TTGTGCTGGACT | 876 |
| 13 | ATTGAGAGCTAT | 877 |
| 14 | TCGCTAATGTAG | 878 |
| 15 | CTACTGGCACAA | 879 |
| 16 | AGAGCCAGTCGT | 880 |
| 17 | AATACTGGCTAA | 881 |
| 18 | CTGCATGCATAA | 882 |
| 19 | TTGTCACAACTC | 883 |
| 20 | TGCTAACTCTCC | 884 |
| 21 | TCTCTAGTTCGG | 885 |
| 22 | TTACGTCCGCAA | 886 |
| 23 | GTGTTGCTACCA | 887 |
| 24 | CGCATGTATGCC | 888 |
| 25 | CCTGTTCTGATT | 889 |
| 26 | TAAGATGCTTGA | 890 |
| 27 | ATATATCTCAGC | 891 |
| 28 | TTCCTCGTGGTT | 892 |
| 29 | ATGTCGATCTAG | 893 |
| 30 | CATCCACTAATC | 894 |
| 31 | GCCTCTGGTAAC | 895 |
| 32 | AGTCAAGAGATT | 896 |
| 33 | ACTGAGGCGTTC | 897 |
| 34 | TAAGGCTGACAT | 898 |
| 35 | AGTTCGCATACA | 899 |
| 36 | GCAGAATTGCGA | 900 |
| 37 | GGTTATGAAGAA | 901 |
| 38 | AGAAGTCGCCTC | 902 |
| 39 | TTCGCGTTATTG | 903 |
| 40 | TACCTGGTCGGT | 904 |
| 41 | GGTTACCGAGGA | 905 |
| 42 | ACACACTTCTAG | 906 |
| 43 | GGAAGTGATTAA | 907 |
| 44 | TCCATCAGATAA | 908 |
| 45 | TGTCTGTATCAT | 909 |
| 46 | AATTGGCTATAG | 910 |
| 47 | ACGTCGGAAGGT | 911 |
| 48 | AGGCATCCGTTG | 912 |
| 49 | ACCGTCGCTTGA | 913 |
| 50 | TACCGTCAAGTG | 914 |
| 51 | CTCGATATAGTT | 915 |
| 52 | CGTCAACGTGGT | 916 |
| 53 | TAGTCAACGTAG | 917 |
| 54 | TGAGTAGGTCAG | 918 |
| 55 | CTTGGCATGTAC | 919 |
| 56 | TGCCGAGACTTC | 920 |
| 57 | CTAAGACTTAAG | 921 |
| 58 | TTCTCGTGTGCG | 922 |
| 59 | CACCTGCACGAT | 923 |
| 60 | ATTAAGCCTAAG | 924 |
| 61 | GGTGGAACCATG | 925 |
| 62 | ACTAACGCGACT | 926 |
| 63 | CAGTTGTGCTAT | 927 |
| 64 | ACGCTGTTAGCA | 928 |

**[Table 10-2]**

| No | Primer sequence | SEQ ID NO |
|---|---|---|
| 65 | GTCAACGCTAAG | 929 |
| 66 | AGCTTAGGTATG | 930 |
| 67 | CGCAGGACGATT | 931 |
| 68 | AACCGGCTGTCT | 932 |
| 69 | GTTGCTCACGTG | 933 |
| 70 | GAATCTTCCGCG | 934 |
| 71 | AGAGCGTACACG | 935 |
| 72 | AAGGCTAATGTC | 936 |
| 73 | TCTATGTAGACG | 937 |
| 74 | AGACGGTCTAGT | 938 |
| 75 | TTGGTCACACGC | 939 |
| 76 | GTCGATATATGG | 940 |
| 77 | AACATGGATACG | 941 |
| 78 | TTCGCAGTTCCT | 942 |
| 79 | CGCATGTTGTGC | 943 |
| 80 | TGTTAAGTTGGA | 944 |
| 81 | CAAGTGTGATGA | 945 |
| 82 | CTGGTACCACGT | 946 |
| 83 | CGCTAGGATCAC | 947 |
| 84 | TGCTCATTACGG | 948 |
| 85 | TGCTCAGTAACA | 949 |
| 86 | ACGATCATAGCC | 950 |
| 87 | ACGATACGTGGA | 951 |
| 88 | GTTCGATGATGG | 952 |
| 89 | AAGAGCTGTGCC | 953 |
| 90 | GGTTGGATCAAC | 954 |
| 91 | GCGCGCTTATGA | 955 |
| 92 | CGTCGATCATCA | 956 |
| 93 | GAGACTGCACTC | 957 |
| 94 | GATAGATCGCAT | 958 |
| 95 | GGCCATCATCAG | 959 |
| 96 | GGTGTTCCACTG | 960 |

**[Table 11-1]**

| Table 11 List of random primers (14-base primers) | | |
|---|---|---|
| No | Primer sequence | SEQ ID NO |
| 1 | AGCTATACAGAGGT | 961 |
| 2 | AGGCCGTTCTGTCT | 962 |
| 3 | CATTGGTCTGCTAT | 963 |
| 4 | CTACATACGCGCCA | 964 |
| 5 | GCTTAACGGCGCTT | 965 |
| 6 | TACGATACTCCACC | 966 |
| 7 | ACCGGCATAAGAAG | 967 |
| 8 | GGATGCTTCGATAA | 968 |
| 9 | GTGTACCTGAATGT | 969 |
| 10 | CGCGGATACACAGA | 970 |
| 11 | TTCCACGGCACTGT | 971 |
| 12 | TAGCCAGGCAACAA | 972 |
| 13 | AGCGTCAACACGTA | 973 |
| 14 | TAACGCTACTCGCG | 974 |
| 15 | TAGATAGACGATCT | 975 |
| 16 | ACTCTTGCAATGCT | 976 |
| 17 | ACTCGGTTAGGTCG | 977 |
| 18 | CATTATCTACGCAT | 978 |
| 19 | CACACCGGCGATTA | 979 |
| 20 | TACGCAGTACTGTG | 980 |
| 21 | CAAGCGCGTGAATG | 981 |
| 22 | GAATGGACTGACGA | 982 |
| 23 | CTAGCGCTGAAGTT | 983 |
| 24 | TGCGGCAGACCAAT | 984 |
| 25 | AAGGCATAGAGATT | 985 |
| 26 | TTCTCCTCGCCATG | 986 |
| 27 | TCATTGGTCGTGAA | 987 |
| 28 | ATTACGCTATACGA | 988 |
| 29 | ATGATCCTCCACGG | 989 |
| 30 | CGTCGTTAGTAATC | 990 |
| 31 | TGCACATAGTCTCA | 991 |
| 32 | GTCAAGGAGTCACG | 992 |
| 33 | GGTTGGAATCTTGC | 993 |
| 34 | CATCGGTGCACTCA | 994 |
| 35 | AATGCACTAGACGT | 995 |
| 36 | TACAGTCAGGCTCG | 996 |
| 37 | AGAGAAGCTTAGCC | 997 |
| 38 | CCATAGGATCGTAT | 998 |
| 39 | TTGTGCTACACCTG | 999 |
| 40 | CTCCAGTAATACTA | 1000 |
| 41 | TGATGCCGATGTGG | 1001 |
| 42 | GTCATACCGCTTAA | 1002 |
| 43 | ACGTTCTCTTGAGA | 1003 |
| 44 | CAGCCATATCGTGT | 1004 |
| 45 | TTGAACGTAGCAAT | 1005 |
| 46 | ACAATCGCGGTAAT | 1006 |
| 47 | GTTCCTGTAGATCC | 1007 |
| 48 | AGAGCCTTACGGCA | 1008 |
| 49 | AATATGGCGCCACC | 1009 |
| 50 | ACCATATAGGTTCG | 1010 |
| 51 | ATGCACCACAGCTG | 1011 |
| 52 | CTACTATTGAACAG | 1012 |
| 53 | TGCCATCACTCTAG | 1013 |
| 54 | GCGAACGAGAATCG | 1014 |
| 55 | GAATCAAGGAGACC | 1015 |
| 56 | CAACATCTATGCAG | 1016 |
| 57 | CAATCCGTCATGGA | 1017 |
| 58 | AGCTCTTAGCCATA | 1018 |
| 59 | AACAAGGCAACTGG | 1019 |
| 60 | GTCGTCGCTCCTAT | 1020 |
| 61 | GTCATCATTAGATG | 1021 |
| 62 | GCACTAAGTAGCAG | 1022 |
| 63 | ACCTTACCGGACCT | 1023 |
| 64 | GCTCAGGTATGTCA | 1024 |

**[Table 11-2]**

| No | Primer sequence | SEQ ID NO |
|---|---|---|
| 65 | TGTCACGAGTTAGT | 1025 |
| 66 | CAGATGACTTACGT | 1026 |
| 67 | GAAGTAGCGATTGA | 1027 |
| 68 | GCAGGCAATCTGTA | 1028 |
| 69 | CCTTATACAACAAG | 1029 |
| 70 | CCTTAGATTGATTG | 1030 |
| 71 | AGCCACGAGTGATA | 1031 |
| 72 | GGATGACTCGTGAC | 1032 |
| 73 | CTTCGTTCGCCATT | 1033 |
| 74 | TCTTGCGTATTGAT | 1034 |
| 75 | CTTAACGTGGTGGC | 1035 |
| 76 | TGCTGTTACGGAAG | 1036 |
| 77 | CTGAATTAGTTCTC | 1037 |
| 78 | CCTCCAAGTACAGA | 1038 |
| 79 | CTGGTAATTCGCGG | 1039 |
| 80 | CGACTGCAATCTGG | 1040 |
| 81 | TGGATCGCGATTGG | 1041 |
| 82 | CGACTATTCCTGCG | 1042 |
| 83 | CAAGTAGGTCCGTC | 1043 |
| 84 | AGTAATCAGTGTTC | 1044 |
| 85 | TTATTCTCACTACG | 1045 |
| 86 | CATGTCTTCTTCGT | 1046 |
| 87 | AGGCACATACCATC | 1047 |
| 88 | AGGTTAGAGGATGT | 1048 |
| 89 | CAACTGGCAAGTGC | 1049 |
| 90 | CGCTCACATAGAGG | 1050 |
| 91 | GCAATGTCGAGATC | 1051 |
| 92 | GTTCTGTGGTGCTC | 1052 |
| 93 | AAGTGATCAGACTA | 1053 |
| 94 | ATTGAAGGATTCCA | 1054 |
| 95 | ACGCCATGCTACTA | 1055 |
| 96 | CTGAAGATGTCTGC | 1056 |

**[Table 12-1]**

| Table 12 List of random primers (16-base primers) | | |
|---|---|---|
| No | Primer sequence | SEQ ID NO |
| 1 | GACAATCTCTGCCGAT | 1057 |
| 2 | GGTCCGCCTAATGTAA | 1058 |
| 3 | AGCCACAGGCAATTCC | 1059 |
| 4 | ATCTCAAGTTCTCAAC | 1060 |
| 5 | TGTAACGCATACGACG | 1061 |
| 6 | TATCTCGAATACCAGC | 1062 |
| 7 | ACCGCAACACAGGCAA | 1063 |
| 8 | GGCCAGTAACATGACT | 1064 |
| 9 | GTGAACAGTTAAGGTG | 1065 |
| 10 | CCAGGATCCGTATTGC | 1066 |
| 11 | GACCTAGCACTAGACC | 1067 |
| 12 | CGCCATCCTATTCACG | 1068 |
| 13 | AAGTGCAGTAATGGAA | 1069 |
| 14 | TCAACGCGTTCGTCTA | 1070 |
| 15 | AGCGGCCACTATCTAA | 1071 |
| 16 | CTCGGCGCCATATAGA | 1072 |
| 17 | CGATAACTTAGAAGAA | 1073 |
| 18 | CATAGGATGTGACGCC | 1074 |
| 19 | GGCTTGTCGTCGTATC | 1075 |
| 20 | CTTGTCTGAATATTAG | 1076 |
| 21 | ACAGTTCGAGTGTCGG | 1077 |
| 22 | CTCTAACCTGTGACGT | 1078 |
| 23 | CGCGCTAATTCAACAA | 1079 |
| 24 | ACTCACGAATGCGGCA | 1080 |
| 25 | AATCTTCGGCATTCAT | 1081 |
| 26 | AAGTATCAGGATCGCG | 1082 |
| 27 | AGTAACTCTGCAGACA | 1083 |
| 28 | GGATTGAACATTGTGC | 1084 |
| 29 | GTGATGCTCACGCATC | 1085 |
| 30 | CGTAGCGTAACGGATA | 1086 |
| 31 | TGCGATGCACCGTTAG | 1087 |
| 32 | CCAGTATGCTCTCAGG | 1088 |
| 33 | AATGACGTTGAAGCCT | 1089 |
| 34 | TCGATTCTATAGGAGT | 1090 |
| 35 | CGATAGGTTCAGCTAT | 1091 |
| 36 | CCATGTTGATAGAATA | 1092 |
| 37 | GAGCCACTTCTACAGG | 1093 |
| 38 | GCGAACTCTCGGTAAT | 1094 |
| 39 | GACCTGAGTAGCTGGT | 1095 |
| 40 | CGAGTCTATTAGCCTG | 1096 |
| 41 | GTAGTGCCATACACCT | 1097 |
| 42 | CCAGTGGTCTATAGCA | 1098 |
| 43 | GTCAGTGCGTTATTGC | 1099 |
| 44 | AGTGTCGGAGTGACGA | 1100 |
| 45 | AATCTCCGCTATAGTT | 1101 |
| 46 | CGAGTAGGTCTGACTT | 1102 |
| 47 | CTGTCGCTCTAATAAC | 1103 |
| 48 | GCTGTCAATATAACTG | 1104 |
| 49 | AGCTCAAGTTGAATCC | 1105 |
| 50 | AATTCATGCTCCTAAC | 1106 |
| 51 | CCAAGGTCTGGTGATA | 1107 |
| 52 | CTCCACGTATCTTGAA | 1108 |
| 53 | TAGCCGAACAACACTT | 1109 |
| 54 | AGTACACGACATATGC | 1110 |
| 55 | ACGTTCTAGACTCCTG | 1111 |
| 56 | CGACTCAAGCACTGCT | 1112 |
| 57 | TGAAGCTCACGATTAA | 1113 |
| 58 | TATCTAACGTATGGTA | 1114 |
| 59 | TATACCATGTTCCTTG | 1115 |
| 60 | TTCCTACGATGACTTC | 1116 |
| 61 | CTCTCCAATATGTGCC | 1117 |
| 62 | GAGTAGAGTCTTGCCA | 1118 |
| 63 | GCGAGATGTGGTCCTA | 1119 |
| 64 | AAGCTACACGGACCAC | 1120 |

**[Table 12-2]**

| No | Primer sequence | SEQ ID NO |
|---|---|---|
| 65 | ATACAACTGGCAACCG | 1121 |
| 66 | CGGTAGATGCTATGCT | 1122 |
| 67 | TCTTGACCGGTCATCA | 1123 |
| 68 | AGATCGTGCATGCGAT | 1124 |
| 69 | TCCTCGAGACAGCCTT | 1125 |
| 70 | TAGCCGGTACCACTTA | 1126 |
| 71 | GTAAGGCAGCGTGCAA | 1127 |
| 72 | TAGTCTGCTCCTGGTC | 1128 |
| 73 | TGGATTATAGCAGCAG | 1129 |
| 74 | AAGAATGATCAGACAT | 1130 |
| 75 | CAGCGCTATATACCTC | 1131 |
| 76 | GAGTAGTACCTCCACC | 1132 |
| 77 | GACGTGATCCTCTAGA | 1133 |
| 78 | GTTCCGTTCACTACGA | 1134 |
| 79 | TGCAAGCACCAGGATG | 1135 |
| 80 | TTAGTTGGCGGCTGAG | 1136 |
| 81 | CAGATGCAGACATACG | 1137 |
| 82 | GACGCTTGATGATTAT | 1138 |
| 83 | TGGATCACGACTAGGA | 1139 |
| 84 | CTCGTCGGTATAACGC | 1140 |
| 85 | AAGCACGGATGCGATT | 1141 |
| 86 | AGATCTTCCGGTGAAC | 1142 |
| 87 | GGACAATAGCAACCTG | 1143 |
| 88 | GATAATCGGTTCCAAT | 1144 |
| 89 | CTCAAGCTACAGTTGT | 1145 |
| 90 | GTTGGCATGATGTAGA | 1146 |
| 91 | CAGCATGAGGTAAGTG | 1147 |
| 92 | GCCTCATCACACGTCA | 1148 |
| 93 | TCGATACTACACATCG | 1149 |
| 94 | TACACGAGGCTTGATC | 1150 |
| 95 | TTCTCGTGTCCGCATT | 1151 |
| 96 | GGTGAAGCAACAGCAT | 1152 |

**[Table 13-1]**

| Table 13 List of random primers (18-base primers) | | |
|---|---|---|
| No | Primer sequence | SEQ ID NO |
| 1 | CGAACCGACTGTACAGTT | 1153 |
| 2 | CCGACTGCGGATAAGTTA | 1154 |
| 3 | CGACAGGTAGGTAAGCAG | 1155 |
| 4 | TGATACGTTGGTATACAG | 1156 |
| 5 | CTACTATAGAATACGTAG | 1157 |
| 6 | AGACTGTGGCAATGGCAT | 1158 |
| 7 | GGAAGACTGATACAACGA | 1159 |
| 8 | TATGCACATATAGCGCTT | 1160 |
| 9 | CATGGTAATCGACCGAGG | 1161 |
| 10 | GTCATTGCCGTCATTGCC | 1162 |
| 11 | CCTAAGAACTCCGAAGCT | 1163 |
| 12 | TCGCTCACCGTACTAGGA | 1164 |
| 13 | TATTACTGTCACAGCAGG | 1165 |
| 14 | TGAGACAGGCTACGAGTC | 1166 |
| 15 | AAGCTATGCGAACACGTT | 1167 |
| 16 | AACGGAGGAGTGAGCCAA | 1168 |
| 17 | CCACTATGGACATCATGG | 1169 |
| 18 | ATGGTGGTGGATAGCTCG | 1170 |
| 19 | TCACCGGTTACACATCGC | 1171 |
| 20 | AAGATACTGAGATATGGA | 1172 |
| 21 | GACCTGTTCTTGAACTAG | 1173 |
| 22 | AAGTAGAGCTCTCGGTTA | 1174 |
| 23 | CTATGTTCTTACTCTCTT | 1175 |
| 24 | CAAGGCTATAAGCGGTTA | 1176 |
| 25 | GAAGCTAATTAACCGATA | 1177 |
| 26 | TTCACGTCTGCCAAGCAC | 1178 |
| 27 | ATCGTATAGATCGAGACA | 1179 |
| 28 | GTCACAGATTCACATCAT | 1180 |
| 29 | GTGCCTGTGAACTATCAG | 1181 |
| 30 | CAGCGTACAAGATAGTCG | 1182 |
| 31 | GCATGGCATGGTAGACCT | 1183 |
| 32 | GGTATGCTACTCTTCGCA | 1184 |
| 33 | ATGTTCAGTCACAAGCGA | 1185 |
| 34 | TAGGAAGTGTGTAATAGC | 1186 |
| 35 | AATCCATGTAGCTGTACG | 1187 |
| 36 | CCAGATTCACTGGCATAG | 1188 |
| 37 | TTGTCTCTACGTAATATC | 1189 |
| 38 | GTGGTGCTTGTGACAATT | 1190 |
| 39 | CAGCCTACTTGGCTGAGA | 1191 |
| 40 | TACTCAATGCATCTGTGT | 1192 |
| 41 | TGTAGAGAGACGAATATA | 1193 |
| 42 | GCCTACAACCATCCTACT | 1194 |
| 43 | GCGTGGCATTGAGATTCA | 1195 |
| 44 | GCATGCCAGCTAACTGAG | 1196 |
| 45 | GCGAGTAATCCGGTTGGA | 1197 |
| 46 | GCCTCTACCAGAACGTCA | 1198 |
| 47 | GTCAGCAGAAGACTGACC | 1199 |
| 48 | GATAACAGACGTAGCAGG | 1200 |
| 49 | CAGGAGATCGCATGTCGT | 1201 |
| 50 | CTGGAAGGAATGGAGCCA | 1202 |
| 51 | ATTGGTTCTCTACCACAA | 1203 |
| 52 | CTCATTGTTGACGGCTCA | 1204 |
| 53 | TTCAGGACTGTAGTTCAT | 1205 |
| 54 | AGACCGCACTAACTCAAG | 1206 |
| 55 | GGAATATTGTGCAGACCG | 1207 |
| 56 | CCTATTACTAATAGCTCA | 1208 |
| 57 | ATGGCATGAGTACTTCGG | 1209 |
| 58 | GACACGTATGCGTCTAGC | 1210 |
| 59 | GAAGGTACGGAATCTGTT | 1211 |
| 60 | TATAACGTCCGACACTGT | 1212 |
| 61 | GCTAATACATTACCGCCG | 1213 |
| 62 | GAAGCCAACACTCCTGAC | 1214 |
| 63 | CGAATAACGAGCTGTGAT | 1215 |
| 64 | GCCTACCGATCGCACTTA | 1216 |

**[Table 13-2]**

| No | Primer sequence | SEQ ID NO |
|---|---|---|
| 65 | CTGAGGAGAATAGCCTGC | 1217 |
| 66 | CAGCATGGACAGTACTTC | 1218 |
| 67 | GGTATAGAGCCTTCCTTA | 1219 |
| 68 | CGCTCTGCATATATAGCA | 1220 |
| 69 | CGGCTCTACTATGCTCGT | 1221 |
| 70 | CCTAATGCGAAGCTCACC | 1222 |
| 71 | ACAACCGGTGAGGCAGTA | 1223 |
| 72 | TTGGTTCGAACCAACCGC | 1224 |
| 73 | ATACTAGGTTGAACTAAG | 1225 |
| 74 | GCGTTGAGAGTAACATAT | 1226 |
| 75 | AGTTGTATAATAAGCGTC | 1227 |
| 76 | GTATGATGCCGTCCAATT | 1228 |
| 77 | GGACTCTCTGAAGAGTCT | 1229 |
| 78 | GGACTCTCTTGACTTGAA | 1230 |
| 79 | GATAACAGTGCTTCGTCC | 1231 |
| 80 | GGCCATTATAGATGAACT | 1232 |
| 81 | ATAGAGAGCACAGAGCAG | 1233 |
| 82 | GTGTGAGTGTATCATAAC | 1234 |
| 83 | ATAACCTTAGTGCGCGTC | 1235 |
| 84 | CCGACTGATATGCATGGA | 1236 |
| 85 | GGATATCTGATCGCATCA | 1237 |
| 86 | CAGCATTAACGAGGCGAA | 1238 |
| 87 | GCGAGGCCTACATATTCG | 1239 |
| 88 | CGATAAGTGGTAAGGTCT | 1240 |
| 89 | AGATCCTGAGTCGAGCAA | 1241 |
| 90 | AAGATATAACGAGACCGA | 1242 |
| 91 | CCGACTGATTGAGAACGT | 1243 |
| 92 | TCGGCTTATATGACACGT | 1244 |
| 93 | AATAACGTACGCCGGAGG | 1245 |
| 94 | AACACAGCATTGCGCACG | 1246 |
| 95 | GTAGTCTGACAGCAACAA | 1247 |
| 96 | AGAATGACTTGAGCTGCT | 1248 |

**[Table 14-1]**

| Table 14 List of random primers (20-base primers) | | |
|---|---|---|
| No | Primer sequence | SEQ ID NO |
| 1 | ACTGGTAGTAACGTCCACCT | 1249 |
| 2 | AGACTGGTTGTTATTCGCCT | 1250 |
| 3 | TATCATTGACAGCGAGCTCA | 1251 |
| 4 | TGGAGTCTGAAGAAGGACTC | 1252 |
| 5 | CATCTGGACTACGGCAACGA | 1253 |
| 6 | AACTGTCATAAGACAGACAA | 1254 |
| 7 | CCTCAACATGACATACACCG | 1255 |
| 8 | CAATACCGTTCGCGATTCTA | 1256 |
| 9 | GCGTCTACGTTGATTCGGCC | 1257 |
| 10 | TGAACAGAGGCACTTGCAGG | 1258 |
| 11 | CGACTAGAACCTACTACTGC | 1259 |
| 12 | GCACCGCACGTGGAGAGATA | 1260 |
| 13 | CTGAGAGACCGACTGATGCG | 1261 |
| 14 | TCGTCCTTCTACTTAATGAT | 1262 |
| 15 | CAAGCTATACCATCCGAATT | 1263 |
| 16 | CAATACGTATAGTCTTAGAT | 1264 |
| 17 | CCATCCACAGTGACCTATGT | 1265 |
| 18 | TATCCGTTGGAGAAGGTTCA | 1266 |
| 19 | CGCCTAGGTACCTGAGTACG | 1267 |
| 20 | CAGAGTGCTCGTGTTCGCGA | 1268 |
| 21 | CGCTTGGACATCCTTAAGAA | 1269 |
| 22 | GACCGCATGATTAGTCTTAC | 1270 |
| 23 | CTTGGCCGTAGTCACTCAGT | 1271 |
| 24 | GATAGCGATATTCAGTTCGC | 1272 |
| 25 | ATCCAACACTAAGACAACCA | 1273 |
| 26 | CCATTCTGTTGCGTGTCCTC | 1274 |
| 27 | ACATTCTGTACGCTTGCAGC | 1275 |
| 28 | TGCTGAACGCCAATCGCTTA | 1276 |
| 29 | TCCTCTACAAGAATATTGCG | 1277 |
| 30 | CGACCAACGCAGCCTGATTC | 1278 |
| 31 | ATTGCGAGCTTGAGTAGCGC | 1279 |
| 32 | AAGGTGCGAGCATAGGAATC | 1280 |
| 33 | CACTTAAGTGTGATATAGAT | 1281 |
| 34 | ATCGGTATGCTGACCTAGAC | 1282 |
| 35 | TACAATCTCGAATGCAGGAT | 1283 |
| 36 | CCATATGAAGCGCAGCCGTC | 1284 |
| 37 | CGTCTCGTGGACATTCGAGG | 1285 |
| 38 | CCGAGTACAGAAGCGTGGAA | 1286 |
| 39 | TTACGTGGTCGACAGGCAGT | 1287 |
| 40 | AGCTGCAATCTGCATGATTA | 1288 |
| 41 | ACCTGCCGAAGCAGCCTACA | 1289 |
| 42 | AACATGATAACCACATGGTT | 1290 |
| 43 | ATCCGACTGATTGAATTACC | 1291 |
| 44 | TCACGCTGACTCTTATCAGG | 1292 |
| 45 | GCGCGCTCGAAGTACAACAT | 1293 |
| 46 | ACAGCCAGATGCGTTGTTCC | 1294 |
| 47 | GGAGCTCTGACCTGCAAGAA | 1295 |
| 48 | AACATTAGCCTCAAGTAAGA | 1296 |
| 49 | TGTGATTATGCCGAATGAGG | 1297 |
| 50 | GAGTAATAATCCAATCAGTA | 1298 |
| 51 | CTCCTTGGCGACAGCTGAAC | 1299 |
| 52 | TTACGCACACATACACAGAC | 1300 |
| 53 | ACGCCGTATGGCGACTTAGG | 1301 |
| 54 | AGAACGACAATTACGATGGC | 1302 |
| 55 | TGCTAACGTACCACTGCCAC | 1303 |
| 56 | CATCCAGAATGTCTATCATA | 1304 |
| 57 | GGAGAACGCCTATAGCACTC | 1305 |
| 58 | ACCTCTTGTGACGGCCAGTC | 1306 |
| 59 | TGCCATAACTTGGCATAAGA | 1307 |
| 60 | ACAATTGTCTGACCACGCTC | 1308 |
| 61 | TCGTCACCTTCACAGAACGA | 1309 |
| 62 | AGCAGCAGATGATGATCCAA | 1310 |
| 63 | TCGTGCCTTGGATTCCAGGA | 1311 |
| 64 | TGTTATAGCCACGATACTAT | 1312 |

**[Table 14-2]**

| No | Primer sequence | SEQ ID NO |
|---|---|---|
| 65 | AATCTCACCTGTACCTTCCG | 1313 |
| 66 | GAGTAGCGGAAGCGTTAGCG | 1314 |
| 67 | AATACTCCGGCGAGGTATAC | 1315 |
| 68 | TTCGCATCCTTGCACGAACA | 1316 |
| 69 | AACCGGCTAATACTACTGGC | 1317 |
| 70 | CTAGCATCTTAGACACCAGA | 1318 |
| 71 | TAGTTGCGTGATACAAGATA | 1319 |
| 72 | TCGTCTCGACACAGTTGGTC | 1320 |
| 73 | TCCGTTCGCGTGCGAACTGA | 1321 |
| 74 | TCTGACTCTGGTGTACAGTC | 1322 |
| 75 | ACAGCGCAATTATATCCTGT | 1323 |
| 76 | AGATCCGTACGTGAGACTAG | 1324 |
| 77 | TACATTGAAGCATCCGAACA | 1325 |
| 78 | CTCCTGAGAGATCAACGCCA | 1326 |
| 79 | TCACCTCGAATGAGTTCGTT | 1327 |
| 80 | TAGCGACTTAAGGTCCAAGC | 1328 |
| 81 | AGTACGTATTGCCGTGCAAG | 1329 |
| 82 | AGCCACGAACCGACGTCATA | 1330 |
| 83 | TGATGTGTACGCTACTACTA | 1331 |
| 84 | CCACTGTGTGCAGCAGACGA | 1332 |
| 85 | CTATTGTACAGCGAACGCTG | 1333 |
| 86 | CTCCGATATCGCACGGATCG | 1334 |
| 87 | AACTTATCGTCGGACGCATG | 1335 |
| 88 | TATCCTAATTCGTGCCGGTC | 1336 |
| 89 | ACAGCCTTCCTGTGTGGACT | 1337 |
| 90 | CCTCCGTGAGGATCGTACCA | 1338 |
| 91 | GCTCTAAGTAACAGAACTAA | 1339 |
| 92 | GACTTACCGCGCGTTCTGGT | 1340 |
| 93 | TCTGAGGATACACATGTGGA | 1341 |
| 94 | TGTAATCACACTGGTGTCGG | 1342 |
| 95 | CACTAGGCGGCAGACATACA | 1343 |
| 96 | CTAGAGCACAGTACCACGTT | 1344 |

**[Table 15-1]**

| Table 15 List of random primers (22-base primers) | | |
|---|---|---|
| No | Primer sequence | SEQ ID NO |
| 1 | TTCAGAGGTCTACGCTTCCGGT | 1345 |
| 2 | AACACAGACTGCGTTATGCCAA | 1346 |
| 3 | TGCTGAGTTCTATACAGCAGTG | 1347 |
| 4 | ACCTATTATATGATAGCGTCAT | 1348 |
| 5 | ATCGTGAGCTACAGTGAATGCA | 1349 |
| 6 | CGTGATGTATCCGGCCTTGCAG | 1350 |
| 7 | TCTTCTGGTCCTAGAGTTGTGC | 1351 |
| 8 | TGATGTCGGCGGCGGATCAGAT | 1352 |
| 9 | TCGGCCTTAGCGTTCAGCATCC | 1353 |
| 10 | TTAAGTAGGTCAGCCACTGCAC | 1354 |
| 11 | CCAGGTGAGTTGATCTGACACC | 1355 |
| 12 | TATACTATTACTGTGTTCGATC | 1356 |
| 13 | CCGCAGTATGTCTAGTGTTGTC | 1357 |
| 14 | GTCTACCGCGTACGAAGCTCTC | 1358 |
| 15 | ATGCGAGTCCGTGGTCGATCCT | 1359 |
| 16 | TGGTAGATTGGTGTGAGAACTA | 1360 |
| 17 | AGGTTCGTCGATCAACTGCTAA | 1361 |
| 18 | ACGACAAGCATCCTGCGATATC | 1362 |
| 19 | TTGAATCACAGAGAGCGTGATT | 1363 |
| 20 | GTACTTAGTGCTTACGTCAGCT | 1364 |
| 21 | GATTATTAAGGCCAAGCTCATA | 1365 |
| 22 | GCATGCAGAGACGTACTCATCG | 1366 |
| 23 | TAGCGGATGGTGTCCTGGCACT | 1367 |
| 24 | TACGGCTGCCAACTTAATAACT | 1368 |
| 25 | CTCATATGACAACTTCTATAGT | 1369 |
| 26 | CAAGCAATAGTTGTCGGCCACC | 1370 |
| 27 | TTCAGCAATCCGTACTGCTAGA | 1371 |
| 28 | TGAGACGTTGCTGACATTCTCC | 1372 |
| 29 | GTTCCGATGAGTTAGATGTATA | 1373 |
| 30 | TTGACGCTTGGAGGAGTACAAG | 1374 |
| 31 | TTCATGTTACCTCCACATTGTG | 1375 |
| 32 | GAGCACGTGCCAGATTGCAACC | 1376 |

**[Table 15-2]**

| No | Primer sequence | SEQ ID NO |
|---|---|---|
| 33 | GGTCGACAAGCACAAGCCTTCT | 1377 |
| 34 | TAGGCAGGTAAGATGACCGACT | 1378 |
| 35 | CGAGGCATGCCAAGTCGCCAAT | 1379 |
| 36 | AGTGTTGATAGGCGGATGAGAG | 1380 |
| 37 | TTCGGTCTAGACCTCTCACAAT | 1381 |
| 38 | GTGACGCTCATATCTTGCCACC | 1382 |
| 39 | GATGTAATTCTACGCGCGGACT | 1383 |
| 40 | GATGGCGATGTTGCATTACATG | 1384 |
| 41 | TATGCTCTGAATTAACGTAGAA | 1385 |
| 42 | AGGCAATATGGTGATCCGTAGC | 1386 |
| 43 | TGACAGCGATGCATACAGTAGT | 1387 |
| 44 | TTCTGCTAACGGTATCCAATAC | 1388 |
| 45 | GAGTCGTCCATACGATCTAGGA | 1389 |
| 46 | AGACGGACTCAACGCCAATTCC | 1390 |
| 47 | GTAGTGTTGAGCGGACCGAGCT | 1391 |
| 48 | AATATAACTAGATCATAGCCAG | 1392 |
| 49 | TCAATCGGAGAATACAGAACGT | 1393 |
| 50 | ATCTCCGTCGTCCGAACCAACA | 1394 |
| 51 | TAGGCGTTCAGCGGTATGCTTA | 1395 |
| 52 | TGCGTGCTATACAACCTATACG | 1396 |
| 53 | ATGGCCGGCATACATCTGTATG | 1397 |
| 54 | TGATGCTGACATAACACTGAAT | 1398 |
| 55 | ATCCAAGGTACCTGAACATCCT | 1399 |
| 56 | TAGTGACGACCAGGTGAGCCTC | 1400 |
| 57 | AGGAGGATCCGTCAAGTCGACC | 1401 |
| 58 | AGAGTATGCCAGATCGTGAGGC | 1402 |
| 59 | CCACTCACTAGGATGGCTGCGT | 1403 |
| 60 | TATCCAACCTGTTATAGCGATT | 1404 |
| 61 | TCTTGCAGTGAGTTGAGTCTGC | 1405 |
| 62 | CCACTGTTGTACATACACCTGG | 1406 |
| 63 | ATGCGCGTAGGCCACTAAGTCC | 1407 |
| 64 | ACAGCGGTCTACAACCGACTGC | 1408 |

**[Table 15-3]**

| No | Primer sequence | SEQ ID NO |
|---|---|---|
| 65 | TCGCGCTCCAGACAATTGCAGC | 1409 |
| 66 | CCGGTAGACCAGGAGTGGTCAT | 1410 |
| 67 | ATCTCCTAACCTAGAGCCATCT | 1411 |
| 68 | CCACATCGAATCTAACAACTAC | 1412 |
| 69 | TAGTCTTATTGAATACGTCCTA | 1413 |
| 70 | TCCTTAAGCCTTGGAACTGGCG | 1414 |
| 71 | CCGTGATGGATTGACGTAGAGG | 1415 |
| 72 | GCCTGGATAACAGATGTCTTAG | 1416 |
| 73 | CTCGACCTATAATCTTCTGCCA | 1417 |
| 74 | AGCTACTTCTCCTTCCTAATCA | 1418 |
| 75 | ACACGCTATTGCCTTCCAGTTA | 1419 |
| 76 | AAGCCTGTGCATGCAATGAGAA | 1420 |
| 77 | TCGTTGGTTATAGCACAACTTC | 1421 |
| 78 | GCGATGCCTTCCAACATACCAA | 1422 |
| 79 | CCACCGTTAGCACGTGCTACGT | 1423 |
| 80 | GTTACCACAATGCCGCCATCAA | 1424 |
| 81 | GGTGCATTAAGAACGAACTACC | 1425 |
| 82 | TCCTTCCGGATAATGCCGATTC | 1426 |
| 83 | AACCGCAACTTCTAGCGGAAGA | 1427 |
| 84 | TCCTTAAGCAGTTGAACCTAGG | 1428 |
| 85 | TACTAAGTCAGATAAGATCAGA | 1429 |
| 86 | TTCGCCATAACTAGATGAATGC | 1430 |
| 87 | AAGAAGTTAGACGCGGTGGCTG | 1431 |
| 88 | GTATCTGATCGAAGAGCGGTGG | 1432 |
| 89 | TCAAGAGCTACGAAGTAAGTCC | 1433 |
| 90 | CGAGTACACAGCAGCATACCTA | 1434 |
| 91 | CTCGATAAGTTACTCTGCTAGA | 1435 |
| 92 | ATGGTGCTGGTTCTCCGTCTGT | 1436 |
| 93 | TCAAGCGGTCCAAGGCTGAGAC | 1437 |
| 94 | TGTCCTGCTCTGTTGCTACCGT | 1438 |
| 95 | AGTCATATCGCGTCACACGTTG | 1439 |
| 96 | GGTGAATAAGGACATGAGAAGC | 1440 |

**[Table 16-1]**

| Table 16 List of random primers (24-base primers | | |
|---|---|---|
| No | Primer sequence | SEQ ID NO |
| 1 | CCTGATCTTATCTAGTAGAGACTC | 1441 |
| 2 | TTCTGTGTAGGTGTGCCAATCACC | 1442 |
| 3 | GACTTCCAGATGCTTAAGACGACA | 1443 |
| 4 | GTCCTTCGACGGAGAACATCCGAG | 1444 |
| 5 | CTTGGTTAGTGTACCGTCAACGTC | 1445 |
| 6 | AAGCGGCATGTGCCTAATCGACGT | 1446 |
| 7 | CGACCGTCGTTACACGGAATCCGA | 1447 |
| 8 | TCGCAAGTGTGCCGTTCTGTTCAT | 1448 |
| 9 | CGTACTGAAGTTCGGAGTCGCCGT | 1449 |
| 10 | CCACTACAGAATGGTAGCAGATCA | 1450 |
| 11 | AGTAGGAGAGAGGCCTACACAACA | 1451 |
| 12 | AGCCAAGATACTCGTTCGGTATGG | 1452 |
| 13 | GTTCCGAGTACATTGAATCCTGGC | 1453 |
| 14 | AGGCGTACGAGTTATTGCCAGAGG | 1454 |
| 15 | GTGGCATCACACATATCTCAGCAT | 1455 |
| 16 | GAGACCGATATGTTGATGCCAGAA | 1456 |
| 17 | CAACTGTAGCCAGTCGATTGCTAT | 1457 |
| 18 | TATCAATGCAATGAGAGGATGCAG | 1458 |
| 19 | GTATGCTCGGCTCCAAGTACTGTT | 1459 |
| 20 | AGAGACTCTTATAGGCTTGACGGA | 1460 |
| 21 | ACTTAACAGATATGGATCATCGCC | 1461 |
| 22 | AATCAGAGCGAGTCTCGCTTCAGG | 1462 |
| 23 | ACCACCGAGGAACAGGTGCGACAA | 1463 |
| 24 | TGGTACATGTCAACCGTAAGCCTG | 1464 |
| 25 | CGTGCCGCGGTGTTCTTGTATATG | 1465 |
| 26 | GACAAGCGCGCGTGAGACATATCA | 1466 |
| 27 | AGTGCACTCCGAACAAGAGTTAGT | 1467 |
| 28 | CCTCATTACCGCGTTAGGAGTCCG | 1468 |
| 29 | TGCTTATTGCTTAGTTGCTATCTC | 1469 |
| 30 | GCGTGATCCTGTTCTATTCGTTAG | 1470 |
| 31 | GGCCAGAACTATGACGAGTATAAG | 1471 |
| 32 | GATGGCGACTATCTAATTGCAATG | 1472 |

**[Table 16-2]**

| No | Primer sequence | SEQ ID NO |
|---|---|---|
| 33 | TAGTAACCATAGCTCTGTACAACT | 1473 |
| 34 | CGTGATCGCCAATACACATGTCGC | 1474 |
| 35 | TAATAACGGATCGATATGCACGCG | 1475 |
| 36 | ATCATCGCGCTAATACTATCTGAA | 1476 |
| 37 | CACGTGCGTGCAGGTCACTAGTAT | 1477 |
| 38 | AGGTCCAATGCCGAGCGATCAGAA | 1478 |
| 39 | CAGCATAACAACGAGCCAGGTCAG | 1479 |
| 40 | ATGGCGTCCAATACTCCGACCTAT | 1480 |
| 41 | AGGAACATCGTGAATAATGAAGAC | 1481 |
| 42 | TCTCGACGTTCATGTAATTAAGGA | 1482 |
| 43 | TCGCGGTTAACCTTACTTAGACGA | 1483 |
| 44 | ATCATATCTACGGCTCTGGCGCCG | 1484 |
| 45 | GCAGATGGAGACCAGAGGTACAGG | 1485 |
| 46 | AGACAGAAGATTACCACGTGCTAT | 1486 |
| 47 | CCACGGACAACATGCCGCTTAACT | 1487 |
| 48 | CTTGAAGTCTCAAGCTATGAGAGA | 1488 |
| 49 | ACAGCAGTCGTGCTTAGGTCACTG | 1489 |
| 50 | AGGTGTTAATGAACGTAGGTGAGA | 1490 |
| 51 | AGCCACTATGTTCAAGGCTGAGCC | 1491 |
| 52 | GCAGGCGGTGTCGTGTGACAATGA | 1492 |
| 53 | AGCCATTGCTACAGAGGTTACTTA | 1493 |
| 54 | ACAATCGAACCTACACTGAGTCCG | 1494 |
| 55 | CCGATCTCAATAGGTACCACGAAC | 1495 |
| 56 | GATACGTGGCGCTATGCTAATTAA | 1496 |
| 57 | AGAGAGATGGCACACATTGACGTC | 1497 |
| 58 | CTCAACTCATCCTTGTAGCCGATG | 1498 |
| 59 | GTGGAATAACGCGATACGACTCTT | 1499 |
| 60 | ATCTACCATGCGAATGCTCTCTAG | 1500 |
| 61 | ATACGCACGCCTGACACAAGGACC | 1501 |
| 62 | GTCCACTCTCAGTGTGTAGAGTCC | 1502 |
| 63 | AATATATCCAGATTCTCTGTGCAG | 1503 |
| 64 | CCTTCCGCCACATGTTCGACAAGG | 1504 |

**[Table 16-3]**

| No | Primer sequence | SEQ ID NO |
|---|---|---|
| 65 | ACTGTGCCATCATCCGAGGAGCCA | 1505 |
| 66 | TCTATGCCGCTATGGCGTCGTGTA | 1506 |
| 67 | CGTAACCTAAGGTAATATGTCTGC | 1507 |
| 68 | TACTGACCGTATCAAGATTACTAA | 1508 |
| 69 | TCATCGGAGCGCCATACGGTACGT | 1509 |
| 70 | GCAAGAGGAATGAACGAAGTGATT | 1510 |
| 71 | GGCTGATTGACATCCTGACTTAGT | 1511 |
| 72 | AAGGCGCTAGATTGGATTAACGTA | 1512 |
| 73 | GCTAGCTAGAAGAATAGGATTCGT | 1513 |
| 74 | CAGGTGACGGCCTCTATAACTCAT | 1514 |
| 75 | CAGGTTACACATACCACTATCTTC | 1515 |
| 76 | TTGCTACGTACCGTCTTAATCCGT | 1516 |
| 77 | CTCAACATGTCTTGCAAGCTTCGA | 1517 |
| 78 | GGTGCGGTACGTAGAACCAGATCA | 1518 |
| 79 | AATGCTCTCCAAGATCCTGACCTA | 1519 |
| 80 | GCTTCGCAGGTCTGGATGATGGAG | 1520 |
| 81 | ACATTGACCAGACAGCACCTTGCG | 1521 |
| 82 | AGGTATCAATGTGCTTAATAGGCG | 1522 |
| 83 | TCCGGACACACGATTAGTAACGGA | 1523 |
| 84 | TACGAAGTACTACAGATCGGTCAG | 1524 |
| 85 | AATTGTCAGACGAATACTGCTGGA | 1525 |
| 86 | TGAATCATGAGCCAGAGGTTATGC | 1526 |
| 87 | CACAAGACACGTCATTAACATCAA | 1527 |
| 88 | GAATGACTACATTACTCCGCCAGG | 1528 |
| 89 | AGCCAGAGATACTGGAACTTGACT | 1529 |
| 90 | TATCAGACACATCACAATGGATAC | 1530 |
| 91 | CTAGGACACCGCTAGTCGGTTGAA | 1531 |
| 92 | GTATAACTGCGTGTCCTGGTGTAT | 1532 |
| 93 | ATGCAATACTAAGGTGGACCTCCG | 1533 |
| 94 | ATGCAGACGCTTGCGATAAGTCAT | 1534 |
| 95 | TTGCTCGATACACGTAGACCAGTG | 1535 |
| 96 | TACTGGAGGACGATTGTCTATCAT | 1536 |

**[Table 17-1]**

| Table 17 List of random primers (26-base primers) | | |
|---|---|---|
| No | Primer sequence | SEQ ID NO |
| 1 | ACTAAGGCACGCTGATTCGAGCATTA | 1537 |
| 2 | CGGATTCTGGCACGTACAAGTAGCAG | 1538 |
| 3 | TTATGGCTCCAGATCTAGTCACCAGC | 1539 |
| 4 | CATACACTCCAGGCATGTATGATAGG | 1540 |
| 5 | AGTTGTAAGCCAACGAGTGTAGCGTA | 1541 |
| 6 | GTATCAGCTCCTTCCTCTGATTCCGG | 1542 |
| 7 | AACATACAGAATGTCTATGGTCAGCT | 1543 |
| 8 | GACTCATATTCATGTTCAGTATAGAG | 1544 |
| 9 | AGAGTGAACGAACGTGACCGACGCTC | 1545 |
| 10 | AATTGGCGTCCTTGCCACAACATCTT | 1546 |
| 11 | TCGTAGACGCCTCGTACATCCGAGAT | 1547 |
| 12 | CCGGCTCGTGAGGCGATAATCATATA | 1548 |
| 13 | AGTCCTGATCACGACCACGACTCACG | 1549 |
| 14 | GGCACTCAATCCTCCATGGAGAAGCT | 1550 |
| 15 | TCATCATTCCTCACGTTCACCGGTGA | 1551 |
| 16 | TCAACTCTGTGCTAACCGGTCGTACA | 1552 |
| 17 | TGTTCTTATGCATTAATGCCAGGCTT | 1553 |
| 18 | GATTCACGACCTCAACAGCATCACTC | 1554 |
| 19 | GGCGAGTTCGACCAGAATGCTGGACA | 1555 |
| 20 | TTCCGTATACAATGCGATTAAGATCT | 1556 |
| 21 | GAGTAATCCGTAACCGGCCAACGTTG | 1557 |
| 22 | CGCTTCCATCATGGTACGGTACGTAT | 1558 |
| 23 | CCGTCGTGGTGTGTTGACTGGTCAAC | 1559 |
| 24 | TATTCGCATCTCCGTATTAGTTGTAG | 1560 |
| 25 | TATTATTGTATTCTAGGCGGTGCAAC | 1561 |
| 26 | AGGCTGCCTACTTCCTCGTCATCTCG | 1562 |
| 27 | GTAACATACGGCTCATCGAATGCATC | 1563 |
| 28 | TTATGGCACGGATATTACCGTACGCC | 1564 |
| 29 | ATAGCACTTCCTCTAATGCTCTGCTG | 1565 |
| 30 | TCACAGGCAATAGCCTAATATTATAT | 1566 |
| 31 | GGCGGATGTTCGTTAATATTATAAGG | 1567 |
| 32 | TGCAATAGCCGTTGTCTCTGCCAGCG | 1568 |

**[Table 17-2]**

| No | Primer sequence | SEQ ID NO |
|---|---|---|
| 33 | TACAGCGCGTTGGCGAGTACTGATAG | 1569 |
| 34 | TGCAGTTAGTACCTTCTCACGCCAAC | 1570 |
| 35 | CCATTGGCTACCTAGCAGACTCTACC | 1571 |
| 36 | AACAGTAGCTCGCGTCTTGCTCTCGT | 1572 |
| 37 | GCAGTCCATCAGCTCTCGCTTATAGA | 1573 |
| 38 | TATCTCTCTGTCGCCAGCTTGACCAA | 1574 |
| 39 | CAGACTGTTCAAGCTTGCTGTAGGAG | 1575 |
| 40 | TAACCGGAACTCGTTCAGCAACATTC | 1576 |
| 41 | TCAATTATGCATGTCGTCCGATCTCT | 1577 |
| 42 | TTGTCTAAGTCAACCTGTGGATAATC | 1578 |
| 43 | TCTAAGAGTGGTATGACCAGGAGTCC | 1579 |
| 44 | TCGTAGTACTACTGGAACAGGTAATC | 1580 |
| 45 | ATGTCAACATTCTAATCATCTCTCGG | 1581 |
| 46 | AGCGCGCAACTGTTACGGTGATCCGA | 1582 |
| 47 | GCGATAGAATAATGGTGTCACACACG | 1583 |
| 48 | AAGGCTGCGATGAGAGGCGTACATCG | 1584 |
| 49 | GGTTCATGGTCTCAGTCGTGATCGCG | 1585 |
| 50 | TAGTGACTCTATGTCACCTCGGAGCC | 1586 |
| 51 | ATGTGATAGCAATGGCACCTCTAGTC | 1587 |
| 52 | TCGCGAAGTGTAATGCATCATCCGCT | 1588 |
| 53 | ATGTGGCGACGATCCAAGTTCAACGC | 1589 |
| 54 | ACCTTGTATGAGTCGGAGTGTCCGGC | 1590 |
| 55 | ACCTCAAGAGAGTAGACAGTTGAGTT | 1591 |
| 56 | GGTGTAATCCTGTGTGCGAAGCTGGT | 1592 |
| 57 | ATAGCGGAACTGTACGACGCTCCAGT | 1593 |
| 58 | AAGCACGAGTCGACCATTAGCCTGGA | 1594 |
| 59 | ATTCCGGTAACATCAGAAGGTACAAT | 1595 |
| 60 | GTGCAACGGCAGTCCAGTATCCTGGT | 1596 |
| 61 | CCATCTTATACACGGTGACCGAAGAT | 1597 |
| 62 | GCACTTAATCAAGCTTGAGTGATGCT | 1598 |
| 63 | AGTATTACGTGAGTACGAAGATAGCA | 1599 |
| 64 | TTCTTAGGTTAAGTTCCTTCTGGACC | 1600 |

**[Table 17-3]**

| No | Primer sequence | SEQ ID NO |
|---|---|---|
| 65 | GTCCTTGCTAGACACTGACCGTTGCT | 1601 |
| 66 | GCCGCTATGTGTGCTGCATCCTAAGC | 1602 |
| 67 | CCATCAATAACAGACTTATGTTGTGA | 1603 |
| 68 | CGCGTGTGCTTACAAGTGCTAACAAG | 1604 |
| 69 | CGATATGTGTTCGCAATAAGAGAGCC | 1605 |
| 70 | CGCGGATGTGAGCGGCTCAATTAGCA | 1606 |
| 71 | GCTGCATGACTATCGGATGGAGGCAT | 1607 |
| 72 | CTATGCCGTGTATGGTACGAGTGGCG | 1608 |
| 73 | CCGGCTGGAGTTCATTACGTAGGCTG | 1609 |
| 74 | TGTAGGCCTACTGAGCTAGTATTAGA | 1610 |
| 75 | CCGTCAAGTGACTATTCTTCTAATCT | 1611 |
| 76 | GGTCTTACGCCAGAGACTGCGCTTCT | 1612 |
| 77 | CGAAGTGTGATTATTAACTGTAATCT | 1613 |
| 78 | GCACGCGTGGCCGTAAGCATCGATTA | 1614 |
| 79 | ATCCTGCGTCGGAACGTACTATAGCT | 1615 |
| 80 | AGTATCATCATATCCATTCGCAGTAC | 1616 |
| 81 | AGTCCTGACGTTCATATATAGACTCC | 1617 |
| 82 | CTTGCAGTAATCTGAATCTGAAGGTT | 1618 |
| 83 | ATAACTTGGTTCCAGTAACGCATAGT | 1619 |
| 84 | GATAAGGATATGGCTGTAGCGAAGTG | 1620 |
| 85 | GTGGAGCGTTACAGACATGCTGAACA | 1621 |
| 86 | CGCTTCCGGCAGGCGTCATATAAGTC | 1622 |
| 87 | ATAACATTCTAACCTCTATAAGCCGA | 1623 |
| 88 | ACGATCTATGATCCATATGGACTTCC | 1624 |
| 89 | TGAAGCTCAGATATCATGCCTCGAGC | 1625 |
| 90 | AGACTTCACCGCAATAACTCGTAGAT | 1626 |
| 91 | AGACTAAGACATACGCCATCACCGCT | 1627 |
| 92 | TGTAGCGTGATGTATCGTAATTCTGT | 1628 |
| 93 | TGTGCTATTGGCACCTCACGCTGACC | 1629 |
| 94 | TGTAGATAAGTATCCAGCGACTCTCT | 1630 |
| 95 | AATTCGCCAATTGTGTGTAGGCGCAA | 1631 |
| 96 | CGATTATGAGTACTTGTAGACCAGCT | 1632 |

**[Table 18-1]**

| Table 18 List of random primers (28-base primers) | | |
|---|---|---|
| No | Primer sequence | SEQ ID NO |
| 1 | TTGCAAGAACAACGTATCTCATATGAAC | 1633 |
| 2 | CACCGTGCTGTTATTACTTGGTATTCGG | 1634 |
| 3 | CACGTGTATTGTTGCACCAGAACGACAA | 1635 |
| 4 | ATGCACGTAATTACTTCCGGAGAAGACG | 1636 |
| 5 | TATGTTGTCTGATATGGTTCATGTGGCA | 1637 |
| 6 | AGCGCGACTAGTTGATGCCAACATTGTA | 1638 |
| 7 | ATAGGCAGGTCCAGGCTCGGAACAAGTC | 1639 |
| 8 | GCGGTAGTCGGTCAAGAACTAGAACCGT | 1640 |
| 9 | ACTATACACTCTAGCTATTAGGAAGCAT | 1641 |
| 10 | GATCATCTTGCTTCTCCTGTGGAGATAA | 1642 |
| 11 | CTACTACGAGTCCATAACTGATAGCCTC | 1643 |
| 12 | GCACAGACACCTGTCCTATCTAGCAGGA | 1644 |
| 13 | AAGCGAGGCGCGAAGGAGATGGAAGGAT | 1645 |
| 14 | CTGAAGACGCCAGTCTGGATAGGTGCCT | 1646 |
| 15 | GTAAGCTCTGTCCTTCGAGATTGATAAG | 1647 |
| 16 | GGTTAGAGAGATTATTGTGCGCATCCAT | 1648 |
| 17 | CCAGGAGGACCTATGATCTTGCCGCCAT | 1649 |
| 18 | ACTATTCGAGCTACTGTATGTGTATCCG | 1650 |
| 19 | GACATCGCGATACGTAACTCCGGAGTGT | 1651 |
| 20 | CCGCAATTCGTCTATATATTCTAGCATA | 1652 |
| 21 | CTACACTTGAGGTTGATGCTCAAGATCA | 1653 |
| 22 | CGATCAGTTCTAGTTCACCGCGGACAAT | 1654 |
| 23 | AAGAATGATGATTGGCCGCGAACCAAGC | 1655 |
| 24 | CACGACCGGAACTAGACTCCTACCAATT | 1656 |
| 25 | AGTTGCCTGTGAGTGAGGCTACTATCTC | 1657 |
| 26 | GATTCTTCCGATGATCATGCCACTACAA | 1658 |
| 27 | CGCTGAAGTGAACTATGCAAGCACCGCA | 1659 |
| 28 | ATTATCGTGATGGTGAGACTGAGCTCGT | 1660 |
| 29 | CGAGGCCACTCTGAGCCAGGTAAGTATC | 1661 |
| 30 | TGCCGAGGACAGCCGATCACATCTTCGT | 1662 |
| 31 | GTTGACATGAAGGTTATCGTCGATATTC | 1663 |
| 32 | GTGGTCCAGGTCAAGCTCTGATCGAATG | 1664 |

**[Table 18-2]**

| No | Primer sequence | SEQ ID NO |
|---|---|---|
| 33 | CCAGTCCGGTGTACTCAGACCTAATAAC | 1665 |
| 34 | CGAGACACTGCATGAGCGTAGTCTTATT | 1666 |
| 35 | GACGGCTTGTATACTTCTCTACGGTCTG | 1667 |
| 36 | TTAGCTGGATGGAAGCCATATTCCGTAG | 1668 |
| 37 | CAGCCTACACTTGATTACTCAACAACTC | 1669 |
| 38 | GTACGTAGTGTCACGCGCCTACGTTCGT | 1670 |
| 39 | CTACAACTTCTCAATCATGCCTCTGTTG | 1671 |
| 40 | CGAGGACAGAATTCGACATAAGGAGAGA | 1672 |
| 41 | GCCGAACGACACAGTGAGTTGATAGGTA | 1673 |
| 42 | GAACACTATATGCTGTCGCTGTCTGAGG | 1674 |
| 43 | GTTAAGTTCTTCGGCGGTCATGCTCATT | 1675 |
| 44 | TTGCTTACAGATCGCGTATCCATAGTAT | 1676 |
| 45 | GAGGACCACCTCTGCGAAGTTCACTGTG | 1677 |
| 46 | AATCCTAGCATATCGAGAACGACACTGA | 1678 |
| 47 | TGAATACTATAGCCATAGTCGACTTCCG | 1679 |
| 48 | GACATCCACGAAGCTGGTAATCGGAACC | 1680 |
| 49 | TTAGCCGTCTTAGAAGTGTCTGACCGGC | - 1681 |
| 50 | CTATTCTGCCGTAATTGATTCCTTCGTT | 1682 |
| 51 | ACGCCTCTGGTCGAAGGTAGATTAGCTC | 1683 |
| 52 | CAGCCTATTGATCGTAAGTAGATGGTCC | 1684 |
| 53 | TTAAGTGAGGTGGACAACCATCAACTTC | 1685 |
| 54 | AAGGCCTTGCGGCTAAGTAGTATTCATC | 1686 |
| 55 | TTGTGATACTAATTCTTCTCAAGAGTCA | 1687 |
| 56 | GCATTAGGTGACGACCTTAGTCCATCAC | 1688 |
| 57 | GCGGATGGACGTATACAGTGAGTCGTGC | 1689 |
| 58 | GAACATGCCAGCCTCAACTAGGCTAAGA | 1690 |
| 59 | TCCGTCATTAGAGTATGAGTGACTACTA | 1691 |
| 60 | AACACTTAGTAACCAGTTCGGACTGGAC | 1692 |
| 61 | CGCTAACTATTGCGTATATTCGCGGCTT | 1693 |
| 62 | GCCATCTACGATCTTCGGCTTATCCTAG | 1694 |
| 63 | CCTGAGAATGTTGACTAAGATCTTGTGA | 1695 |
| 64 | TCGGTTAGTCTAATCATCACGCAACGGA | 1696 |

**[Table 18-3]**

| No | Primer sequence | SEQ ID NO |
|---|---|---|
| 65 | ATTATCTATTGAAGCAGTGACAGCGATC | 1697 |
| 66 | GAGGAGAATCACGGAACACGGTCACATG | 1698 |
| 67 | GCTGCAAGCATTATGACCATGGCATCTG | 1699 |
| 68 | GAACAACCTATAACGACGTTGTGGACAA | 1700 |
| 69 | TTAATCATCGATAGACGACATGGAATCA | 1701 |
| 70 | TCGAGTGTAAGCACACTACGATCTGGAA | 1702 |
| 71 | GCTACGCACAGTCTCTGCACAGCTACAC | 1703 |
| 72 | CCTGTATGTACGTTCTGGCTAATACCTT | 1704 |
| 73 | TGAAGCACCGGTACATGGTGTATCCGGA | 1705 |
| 74 | TGCTGGAACCTAACTCGGTGATGACGAT | 1706 |
| 75 | CGCTATCTTACTGCCAAGTTCTCATATA | 1707 |
| 76 | AACGCGCGCGTATCGGCAATAATCTCAA | 1708 |
| 77 | CCATTAGGATGACCATCGACTATTAGAG | 1709 |
| 78 | TACTGCTAGACTGCGTGCATTCATGGCG | 1710 |
| 79 | CATTGCGCGCTCCACGAACTCTATTGTC | 1711 |
| 80 | GACGCGCCTAGAACTGTATAGCTCTACG | 1712 |
| 81 | CATTGCAACTTGTCGGTGATGGCAATCC | 1713 |
| 82 | TTAATGCACATGCAGTACGGCACCACAG | 1714 |
| 83 | AGCGGTACGTGGACGAGTGGTAATTAAT | 1715 |
| 84 | GACGTATTGCTATGCATTGGAAGATGCT | 1716 |
| 85 | AACACTTCGACCATTGCGCCTCAATGGT | 1717 |
| 86 | CGGTACGCTCTAGCGGTCATAAGATGCA | 1718 |
| 87 | CCTGAATAACAGCCGCGCCTAATTAGAT | 1719 |
| 88 | AAGCGTCTAATGTGCCTTAAGTCACATG | 1720 |
| 89 | GCTCTCCAAGAACCAGAAGTAAGCATCG | 1721 |
| 90 | GAGGAGAGTTGTCCGAGTGGTGTGATGT | 1722 |
| 91 | TAACGAGTGGTGCGTCTAAGCAATTGAG | 1723 |
| 92 | CCAACAGTATGCTGACATAACTATGATA | 1724 |
| 93 | GATCCTTGCCACGCCTATGAGATATCGC | 1725 |
| 94 | AACGCGCTACCGTCCTTGTGCATAGAGG | 1726 |
| 95 | CTACATGTGCCTTATAGTACAGAGGAAC | 1727 |
| 96 | CAGCCTCGTAGTTAGCGTGATTCATGCG | 1728 |

**[Table 19-1]**

| Table 19 List of random primers (29-base primers) | | |
|---|---|---|
| No | Primer sequence | SEQ ID NO |
| 1 | CTCCTCGCCGATTGAAGTGCGTAGAACTA | 1729 |
| 2 | CAGCAGGCCTCAATAGGATAAGCCAACTA | 1730 |
| 3 | GACCATCAATCTCGAAGACTACGCTCTGT | 1731 |
| 4 | GGTTGCTCCGTCTGTTCAGCACACTGTTA | 1732 |
| 5 | AATGTCGACTGGCCATTATCGCCAAGTGT | 1733 |
| 6 | GATAGCTTGCCATGCGAATGGATCTCCAG | 1734 |
| 7 | CCAGACCGGAGCCAATTGGCTGCCAATAT | 1735 |
| 8 | AACGTCGCTCCATACGTTACCTAATGCAG | 1736 |
| 9 | GAATATGACGCGAACAGTCTATTCGGATC | 1737 |
| 10 | GACGAGAATGTATTAAGGATAAGCAAGGT | 1738 |
| 11 | AAGTCGTATGAATCGCTATCACATGAGTC | 1739 |
| 12 | GTCGTGGAGACTACAATTCTCCTCACGTT | 1740 |
| 13 | GTTGCCACCGTTACACGACTATCGACAGT | 1741 |
| 14 | AGGATAGGCTACGCCTTACTCTCCTAAGC | 1742 |
| 15 | TAATCATCCTGTTCGCCTCGAGGTTGTTA | 1743 |
| 16 | GACAAGCAGTAATAATTACTGAGTGGACG | 1744 |
| 17 | TACAGCGTTACGCAGGTATATCAAGGTAG | 1745 |
| 18 | CTAACATCACTTACTATTAGCGGTCTCGT | 1746 |
| 19 | CCGCGCTTCTTGACACGTTCTCCACTAGG | 1747 |
| 20 | CAAGTAACATGAGATGCTATCGGTACATT | 1748 |
| 21 | CGACCACTAGGCTGTGACCACGATACGCT | 1749 |
| 22 | CAGGTCATGTGACGCAGTCGGCAGTCAAC | 1750 |
| 23 | ACTCCATCGTTAGTTCTTCCGCCGTGCTG | 1751 |
| 24 | CTCACCACGTATGCGTCACTCGGTTACGT | 1752 |
| 25 | TGCCTATGCTATGGACCTTGCGCGACTCT | 1753 |
| 26 | AATGAAGGTCAACGCTCTGTAGTTACGCG | 1754 |
| 27 | CACCATTGATTCATGGCTTCCATCACTGC | 1755 |
| 28 | GACACGCAAGGTAATTCGAGATTGCAGCA | 1756 |
| 29 | CACCGAGAGGAAGGTTCGATCGCTTCTCG | 1757 |
| 30 | CAGTTATCGGATTGTGATATTCACTCCTG | 1758 |
| 31 | ATACTGTAACGCCTCAACCTATGCTGACT | 1759 |
| 32 | ATCTGTCTTATTCTGGCACACTCAGACTT | 1760 |

**[Table 19-2]**

| No | Primer sequence | SEQ ID NO |
|---|---|---|
| 33 | TCCAACCGGTGACGTGCTCTTGATCCAAC | 1761 |
| 34 | CACACTCAGTTCGGCTATCTCTGCGATAG | 1762 |
| 35 | AGCTGTAAGTCAGGTCTACGACTCGTACT | 1763 |
| 36 | GTCGGCGGCACGCACAGCTAACATTCGTA | 1764 |
| 37 | ATATGGTAGCCAGCCACGTATACTGAACA | 1765 |
| 38 | TGGACAATCCGACTCTAACACAGAGGTAG | 1766 |
| 39 | TCCGCCGCTGACAGTTCAATCTATCAATT | 1767 |
| 40 | GGTTCCTTAGAATATGCACCTATCAGCGA | 1768 |
| 41 | CGGCTGTACGACATGGATCATAAGAGTGT | 1769 |
| 42 | TGCAGATGTACGCTGTGGCCAGTGGAGAG | 1770 |
| 43 | CCTACTCACTTAACAATAATCGGTTCGGT | 1771 |
| 44 | CGCTTCCTACTGCCTGTGCCGCGACATAA | 1772 |
| 45 | CTAGACCGACCGGTTATGCGCTATTGTTC | 1773 |
| 46 | TTGTGAGCACGTCTGCGGCAAGCCTATGG | 1774 |
| 47 | TCATCGGCCGGCGCTGTTGTTGTTACCAT | 1775 |
| 48 | GCGGTTAGGTGCAGTTAGGAAGACTATCA | 1776 |
| 49 | TATGCGGTCGTGAGGCGTAGCATTCTAGA | 1777 |
| 50 | CCATCTATTCGTCGAACTCTCAGCTCGTA | 1778 |
| 51 | ATCAGATCTACTGATCGCGGTAGAGTATC | 1779 |
| 52 | TACACATAGGCGGCGCAGCCTTCTAATTA | 1780 |
| 53 | TTAACCGTAGTTCTTAGCTTACGCCGCTC | 1781 |
| 54 | ACTATAGAGGACATGGCACTCCTCTTCTA | 1782 |
| 55 | CAGTTCGTATTAAGATTGAATGTAGCGGT | 1783 |
| 56 | AGTTATCGGTATCCGCTTATCCGTACGTA | 1784 |
| 57 | AGCTTATTCATACACTGCACCACAGCAAG | 1785 |
| 58 | CCGTCGGCTAGTCTATCCTCTAATTAGAA | 1786 |
| 59 | GTCCGCTTCCATGCCTGCTGTACGAACAC | 1787 |
| 60 | TCTCTTCCTCCTTCATTGTTCGCTAGCTC | 1788 |
| 61 | TCTCTTGAGCGGTCCTCATACAGGTCTGC | 1789 |
| 62 | GACCAAGTGTAGGTGATATCACCGGTACT | 1790 |
| 63 | AAGATTGTGATAGGTTGGTAGTTACCACA | 1791 |
| 64 | TCGCCTCCGAAGAGTATAGCATCGGCAGA | 1792 |

**[Table 19-3]**

| No | Primer sequence | SEQ ID NO |
|---|---|---|
| 65 | GAGGTAGTTATGAGCATCGAGGTCCTGTT | 1793 |
| 66 | GGACGCAAGATCGCAGGTACTTGTAAGCT | 1794 |
| 67 | ACTCGTACACGTCATCGTGCAGGTCTCAG | 1795 |
| 68 | TAATCCGTCAGGAGTGAGATGGCTCGACA | 1796 |
| 69 | AAGATGGTTCCGCGCATTGACTAGCAAGT | 1797 |
| 70 | TCCGCGATCTGCGGATCTTGAATGCTCAC | 1798 |
| 71 | TTCACGAGAGTCAACTGCTAGTATCCTAG | 1799 |
| 72 | TTCCAACTGGATTCTTCCAACTCCTCGAA | 1800 |
| 73 | CACTACTACTCAAGTTATACGGTGTTGAC | 1801 |
| 74 | CAACTGGATTCTCAGGATGCGTCTCTAGC | 1802 |
| 75 | TGGACTAGAGTGGAGCGATTACGTAATAT | 1803 |
| 76 | GAGGTCATTCAACTGGACTCGCCACGGAC | 1804 |
| 77 | CAGGTGTGTAACGCTGCAATCACATGAAT | 1806 |
| 78 | TATGCTGAGGTATTAGTTCTAACTATGCG | 1806 |
| 79 | CGTCTGAGTCGGATAAGGAAGGTTACCGC | 1807 |
| 80 | GTACTATCGTCGCAGGCACTATCTCTGCC | 1808 |
| 81 | GCTTCCTCCTTGCAACTTCATTGCTTCGA | 1809 |
| 82 | TGTCTACGAAGTAGAAGACACGAATAATG | 1810 |
| 83 | CCGTCATCTAAGGCAGAGTACATCCGCGA | 1811 |
| 84 | CCGGAGGCGTACTAACTGACCACAACACC | 1812 |
| 85 | AACTCGTCGCTGCCTGAATAGGTCAGAGT | 1813 |
| 86 | TTATAAGATTAATGTCGGTCAGTGTCGGA | 1814 |
| 87 | CGTCTCGATGGATCCACACGAACCTGTTG | 1815 |
| 88 | ATGCCATCATGGTCGTCCTATCTTAAGGC | 1816 |
| 89 | GCGCTTCAGCGATTCGTCATGCAAGGCAC | 1817 |
| 90 | CCAAGCGATACCGAGGTACGGTTAACGAG | 1818 |
| 91 | ATATGACAGACAGGTGGACCTAAGCAAGC | 1819 |
| 92 | CACTACATCGTCAGGCCTGGAAGCCTCAG | 1820 |
| 93 | GCCGTGTAGACGAGGACATTATGTCGTAT | 1821 |
| 94 | CAACGTATATACACACCTTGTGAAGAGAA | 1822 |
| 95 | TCCAACGTAATTCCGCCGTCTGTCGAGAC | 1823 |
| 96 | AATTCGTGCTTCGATCACCGTAGACTCAG | 1824 |

**[Table 20-1]**

| Table 20 List of random primers (30-base primers) | | |
|---|---|---|
| No | Primer sequence | SEQ ID NO |
| 1 | ACTATATTGTATTCACGTCCGACGACTCGC | 1825 |
| 2 | GACGAGCTTGTGGTACACTATACCTATGAG | 1826 |
| 3 | TGATTCAAGCACCAGGCATGCTTAAGCTAG | 1827 |
| 4 | CGGTCTCCTATAGGAAGGCTCATTCTGACG | 1828 |
| 5 | AGTCAGTGTCGAATCAATCAAGGCGTCCTT | 1829 |
| 6 | CGAACGTAATGGCCATCACGCGCTGGCCTA | 1830 |
| 7 | CGAACCTGGACCACCTGGCATTACCATTAC | 1831 |
| 8 | ACATTAGGTTCCTGTAATGTCTTATCAACG | 1832 |
| 9 | CGTCTAATGCACCGTATCGTCTTCGCGCAT | 1833 |
| 10 | TCTATGACTTACAACGGAATCTTACTTCGT | 1834 |
| 11 | GTAACCGATCGGTACCGTCTGCTATTGTTC | 1835 |
| 12 | GGTGATTGATAAGCAACACATATTAGGAGG | 1836 |
| 13 | AATTATCGACGCTAATAGGCGAGCTGTTCA | 1837 |
| 14 | GGAGGTACATGACGAGTGGACAGACAGACC | 1838 |
| 15 | CTCTAATCCGTTATGCGGTGATGTAATCCG | 1839 |
| 16 | GCAAGCACGCGGCTTGGCGAACTTCTATGC | 1840 |
| 17 | TAGATGTAGGCCTGGTAGGCAGAGGAGTAA | 1841 |
| 18 | CCGAGTGGCGACCACACAGGTACGCATTAA | 1842 |
| 19 | GTCCTGGCTCAGATTAGTGCACTTAGTTAT | 1843 |
| 20 | GCGGTACCTACATGTTATGACTCAGACGAC | 1844 |
| 21 | TCTCTGCCAATGCTGGTCTCATCGAATCCA | 1845 |
| 22 | TCTCTACACAGCTACATACTATACTGTAAC | 1846 |
| 23 | TACGACGGACGCTGGTGGTGTAAGAGAAGG | 1847 |
| 24 | GCCTCGATATATCTACGTATAGTTCAAGTT | 1848 |
| 25 | GGCTCCTGCATTCATTGAAGGTCGGCCTTG | 1849 |
| 26 | CAGTTCGGTGATTCAAGAGAACAATGGTGG | 1850 |
| 27 | TATAACGAAGCCGGCTGGAACGGTAACTCA | 1851 |
| 28 | CTGTATCAATTCAAGTGACAGTGGCACGTC | 1852 |
| 29 | AGCAATTGCGGTTCATAGGCGTAATTATAT | 1853 |
| 30 | CATATGGACCTGGAGATCACCGTTCAGTCC | 1854 |
| 31 | GAAGGCCGTTGGTCTATCTCTTACTGGAGC | 1855 |
| 32 | GTGCGTTCATCTAGCCTAAGACGCTGACCT | 1856 |

**[Table 20-2]**

| No | Primer sequence | SEQ ID NO |
|---|---|---|
| 33 | GAGTAACTTATATCCTCTCTACGACATCGA | 1857 |
| 34 | ATTCTACGCTGATGTCTCCGCTGAACAGGA | 1858 |
| 35 | TCATCAACGTTACTCACTAGTACCACGGCT | 1859 |
| 36 | AACCATTCTTGAACGTTGAGAACCTGGTGG | 1860 |
| 37 | ACGACACCTCCGCGGAACATACCTGATTAG | 1861 |
| 38 | GCGCACTTATTGAAGTAATCTCATGGCCAA | 1862 |
| 39 | GCGCCAATTCAGCCAGTTAGCGTCTCCGTG | 1863 |
| 40 | AGCAACAAGTCGCTGTATATCGACTGGCCG | 1864 |
| 41 | CCTTACAATAGACCTCGCGGCGTTCATGCC | 1865 |
| 42 | GGATCCAACTTCAGCGAAGCACCAACGTCG | 1866 |
| 43 | GCGCCAGTTCTCGTACTCTCGAGAAGCGAC | 1867 |
| 44 | GAGTGCGGCCAATCTGGAACTCATGACGTT | 1868 |
| 45 | CCTGAGAGTGATTCGTGTCTGCGAAGATGC | 1869 |
| 46 | GTGACTGGTTAAGGCAATATTGGTCGACCG | 1870 |
| 47 | CTATCAAGCCTTACAAGGTCACGTCCACTA | 1871 |
| 48 | ACTGCGTCCTTGCGTCGGAACTCCTTGTGT | 1872 |
| 49 | TGCAACTCAGTGGCGGCGACACCAAGAGCT | 1873 |
| 50 | TTCGGTTCTACTAGGATCTCTATCTGAGCT | 1874 |
| 51 | AGCTAATCTATTAAGACAGATTAGACAGGA | 1875 |
| 52 | GGACCGCTCTTAGGTTATGCACCTGCGTAT | 1876 |
| 53 | CTCTAATACTAGTCCACAGGTTAGTACGAA | 1877 |
| 54 | ATCCATATATGCTCGTCGTCAGCCAGTGTT | 1878 |
| 55 | GCTATTACTGTGTTGATGTCCACAGGAGAA | 1879 |
| 56 | GCTACGGCGCAGATCTAGACAACTGGAAGT | 1880 |
| 57 | GCCTCTTGTGTTAGCCGAATACCAATGACC | 1881 |
| 58 | TGAGGACGATAACATTACCTCTCGAGTCGC | 1882 |
| 59 | CGATTACCAATCCGACGACTTCGCAGCAGC | 1883 |
| 60 | ATGACACGAGTCCAGTACATATGCGAAGAC | 1884 |
| 61 | GCGCTCGCATGCACTAGTGTAGACTGACGA | 1885 |
| 62 | GCACATCTCAGAATTGATGGTCTATGTCGC | 1886 |
| 63 | TTCTTCGACGCCGCGTACTAATAGGTCAAT | 1887 |
| 64 | GGAAGCGCCTCTAACAACCGATGCTTGTGG | 1888 |

**[Table 20-3]**

| No | Primer sequence | SEQ ID NO |
|---|---|---|
| 65 | CTCTAGACGCGTCGTGACTCCAATCTGTTG | 1889 |
| 66 | GTAGTTCGTCGGAGTGACCTCGTACTCACT | 1890 |
| 67 | ATGCTGTCGAGTGTCCGGCATAGAGCACAC | 1891 |
| 68 | GCGCATCTTGCAGCGTCCTGTAGTTCTGAA | 1892 |
| 69 | GCGATTGTTGAGGAACCACAGCGGCACCTA | 1893 |
| 70 | CACGCGTACTCTGCTTGCTGTGTGGTCGGT | 1894 |
| 71 | CATCCAACGCAGGACCTAGTAGTCATGCTT | 1895 |
| 72 | TTCTAGTTGTGATGAGAATCGCTAGCGTGC | 1896 |
| 73 | CATTCTGAATCTGGTCTCTCTCGATCATCC | 1897 |
| 74 | ATTAATGTAGAGGATAGTTCCGTTCTCTCC | 1898 |
| 75 | GTATCGCGCTTACGAATGAGGTGTGGCTTC | 1899 |
| 76 | GCTGGTGAGAGAGCCAGATTATCGGTGGAG | 1900 |
| 77 | GGCACGAGCAGGTAGAACTAGAACCTAGAT | 1901 |
| 78 | TGTATTATCTCGAAGCGGTGCGTTAGAGTC | 1902 |
| 79 | CACGTGTTCTAGCTACTAATGGCGTCAATT | 1903 |
| 80 | CGCGCTACATTACTTCCTACACCATGCGTA | 1904 |
| 81 | TGAGGCAACTAGTGTTCGCAAGATGACGGA | 1905 |
| 82 | TTATTATTGTCTGTGGAACGCACGCCAGTC | 1906 |
| 83 | GCTATAGTATTATCCATGAATTCCGTCGGC | 1907 |
| 84 | GTATCAATAGCTCAATTCGTCAGAGTTGTG | 1908 |
| 85 | TAGTCCATGCGTGGATATATTGAGAGCTGA | 1909 |
| 86 | GCACAGTACGACTTATAACAGGTCTAGATC | 1910 |
| 87 | ACTCAATGGTGGCACGCTCGGCGCAGCATA | 1911 |
| 88 | GTAGTACCACTCCGCCTTAGGCAGCTTAAG | 1912 |
| 89 | CGCTCAACTGATGCGTGCAACCAATGTTAT | 1913 |
| 90 | GCAGCTTGACTGCCTAGACAGCAGTTACAG | 1914 |
| 91 | GCAACTTCTTAGTACGAATTCATCGTCCAA | 1915 |
| 92 | ATCCGTATGCTGCGGCAGTGGAGGTGGCTT | 1916 |
| 93 | TGCGGATCAATCCAGTTCTGTGTACTGTGA | 1917 |
| 94 | TTATGATTATCACCGGCGTAACATTCCGAA | 1918 |
| 95 | GCTACCTAGATTCTTCAACTCATCGCTACC | 1919 |
| 96 | CAGTGTTAGAATGGCGGTGTGTAGCCGCTA | 1920 |

**[Table 21-1]**

| Table 21 List of random primers (3 5-base primers) | | |
|---|---|---|
| No | Primer sequence | SEQ ID NO |
| 1 | GCTTATAGACTACAGCTGCGAGGTATAAGGTCACT | 1921 |
| 2 | CGCTCAGCAGGATGCTATCCTAAGTTAATGTGGTG | 1922 |
| 3 | GAACTGAGCGGACATCAGCTAGGCCTACAATACAT | 1923 |
| 4 | TCGTGAACTTCTGCGTTGGTCTCTACCAAGGCGGT | 1924 |
| 5 | TAAGTCAGGTATCTTATCAGTGGTACACGGTACGA | 1925 |
| 6 | TAATAATGTTGCGCGTGACCGAGGAGGAATCCACT | 1926 |
| 7 | CTAGGAGTTCTCGTAAGCTGGAGTACCGTAACGTG | 1927 |
| 8 | GGACTCTCCTCAGAGGATCCTTCTTGCGCAGGCAT | 1928 |
| 9 | GCTAGAGGCCTGAGTACACCTTCTCGCATCAGGAT | 1929 |
| 10 | ATATCGCGAGCACTAACGTCGTTGTCGTTCTAGGA | 1930 |
| 11 | AGCGGTTACTATACCTGGCGGCTGACGTTGTTAGT | 1931 |
| 12 | GAGCTAGGTAGATCTCCAAGTGTAGCTAAGAAGAG | 1932 |
| 13 | GGAGTCGCTGGTGACGTATGCCGAGGATGAGCTTC | 1933 |
| 14 | CGCCGACCTCCTGTTCACGAAGCCGCCTGATGTAA | 1934 |
| 15 | AGTAGGCACTTAGTTATCGATTACGTTAGTTAGTC | 1935 |
| 16 | GGATGACGTCTCAGTCTACCTCGCAGTGTCGTCTA | 1936 |
| 17 | CTGGTTCGCGTTAGCAATACTAAGGCAGTCAGGAG | 1937 |
| 18 | ATATGGTCATATTGGCCTCTTCGAACACAGACTGT | 1938 |
| 19 | TATCAGAGGATAGCAGGTCTGAGTTGCAAGGCTAA | 1939 |
| 20 | GGTGGTCTGACCATAGCTGTTCTTCTCACAGAGAC | 1940 |
| 21 | GCAATACCAACGAGATGAGTATTCGTTGAAGCTCT | 1941 |
| 22 | CCAAGTCGACGCTGCATGAATGAGCGCTATTCACT | 1942 |
| 23 | CCATTAGATCGCTTCGAGACAATTAGGAGAGATGA | 1943 |
| 24 | GATGACTGTACCTCCTATCATTGAGTGTGGACCAA | 1944 |
| 25 | ATATCTGGATGAATAGTGGTTAGGTAAGCAAGTAA | 1945 |
| 26 | ACCGACTATGTTAATTCGTGTCTGGATGGCAGAAT | 1946 |
| 27 | GTGGCAGTCTTGCTAGTATCTTAGACCATCACCAA | 1947 |
| 28 | CGCTATCTTAGTCGAGCACAATGTCTTCGTATAGG | 1948 |
| 29 | ATTAGTACGGCACGAACCGGCCATTCATGGCAGCT | 1949 |
| 30 | AGTACGACTATCAAGACTCCAGCGCTCTCCTTGGA | 1950 |
| 31 | ATGAGCCTCGGAGCGAACGTTATCGATCAGGCTGT | 1951 |
| 32 | TTGCGTGCAGTAGCACCGATACACAGCGCTTGTAT | 1952 |

**[Table 21-2]**

| No | Primer sequence | SEQ ID NO |
|---|---|---|
| 33 | AACGGCTGCATCACCTACACTATACTCAACATCTA | 1953 |
| 34 | GTCGCTATGCGAGAAGTGGCGTGGAATGCTATGGT | 1954 |
| 35 | CATGGATACCTACTGACTTGACTTCTAGAGGACCG | 1955 |
| 36 | GAGTGACGCAGACACCGTAACGTCGAATCTTCTAG | 1956 |
| 37 | AGTACCGTCTGTGTGAATATTGTTCCTACGTTACA | 1957 |
| 38 | GGCTAATCGATAGTGACGAGTTCTGCACGCCTGAA | 1958 |
| 39 | GGCGAGCGCTCGTGGTTCTGAGTCGCTGTTAGATG | 1959 |
| 40 | TATCTCCAGCGTTATAAGCTACTGGAGCCGCTCGG | 1960 |
| 41 | CCTTCTGCGCAAGTCAAGGATTCGCTTAGATGGAC | 1961 |
| 42 | GTTGCTGACAGCCGTTGCGTACTTGCCTTAAGAAC | 1962 |
| 43 | GTGGCCTAATCACTCGCGCTTCATAGGCCGATAGG | 1963 |
| 44 | TGCATCTAGCCTACATCGGACCTTGTTATGGTAAT | 1964 |
| 45 | GGACAGCTACTGGACACCACCGAACTGGTAGTGTC | 1965 |
| 46 | AACTGGCGATGGACGGCCGCTCTTCCGCTACATAG | 1966 |
| 47 | GGAGCAGTTAGCTATGGAGCAGGCCGATAACCTGA | 1967 |
| 48 | ACTCTACGGTGCACCTCAGCCTTCATGCAATAGGC | 1968 |
| 49 | CTTGTAGCACAATACATTACTCTCCACGTGATAGC | 1969 |
| 50 | GGACGCTATCGATACCGTTATTCCTACTCTGTCGG | 1970 |
| 51 | GGATGATCGTCAACGATCAACTGACAGTTAGTCGA | 1971 |
| 52 | TGACAGTAGCAATGTCTCACGTCTGCACAACGGAA | 1972 |
| 53 | GTCGCAGGACCTCACGGATAGTAGTGCGAGGTCTA | 1973 |
| 54 | ATATCGGCGGACGCAATGACAGTTGTTGGCTGATG | 1974 |
| 55 | AAGCACCAAGGAGGTATGTTCCATCGAGGCGCTCG | 1975 |
| 56 | GACCGCACCTTATAGCTATATCCTGGTCTAGTACT | 1976 |
| 57 | TCTCAGAGGAAGGTTGAGCGTCTGACCAGGTTGGC | 1977 |
| 58 | TGGACCTAGAGACCTAGCTCGTCTCTTCGCGATCG | 1978 |
| 59 | CGGAGTGGTTCCACGCGACCTCGCAACTAATCCTT | 1979 |
| 60 | GGAGCCGCGCGCAGACTGACCTTGCTTGATCTACT | 1980 |
| 61 | ACTCTAAGTATATGCGCAGTTAGTATACTGAACCA | 1981 |
| 62 | GAGCATTGCTTCGCTTCGATGTCTATTCTGATCAG | 1982 |
| 63 | GCTTGTATTGCCACTCGAGTAGGTCGTGGCAGTAG | 1983 |
| 64 | ATCTGGACATTGCATTCGGTGTGTATACAGAAGGC | 1984 |

**[Table 21-3]**

| No | Primer sequence | SEQ ID NO |
|---|---|---|
| 65 | GGTTGCGATCAGCTTGATAGCAGGTCATATCCTCA | 1985 |
| 66 | GCAGGTACTAACCTGAGATGCGTAGCTAACACAGG | 1986 |
| 67 | ATCTGCAAGGACGTAACGTCCTCGGAAGGTGAGGT | 1987 |
| 68 | ATAATCTTACGAGCCTCCAGTGAATAATGCAAGCA | 1988 |
| 69 | CAATCTCCGCACAGTCTTGTTCAGGTACAGACTTA | 1989 |
| 70 | ATGTGCGCAATTCAGCGTAAGTGCCTATTCATAAT | 1990 |
| 71 | TCGGACGCACACATCCTGTTGTCGAGAAGAGGAAG | 1991 |
| 72 | TCGGAAGCATCACATGAGCATCAGGAGTTCATTGC | 1992 |
| 73 | ATCTGGTTGTGGACTTCTATACAGTACCAGAGTGG | 1993 |
| 74 | CGTCTGAATATAGTTAGCTAGTAGTGTAATCCAGG | 1994 |
| 75 | TAATATCTGATCCGACCTATTATCTAGGACTACTC | 1995 |
| 76 | TATGCGGCCGTCCGTACCTCGTCTGCTTCAGTTGG | 1996 |
| 77 | TGGCTCAAGTTCCATATTGCCAAGACGACCTGGAG | 1997 |
| 78 | GCAGTTCTGCTAGGCGGTCCGAGGCAATTGAAGAG | 1998 |
| 79 | CATGGCACAGACGAAGTATGCACCACGCTCATTAA | 1999 |
| 80 | GGAGCGTACTACGACCATTCAACCGAATATGTTAC | 2000 |
| 81 | GCGTAGATCTCGCGACAGAGACAAGGTGCGAATGG | 2001 |
| 82 | TGGACTGAGGTTCTCCGGTCTATACTCCTGTAGGA | 2002 |
| 83 | TGGCTATAGCAACGGCTTCTTGTGATCGCATTGCA | 2003 |
| 84 | GGCGAAGAATCATGCGAGACGGAGTAGACGGACGT | 2004 |
| 85 | GAGCATTGCGAGTTGCACACGTGATATCAGACTGT | 2005 |
| 86 | CTGTTGACCTATGCCAGAATCAATACCTCAGATTA | 2006 |
| 87 | GTTAACAAGTAGATGCCAAGATACAACGAGAGACC | 2007 |
| 88 | GAGCAAGATTATAGTTAGGAAGATAGTTAACTCGC | 2008 |
| 89 | TCCGGAGTCGAGCATATGTGACCAACTCTCAACGC | 2009 |
| 90 | GGAGCTGCGATGCCGTTACCGACGTCATCTTCAAG | 2010 |
| 91 | GCTCTATCTTACACATTGGCGTACTGGACTCGCGA | 2011 |
| 92 | TTCTACATATTCATCGCCTACCGAGTTGCGCGAAG | 2012 |
| 93 | TGGACGTCTGACCTGTGTCTACATCGGTGGTGCTA | 2013 |
| 94 | GGCAGGACAGCTCCGTGTTCTACTCGAACCGCACT | 2014 |
| 95 | TGACAACCTCATGTCTCCGACCGCAGGCATACAAT | 2015 |
| 96 | GCAGGCCTAACAAGTGGTCACGAGGAGTCCTTATT | 2016 |

### 3.1.2 Standard PCR

To the genomic DNA described in 2. above (15 ng, NiF8-derived genomic DNA), random primers (final concentration: 0.6 microM; 10-base primer A), a 0.2mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. In this example, numerous nucleic acid fragments obtained via PCR using random primers, including the standard PCR described above, are referred to as DNA libraries.

### 3.1.3 Purification of DNA library and electrophoresis

The DNA library obtained in 3.1.2 above was purified with the use of the MinElute PCR Purification Kit (QIAGEN) and subjected to electrophoresis with the use of the Agilent 2100 bioanalyzer (Agilent Technologies) to obtain a fluorescence unit (FU).

### 3.1.4 Examination of annealing temperature

To the genomic DNA described in 2. above (15 ng, NiF8-derived genomic DNA), random primers (final concentration: 0.6 microM; 10-base primer A), a 0.2mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, various annealing temperatures for 15 seconds, and 72 degrees C for 20 seconds, followed by storage at 4 degrees C. In this example, annealing temperature of 37 degrees C, 40 degrees, and 45 degrees C were examined. The DNA library obtained in this experiment was subjected to purification and electrophoresis in the same manner as in 3.1.3.

### 3.1.5 Examination of enzyme amount

To the genomic DNA described in 2. above (15 ng, NiF8-derived genomic DNA), random primers (final concentration: 0.6 microM; 10-base primer A), a 0.2mM dNTP mixture, 1.0 mM MgCl₂, and 2.5 units or 125 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3.

### 3.1.6 Examination of MgCl₂ concentration

To the genomic DNA described in 2. above (15 ng, NiF8-derived genomic DNA), random primers (final concentration: 0.6 microM; 10-base primer A), a 0.2mM dNTP mixture, MgCl₂ at a given concentration, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. In this example, MgCl₂ concentrations, which are 2 times (2.0 mM), 3 times (3.0 mM), and 4 times (4.0 mM) greater than a common level, respectively, were examined. The DNA library obtained in this experiment was subjected to purification and electrophoresis in the same manner as in 3.1.3.

### 3.1.7 Examination of base length of random primer

To the genomic DNA described in 2. above (15 ng, NiF8-derived genomic DNA), random primers (final concentration: 0.6 microM), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. In this example, the random primers comprising 8 bases (Table 7), 9 bases (Table 8), 11 bases (Table 9), 12 bases (Table 10), 14 bases (Table 11), 16 bases (Table 12), 18 bases (Table 13), and 20 bases (Table 14) were examined. The DNA library obtained in this experiment was subjected to purification and electrophoresis in the same manner as in 3.1.3.

### 3.1.8 Examination of random primer concentration

To the genomic DNA described in 2. above (15 ng, NiF8-derived genomic DNA), random primers at a given concentration (10-base primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. In this example, random primer concentrations of 2, 4, 6, 8, 10, 20, 40, 60, 100, 200, 300, 400, 500, 600, 700, 800, 900, and 1000 microM were examined. The DNA library obtained in this experiment was subjected to purification and electrophoresis in the same manner as in 3.1.3. In this experiment, the reproducibility of the repeated data was evaluated on the basis of the Spearman's rank correlation (rho > 0.9).

### 3.2 Verification of reproducibility via MiSeq

### 3.2.1 Preparation of DNA library

To the genomic DNA described in 2. above (15 ng, NiF8-derived genomic DNA), random primers (final concentration: 60 microM, 10-base primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3.

### 3.2.2 Preparation of sequence library

From the DNA library obtained in 3.2.1, a sequence library for MiSeq analysis was prepared using the KAPA Library Preparation Kit (Roche).

### 3.2.3 MiSeq analysis

With the use of the MiSeq Reagent Kit V2 500 Cycle (Illumina), the sequence library for MiSeq analysis obtained in 3.2.2 was analyzed via 100 base paired-end sequencing.

### 3.2.4 Read data analysis

Random primer sequence information was deleted from the read data obtained in 3.2.3, and the read patterns were identified. The number of reads was counted for each read pattern, the number of reads of the repeated analyses, and the reproducibility was evaluated using the correlational coefficient.

### 3.3 Analysis of rice variety Nipponbare

### 3.3.1 Preparation of DNA library

To the genomic DNA described in 2. above (15 ng, Nipponbare-derived genomic DNA), a random primer (final concentration: 60 microM, (10-base primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3.

### 3.3.2 Preparation of sequence library, MiSeq analysis, and read data analysis

Preparation of a sequence library using the DNA library prepared from Nipponbare-derived genomic DNA, MiSeq analysis, and analysis of the read data were performed in accordance with the methods described in 3.2.2, 3.2.3, and 3.2.4, respectively.

### 3.3.3 Evaluation of genomic homogeneity

The read patterns obtained in 3.3.2 were mapped to the genomic information of Nipponbare (NC_008394 to NC_008405) using bowde2, and the genomic positions of the read patterns were identified.

### 3.3.4 Non-specific amplification

On the basis of the positional information of the read patterns identified in 3.3.3, the sequences of random primers were compared with the genome sequences to which such random primers would anneal, and the number of mismatches was determined.

### 3.4 Detection of polymorphism and identification of genotype

### 3.4.1 Preparation of DNA library

To the genomic DNA described in 2. above (15 ng, NiF8-derived genomic DNA, Ni9-derived genomic DNA, hybrid progeny-derived genomic DNA, or Nipponbare-derived genomic DNA), random primers (final concentration: 60 microM, 10-base primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3.

### 3.4.2 HiSeq analysis

Analysis of the DNA libraries prepared in 3.4.1 was consigned to TakaraBio under conditions in which the number of samples was 16 per lane via 100 base paired-end sequencing, and the read data were obtained.

### 3.4.3 Read data analysis

Random primer sequence information was deleted from the read data obtained in 3.4.2, and the read patterns were identified. The number of reads was counted for each read pattern.

### 3.4.4 Detection of polymorphism and identification of genotype

On the basis of the read patterns and the number of reads obtained as a results of analysis conducted in 3.4.3, polymorphisms peculiar to NiF8 and Ni9 were detected, and the read patterns thereof were designated as markers. On the basis of the number of reads, the genotypes of the 22 hybrid progeny lines were identified. The accuracy for genotype identification was evaluated on the basis of the reproducibility attained by the repeated data concerning the 22 hybrid progeny lines.

### 3.5 Experiment for confirmation with PCR marker

### 3.5.1 Primer designing

Primers were designed for a total of 6 markers (i.e., 3 NiF8 markers and 3 Ni9 markers) among the markers identified in 3.4.4 based on the marker sequence information obtained via paired-end sequencing (Table 22).

**[Table 22]**

| Table 22 Marker sequence information and PCR marker primer information | | | | | |
|---|---|---|---|---|---|
| Genotype | Marker name | Marker sequence 1* | Marker sequence 2* | Primer 1 | Primer 2 |
| NiF8 type | N80521152 | CCCATACACACACCATGAAGCTTGAACTAATT AACATTCTCAAACTAATTAACAAGCATGCAAG CATGTTTTTACACAATGACAATATAT (SEQ ID NO:2017) | ATGGGTGAGGGCGCAGAGGCAAAGACATGGAG GTCCGGAAGGGTAGAAGCTCACATCAAGTCGA GTATGTTGAATGCAATCCCATATATA (SEQ ID NO:2018) | CCCATACACACACCA TGAAGCTTG (SEQ ID NO:2019) | GGTAGAAGCTCACAT CAAGTCGAG (SEQ ID NO:2020) |
| | N80997192 | AATCACAGAACGAGGTCTGGACGAGAACAGAG CTGGACATCTACACGCACCGCATGGTAGTAGA GCATGTACTGCAAAAGCTTGAAGCGC (SEQ ID NO:2021) | GATGCTGAGGGCGAAGTTGTCAGCCAAGTCCT CAATGTCATAGGCGAGATCGCAGTAGTTCTGT AACCATTCCCTGCTAAACTGGTCCAT (SEQ ID NO:2022) | ACGAGAACAGAGCTG GACATCTAC (SEQ ID NO:2023) | TCAATGTCATAGGCG AGATCGCAG (SEQ ID NO:2024) |
| | N80533142 | AGACCAACAAGCAGCAAGTAGTCAGAGAAGTA CAAGAGAAGGAGAGCAAGAAGGATAGTAAGTT GCAAGCTTACCGTTACAAAGATGATA (SEQ ID NO:2025) | GGAGGAGCACAACTAGGCGTTTATCAAGATGG GTCATCGAGCTCTTGGTGTCTTCAACCTTCTT GACATCAACTTCTCCAATCTTCGTCT (SEQ ID NO:2026) | GGAGAGCAAGAAGGA TAGTAAGTTGC (SEQ ID NO:2027) | CGAGCTCTTGGTGTC TTCAACCTTC (SEQ ID NO:2028) |
| Ni9 type | N91552391 | TGGGGTAGTCCTGAAGCTCTAGGTATGCCTCT TCATCTCCCTGCACCTCTGGTGCTAGCACCTC CTGCTCTTCGGGCACCTCTACCGGGG (SEQ ID NO:2029) | GGATACTGATGTAGCTTTCACCCGGGAGTATT CCAAGGTATCGATTTTCCACGGGGAACGCGAA GTGCACTAGTTGAGGTTTAGATTGCC (SEQ ID NO:2030) | GAAGCTCTAGGTATG CCTCTTCATC (SEQ ID NO:2031) | GTGCACTAGTTGAGG TTTAGATTGC (SEQ ID NO:2032) |
| | N91653962 | TCGGGAAAACGAACGGGCGAACTACAGATGTC AGTACGAAGTAGTCTATGGCAGGAAATACGTA GTCCATACGTGGTGCCAGCCCAAGCC (SEQ ID NO:2033) | AGCAGGAGGGAGAAAGGAAACGTGGCATTCAT CGGCTGTCTGCCATTGCCATGTGAGACAAGGA AATCTACTTCACCCCCATCTATCGAG (SEQ ID NO:2034) | GGGCGAACTACAGAT GTCAGTACG (SEQ ID NO:2035) | CTGTCTGCCATTGCC ATGTGAGAC (SEQ ID NO:2036) |
| | N91124801 | AGACATAAGATTAACTATGAACAAATTCACGG GTCCGATTCCTTTGGGATTTGCAGCTTGCAAG AACCTTCAAATACTCATTATATCTTC (SEQ ID NO:2037) | TTAAGTTGCAGAATTTGATACGAAGAACTTGA AGCATGGTGAGGTTGCCGAGCTCATTGGGGAT GGTTCCAGAAAGGCTATTGTAGCTTA (SEQ ID NO:2038) | GAACAAATTCACGGG TCCGATTCC (SEQ ID NO:2039) | CGAAGAACTTGAAGC ATGGTGAGG (SEQ ID NO:2040) |

| | | | | | |
|---|---|---|---|---|---|
| * Marker sequence information by paired-end sequencing | | | | | |

### 3.5.2 PCR and electrophoresis

With the use of the TaKaRa Multiplex PCR Assay Kit Ver.2 (TAKARA) and the genomic DNA described in 2. above (15 ng, NiF8-derived genomic DNA, Ni9-derived genomic DNA, or hybrid progeny-derived genomic DNA) as a template, 1.25 microliters of Multiplex PCR enzyme mix, 12.5 microliters of 2x Multiplex PCR buffer, and the 0.4 microM primer designed in 3.5.1 were added, and a reaction solution was prepared while adjusting the final reaction level to 25 microliters. PCR was carried out under thermal cycling conditions comprising 94 degrees C for 1 minute, 30 cycles of 94 degrees C for 30 seconds, 60 degrees C for 30 seconds, and 72 degrees C for 30 seconds, and retention at 72 degrees C for 10 minutes, followed by storage at 4 degrees C. The amplified DNA fragment was subjected to electrophoresis with the use of TapeStation (Agilent Technologies).

### 3.5.3 Comparison of genotype data

On the basis of the results of electrophoresis obtained in 3.5.2, the genotype of the marker was identified on the basis of the presence or absence of a band, and the results were compared with the number of reads of the marker.

### 3.6 Correlation between random primer density and length

### 3.6.1 Influence of random primer length at high concentration

To the genomic DNA described in 2. above (15 ng, NiF8-derived genomic DNA), random primers of a given length (final concentration: 10 microM), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. In this experiment, the random primer lengths of 9 bases (Table 8), 10 bases (Table 1, 10-base primer A), 11 bases (Table 9), 12 bases (Table 10), 14 bases (Table 11), 16 bases (Table 12), 18 bases (Table 13), and 20 bases (Table 14) were examined. In the reaction system using a random primer of 9 bases, PCR was carried out under thermal cycling conditions comprising 98 degrees C for 2 minutes and 30 cycles of 98 degrees C for 10 seconds, 37 degrees C for 15 seconds, and 72 degrees C for 20 seconds, followed by storage at 4 degrees C. In the reaction system using a random primer of 10 or more bases, PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3.

### 3.6.2 Correlation between random primer density and length

To the genomic DNA described in 2. above (15 ng, NiF8-derived genomic DNA), random primers of a given length were added to a given concentration therein, a 0.2 mM dNTP mixture, 1.0 mM MgCl₂, and 1.25 units of DNA polymerase (PrimeSTAR, TAKARA) were added thereto, and a reaction solution was prepared while adjusting the final reaction level to 50 microliters. In this experiment, random primers comprising 8 to 35 bases shown in Tables 1 to 21 were examined, and the random primer concentration from 0.6 to 300 microM was examined.

In the reaction system using random primers each comprising 8 bases and 9 bases, PCR was carried out under thermal cycling conditions comprising 98 degrees C for 2 minutes and 30 cycles of 98 degrees C for 10 seconds, 37 degrees C for 15 seconds, and 72 degrees C for 20 seconds, followed by storage at 4 degrees C. In the reaction system using a random primer of 10 or more bases, PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3. Also, the reproducibility of the repeated data was evaluated on the basis of the Spearman's rank correlation (rho > 0.9).

### 3.7 Number of random primers

To the genomic DNA described in 2. above (15 ng, NiF8-derived genomic DNA), 1, 2, 3, 12, 24, or 48 types of random primers selected from the 96 types of random primers comprising 10 bases (10-base primer A) shown in Table 1 were added to the final concentration of 60 microM therein, a 0.2 mM dNTP mixture, 1.0 mM MgCl₂, and 1.25 units of DNA polymerase (PrimeSTAR, TAKARA) were added thereto, and a reaction solution was prepared while adjusting the final reaction level to 50 microliters. In this experiment, as the 1, 2, 3, 12, 24, or 48 types of random primers, random primers were selected successively from No. 1 shown in Table 1, and the selected primers were then examined. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3. Also, the reproducibility of the repeated data was evaluated on the basis of the Spearman's rank correlation (rho > 0.9).

### 3.8 Random primer sequence

To the genomic DNA described in 2. above (15 ng, NiF8-derived genomic DNA), a set of primers selected from the 5 sets of random primers shown in Tables 2 to 6 was added to the final concentration of 60 microM therein, a 0.2 mM dNTP mixture, 1.0 mM MgCl₂, and 1.25 units of DNA polymerase (PrimeSTAR, TAKARA) were added thereto, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3. Also, the reproducibility of the repeated data was evaluated on the basis of the Spearman's rank correlation (rho > 0.9).

### 3.9 DNA library using human-derived genomic DNA

To the genomic DNA described in 2. above (15 ng, human-derived genomic DNA), a random primer (final concentration: 60 microM; 10-base primer A), a 0.2 mM dNTP mixture, 1.0 mM MgCl₂, and 1.25 units of DNA polymerase (PrimeSTAR, TAKARA) were added, and a reaction solution was prepared while adjusting the final reaction level to 50 microliters. PCR was carried out under thermal cycling conditions comprising 98 degrees C for 2 minutes and 30 cycles of 98 degrees C for 10 seconds, 50 degrees C for 15 seconds, and 72 degrees C for 20 seconds, followed by storage at 4 degrees C. The DNA library obtained in this experiment was subjected to purification and electrophoresis in the same manner as in 3.1.3. Also, the reproducibility of the repeated data was evaluated on the basis of the Spearman's rank correlation (rho > 0.9).

### 4. Results and examination

### 4.1 Correlation between PCR conditions and DNA library size

When PCR was conducted with the use of random primers in accordance with conventional PCR conditions (3.1.2 described above), the amplified DNA library size was as large as 2 kbp or more, but amplification of the DNA library of a target size (i.e., 100-bp to 500-bp) was not observed (Fig. 2). A DNA library of 100 bp to 500 bp could not be obtained because it was highly unlikely that a random primer would function as a primer in a region of 500 bp or smaller. In order to prepare a DNA library of the target size (i.e., 100 bp to 500 bp), it was considered necessary to induce non-specific amplification with high reproducibility.

The correlation between the annealing temperature (3.1.4 above), the enzyme amount (3.1.5 above), the MgCl₂ concentration (3.1.6 above), the primer length (3.1.7 above), and the primer concentration (3.18 above), which are considered to affect PCR specificity, and the DNA library size were examined.

Fig. 3 shows the results of the experiment described in 3.1.4 attained at an annealing temperature of 45 degrees C, Fig. 4 shows the results attained at an annealing temperature of 40 degrees C, and Fig. 5 shows the results attained at an annealing temperature of 37 degrees C. By reducing the annealing temperature from 45 degrees C, 40 degrees C, to 37 degrees C, as shown in Figs. 3 to 5, the amounts of high-molecular-weight DNA library amplified increased, although amplification of low-molecular-weight DNA library was not observed.

Fig. 6 shows the results of the experiment described in 3.1.5 attained when the enzyme amount is increased by 2 times, and Fig. 7 shows the results attained when the enzyme amount is increased by 10 times the original amount. By increasing the enzyme amount by 2 times or 10 times a common amount, as shown in Figs. 6 and 7, the amounts of high-molecular-weight DNA library amplified increased, although amplification of low-molecular-weight DNA library was not observed.

Fig. 8 shows the results of the experiment described in 3.1.6 attained when the MgCl₂ concentration is increased by 2 times a common amount, Fig. 9 shows the results attained when the MgCl₂ concentration is increased by 3 times, and Fig. 10 shows the results attained when the MgCl₂ concentration is increased by 4 times. By increasing the MgCl₂ concentration by 2 times, 3 times, and 4 times the common amount, as shown in Figs. 8 to 10, the amounts of high-molecular-weight DNA library amplified varied, although amplification of a low-molecular-weight DNA library was not observed.

Figs. 11 to 18 show the results of the experiment described in 3.1.7 attained at the random primer lengths of 8 bases, 9 bases, 11 bases, 12 bases, 14 bases, 16 bases, 18 bases, and 20 bases, respectively. Regardless of the length of a random primer, as shown in Figs. 11 to 18, no significant change was observed in comparison with the results shown in Fig. 2 (a random primer comprising 10 bases).

The results of experiment described in 3.1.8 are summarized in Table 23.

**[Table 23]**

| Concentration (*µ*M) | Repeat | Fig. No. | Correlational coefficient (*ρ*) |
|---|---|---|---|
| 2 | - | Fig. 19 | - |
| 4 | - | Fig. 20 | - |
| 6 | First | Fig. 21 | 0.889 |
| | Second | Fig. 22 | |
| 8 | First | Fig. 23 | 0.961 |
| | Second | Fig. 24 | |
| 10 | First | Fig. 25 | 0.979 |
| | Second | Fig. 26 | |
| 20 | First | Fig. 27 | 0.950 |
| | Second | Fig. 28 | |
| 40 | First | Fig. 29 | 0.975 |
| | Second | Fig. 30 | |
| 60 | First | Fig. 31 | 0.959 |
| | Second | Fig. 32 | |
| 100 | First | Fig. 33 | 0.983 |
| | Second | Fig. 34 | |
| 200 | First | Fig. 35 | 0.991 |
| | Second | Fig. 36 | |
| 300 | First | Fig. 37 | 0.995 |
| | Second | Fig. 38 | |
| 400 | First | Fig. 39 | 0.988 |
| | Second | Fig. 40 | |
| 500 | First | Fig. 41 | 0.971 |
| | Second | Fig. 42 | |
| 600 | - | Fig. 43 | - |
| 700 | - | Fig. 44 | - |
| 800 | - | Fig. 45 | - |
| 900 | - | Fig. 46 | - |
| 1000 | - | Fig. 47 | - |

With the use of random primers comprising 10 bases, as shown in Figs. 19 to 47, amplification was observed in a 1-kbp DNA fragment at the random primer concentration of 6 microM. As the concentration increased, the molecular weight of a DNA fragment decreased. Reproducibility at the random primer concentration of 6 to 500 microM was examined. As a result, a relatively low rho value of 0.889 was attained at the concentration of 6 microM, which is 10 times higher than the usual level. At the concentration of 8 microM, which is equivalent to 13.3 times higher than the usual level, and at 500 microM, which is 833.3 times higher than the usual level, a high rho value of 0.9 or more was attained. The results demonstrate that a DNA fragment of 1 kbp or smaller can be amplified while achieving high reproducibility by elevating the random primer concentration to a level significantly higher than the concentration employed under general PCR conditions. When the random primer concentration is excessively higher than 500 microliter, amplification of a DNA fragment of a desired size cannot be observed. In order to amplify a low-molecular-weight DNA fragment with excellent reproducibility, accordingly, it was found that the random primer concentration should fall within an optimal range, which is higher than the concentration employed in a general PCR procedure and equivalent to or lower than a given level.

### 4.2 Confirmation of reproducibility via MiSeq

In order to confirm the reproducibility for DNA library production, as described in 3.2 above, the DNA library amplified with the use of the genomic DNA extracted from NiF8 as a template and random primers was analyzed with the use of a next-generation sequencer (MiSeq), and the results are shown in Fig. 48. As a result of 3.2.4 above, 47,484 read patterns were obtained. As a result of comparison of the number of reads obtained through repeated measurements, a high correlation (i.e., a correlational coefficient "r" of 0.991) was obtained, as with the results of electrophoresis. Accordingly, it was considered that a DNA library could be produced with satisfactory reproducibility with the use of random primers.

### 4.3 Analysis of rice variety Nipponbare

As described in 3.3 above, a DNA library was prepared with the use of genomic DNA extracted from the rice variety Nipponbare, the genomic information of which has been disclosed, as a template, and random primers and subjected to electrophoresis, and the results are shown in Figs. 49 and 50. On the basis of the results shown in Figs. 49 and 50, the rho value was found to be as high as 0.979. Also, Fig. 51 shows the results of analysis of the read data with the use of MiSeq. On the basis of the results shown in Fig. 51, the correlational coefficient "r" was found to be as high as 0.992. These results demonstrate that a DNA library of rice could be produced with very high reproducibility with the use of random primers.

As described in 3.3.3, the obtained read pattern was mapped to the genomic information of Nipponbare. As a result, DNA fragments were found to be evenly amplified throughout the genome at intervals of 6.2 kbp (Fig. 52). As a result of comparison of the sequence and genome information of random primers, 3.6 mismatches were found on average, and one or more mismatches were observed in 99.0% of primer pairs (Fig. 53). The results demonstrate that a DNA library involving the use of random primers is produced with satisfactory reproducibility via non-specific amplification evenly throughout the genome.

### 4.4 Detection of polymorphism and genotype identification of sugarcane

As described in 3.4, DNA libraries of the sugarcane varieties NiF8 and Ni9 and 22 hybrid progeny lines were produced with the use of random primers, the resulting DNA libraries were analyzed with the next-generation sequencer (HiSeq), the polymorphisms of the parent varieties were detected, and the genotypes of the hybrid progenies were identified on the basis of the read data. Table 24 shows the results.

**[Table 24]**

| Table 24 Number of sugarcane NiF8 and Ni0 markers and accuracy for genotype identification | | | | | | | |
|---|---|---|---|---|---|---|---|
| | Number of markers | F1_01 | | F1_02 | | Total | |
| | | Consistency | Reproducibility | Consistency | Reproducibility | Consistency | Reproducibility |
| NiF8 type | 8,683 | 8,680 | 99.97% | 8, 682 | 99.99% | 17,362 | 99.98% |
| Ni9 type | 11,655 | 11,650 | 99.96% | 11,651 | 99.97% | 23,301 | 99.96% |
| Total | 20,338 | 20,330 | 99.96% | 20,333 | 99.98% | 40,663 | 99.97% |

As shown in Table 24, 8,683 NiF8 markers and 11,655 Ni9 markers; that in, a total of 20,338 markers, were produced. In addition, reproducibility for genotype identification of hybrid progeny lines was as high as 99.97%. This indicates that the accuracy for genotype identification is very high. In particular, sugarcane is polyploid (8x+n), the number of chromosomes is as large as 100 to 130, and the genome size is as large as 10 Gbp, which is at least 3 times greater than that of humans. Accordingly, it is very difficult to identify the genotype throughout the genomic DNA. As described above, numerous markers can be produced with the use of random primers, and the sugarcane genotype can thus be identified with high accuracy.

### 4.5 Experiment for confirmation with PCR marker

As described in 3.5 above, the sugarcane varieties NiF8 and Ni9 and 22 hybrid progeny lines were subjected to PCR with the use of the primers shown in Table 22, genotypes were identified via electrophoresis, and the results were compared with the number of reads. Figs. 54 and 55 show the number of reads and the electrophoretic pattern of the NiF8 marker N80521152, respectively. Figs. 56 and 57 show the number of reads and the electrophoretic pattern of the NiF8 marker N80997192, respectively. Figs. 58 and 59 show the number of reads and the electrophoretic pattern of the NiF8 marker N80533142, respectively. Figs. 60 and 61 show the number of reads and the electrophoretic pattern of the Ni9 marker N91552391, respectively. Figs. 62 and 63 show the number of reads and the electrophoretic pattern of the Ni9 marker N91653962, respectively. Figs. 64 and 65 show the number of reads and the electrophoretic pattern of the Ni9 marker N91124801, respectively.

As shown in Figs. 54 to 65, the results for all the PCR markers designed in 3.5 above were consistent with the results of analysis with the use of a next-generation sequencer. It was thus considered that genotype identification with the use of a next-generation sequencer would be applicable as a marker technique.

### 4.6 Correlation between random primer density and length

As described in 3.6.1, the results of DNA library production with the use of random primers comprising 9 bases (Table 8), 10 bases (Table 1, 10-base primer A), 11 bases (Table 9), 12 bases (Table 10), 14 bases (Table 11), 16 bases (Table 12), 18 bases (Table 13), and 20 bases (Table 14) are shown in Figs. 66 to 81. The results are summarized in Table 25.

**[Table 25]**

| Random primer length | Repeat | Fig. No. | Correlational coefficient (ρ) |
|---|---|---|---|
| 9 | First | Fig. 66 | 0.981 |
| | Second | Fig. 67 | |
| 10 | First | Fig. 68 | 0.979 |
| | Second | Fig. 69 | |
| 11 | First | Fig. 70 | 0.914 |
| | Second | Fig. 71 | |
| 12 | First | Fig. 72 | 0.957 |
| | Second | Fig. 73 | |
| 14 | First | Fig. 74 | 0.984 |
| | Second | Fig. 75 | |
| 16 | First | Fig. 76 | 0.989 |
| | Second | Fig. 77 | |
| 18 | First | Fig. 78 | 0.995 |
| | Second | Fig. 79 | |
| 20 | First | Fig. 80 | 0.999 |
| | Second | Fig. 81 | |

When random primers were used at a high concentration of 10.0 microM, which is 13.3 times greater than the usual level, as shown in Figs. 66 to 81, it was found that a low-molecular-weight DNA fragment could be amplified with the use of random primers comprising 9 to 20 bases while achieving very high reproducibility. As the base length of a random primer increased (12 bases or more, in particular), the molecular weight of the amplified fragment was likely to be decreased. When random primers comprising 9 bases were used, the amount of the DNA fragment amplified was increased by setting the annealing temperature at 37 degrees C.

In order to elucidate the correlation between the density and the length of random primers, as described in 3.6.2 above, PCR was carried out with the use of random primers comprising 8 to 35 bases at the concentration of 0.6 to 300 microM, so as to produce a DNA library. The results are shown in Table 26.

**[Table 26]**

| Correlation between concentration and length of random primer relative to DNA library | | | | | | | | | | | | | | | | | |
|---|---|---|---|---|---|---|---|---|---|---|---|---|---|---|---|---|---|
| Primer µM | Concentration relative to standard | Primer length | | | | | | | | | | | | | | | |
| | | 8 | 9 | 10 | 11 | 12 | 14 | 16 | 18 | 20 | 22 | 24 | 26 | 28 | 29 | 30 | 35 |
| 0.6 | standard | × | × | × | × | × | × | × | × | × | × | × | × | × | × | × | × |
| 2 | 3.3 x | × | × | × | × | × | × | × | × | × | × | × | × | × | × | × | × |
| 4 | 6.7 x | × | × | × | × | × | ○ | ○ | ○ | ○ | ○ | ○ | ○ | ○ | × | × | × |
| 6 | 10.0 x | × | × | × | × | × | ○ | ○ | ○ | ○ | ○ | ○ | ○ | ○ | ○ | ○ | × |
| 8 | 13.3 x | × | × | × | × | ○ | ○ | ○ | ○ | ○ | ○ | ○ | ○ | ○ | ○ | × | × |
| 10 | 16.7 x | × | × | × | × | ○ | ○ | ○ | ○ | ○ | ○ | ○ | ○ | ○ | ○ | × | × |
| 20 | 33.3 x | × | × | × | ○ | ○ | ○ | ○ | ○ | ○ | ○ | ○ | × | × | × | × | × |
| 40 | 66.7 x | × | ○ | ○ | ○ | ○ | ○ | ○ | ○ | ○ | × | × | × | × | × | × | × |
| 60 | 100.0 x | × | ○ | ○ | ○ | ○ | ○ | ○ | ○ | ○ | × | × | × | × | × | × | × |
| 100 | 166.7 x | - | × | ○ | ○ | ○ | ○ | ○ | ○ | × | - | - | - | - | - | - | - |
| 200 | 333.3 x | - | × | ○ | ○ | × | × | × | × | × | - | - | - | - | - | - | - |
| 300 | 500.0 x | - | × | × | × | × | × | × | × | × | - | - | - | - | - | - | - |

| | | | | | | | | | | | | | | | | | |
|---|---|---|---|---|---|---|---|---|---|---|---|---|---|---|---|---|---|
| ○: DNA library covering 100 to 500 bases is amplified with good reproducibility (ρ > 0.9) × : DNA library not covering 100 to 500 bases or reproducibility is poor (ρ ≤ 0.9) ·: Unperformed | | | | | | | | | | | | | | | | | |

As shown in Table 26, it was found that a low-molecular-weight (100 to 500 bases) DNA fragment could be amplified with high reproducibility with the use of random primers comprising 9 to 30 bases at 4.0 to 200 microM. In particular, it was confirmed that low-molecular-weight (100 to 500 bases) DNA fragments could be amplified assuredly with high reproducibility with the use of random primers comprising 9 to 30 bases at 4.0 to 100 microM.

The results shown in Table 26 are examined in greater detail. As a result, the correlation between the length and the concentration of random primers is found to be preferably within a range surrounded by a frame as shown in Fig. 82. More specifically, the random primer concentration is preferably 40 to 60 microM when the random primers comprise 9 to 10 bases. It is preferable that a random primer concentration satisfy the condition represented by an inequation: y > 3E + 08x^{-6.974}, provided that the base length of the random primer is represented by y and the random primer concentration is represented by x, and 100 microM or lower, when the random primer comprises 10 to 14 bases. The random primer concentration is preferably 4 to 100 mM when the random primer comprises 14 to 18 bases. When a random primer comprises 18 to 28 bases, the random primer concentration is preferably 4 microM or higher, and it satisfies the condition represented by an inequation: y < 8E +08x^{-5.533}. When a random primer comprises 28 to 29 bases, the random primer concentration is preferably 4 to 10 microM. The inequations y > 3E + 08x^{-6.974} and y < 8E +08x^{-5.533} are determined on the basis of the Microsoft Excel power approximation.

By prescribing the number of bases and the concentration of random primers within given ranges as described above, it was found that low-molecular-weight (100 to 500 bases) DNA fragments could be amplified with high reproducibility. For example, the accuracy of the data obtained via analysis of high-molecular-weight DNA fragments with the use of a next-generation sequencer is known to deteriorate to a significant extent. As described in this example, the number of bases and the concentration of random primers may be prescribed within given ranges, so that a DNA library with a molecular size suitable for analysis with a next-generation sequencer can be produced with satisfactory reproducibility, and such DNA library can be suitable for marker analysis with the use of a next-generation sequencer.

### 4.7 Number of random primers

As described in 3.7 above, 1, 2, 3, 12, 24, or 48 types of random primers (concentration: 60 microM) were used to produce a DNA library, and the results are shown in Figs. 83 to 94. The results are summarized in Table 27.

**[Table 27]**

| Number of random primers | Repeat | Fig. No. | Correlational coefficient (ρ) |
|---|---|---|---|
| 1 | First | Fig. 83 | 0.984 |
| | Second | Fig. 84 | |
| 2 | First | Fig. 85 | 0.968 |
| | Second | Fig. 86 | |
| 3 | First | Fig. 87 | 0.974 |
| | Second | Fig. 88 | |
| 12 | First | Fig. 89 | 0.993 |
| | Second | Fig. 90 | |
| 24 | First | Fig. 91 | 0.986 |
| | Second | Fig. 92 | |
| 48 | First | Fig. 93 | 0.978 |
| | Second | Fig. 94 | |

As shown in Figs. 83 to 94, it was found that low-molecular-weight DNA fragments could be amplified with the use of any of 1, 2, 3, 12, 24, or 48 types of random primers while achieving very high reproducibility. As the number of types of random primers increases, in particular, a peak in the electrophoretic pattern lowers, and a deviation is likely to disappear.

### 4.8 Random primer sequence

As described in 3.8 above, DNA libraries were produced with the use of sets of random primers shown in Tables 2 to 6 (i.e., 10-base primer B, 10-base primer C, 10-base primer D, 10-base primer E, and 10-base primer F), and the results are shown in Figs. 95 to 104. The results are summarized in Table 28.

**[Table 28]**

| Set of random primers | Repeat | Fig. No. | Correlational coefficient (ρ) |
|---|---|---|---|
| 10-base primers B | First | Fig. 95 | 0.916 |
| | Second | Fig. 96 | |
| 10-base primers C | First | Fig. 97 | 0.965 |
| | Second | Fig. 98 | |
| 10-base primers D | First | Fig. 99 | 0.986 |
| | Second | Fig. 100 | |
| 10-base primers E | First | Fig. 101 | 0.983 |
| | Second | Fig. 102 | |
| 10-base primers F | First | Fig. 103 | 0.988 |
| | Second | Fig. 104 | |

As shown in Figs. 95 to 104, it was found that low-molecular-weight DNA fragments could be amplified with the use of any sets of 10-base primer B, 10-base primer C, 10-base primer D, 10-base primer E, or 10-base primer F while achieving very high reproducibility.

### 4.9 Production of human DNA library

As described in 3.9 above, a DNA library was produced with the use of human-derived genomic DNA and random primers at a final concentration of 60 microM (10-base primer A), and the results are shown in Figs. 105 and 106. Fig. 105 shows the results of the first repeated experiment, and Fig. 106 shows the results of the second repeated experiment. As shown in Figs. 105 and 106, it was found that low-molecular-weight DNA fragments could be amplified while achieving very high reproducibility even if human-derived genomic DNA was used.

### Example 2

In Example 2, a DNA probe was designed in accordance with the step schematically shown in Fig. 107, and a DNA microarray comprising the designed DNA probe was produced. In this example, whether or not a DNA marker could be detected with the use of such DNA microarray was examined.

In this example, a DNA library was produced in the same manner as described in 3.2.1 of Example 1, except that the random primers comprising 10 bases shown in Table 1 and 30 ng of genomic DNAs of the sugarcane varieties NiF8 and Ni9 were used. In this example, also, a sequence library was produced in the same manner as described in 3.2.2 of Example 1 and the sequence library was subjected to MiSeq analysis in the same manner as described in 3.2.3.

In this example, 306,176 types of DNA probes comprising 50 to 60 bases were designed on the basis of the sequence information of the DNA libraries of NiF8 and Ni9 obtained as a result of MiSeq analysis, so as to adjust a TM at around 80 degrees C. The sequences of the designed DNA probes were compared with the sequence information of NiF8 and Ni9, and 9,587 types of probes peculiar to NiF8, which are not found in the Ni9 DNA library, and 9,422 types of probes peculiar to Ni9, which are not found in the NiF8 DNA library, were selected. On the bases of a total of 19,002 types of the selected DNA probes, production of G3 CGH 8×60K Microarrays was consigned to Agilent Technologies, Inc.

With the use of the DNA microarrays thus produced, DNA libraries produced from NiF8, Ni9, and 22 hybrid progeny lines were subjected to detection.

DNA libraries of NiF8, Ni9, and 22 hybrid progeny lines were produced in the same manner as described in 3.2.1 of Example 1. Two DNA libraries were produced for Ni9 and for 2 hybrid progeny lines (i.e., Fl_01 and Fl_02), so as to obtain the repeated data. The DNA libraries were fluorescently labeled with the use of Cy3-Random Nonamers of the NimbleGen One-Color DNA Labeling Kit in accordance with the NimbleGen Arrays User's Guide.

With the use of the DNA microarrays and the fluorescently-labeled DNA libraries, subsequently, hybridization was carried out in accordance with the array-comparative genomic hybridization (array-CGH) method using the Agilent in-situ oligo-DNA microarray kit. Subsequently, signals on the DNA microarrays when a relevant DNA library was used were detected with the use of the SureScan scanner.

On the basis of the signals detected for NiF8, Ni9, and 22 hybrid progeny lines, 7,140 types of DNA probes exhibiting clear signal intensities were identified. DNA fragments corresponding to such DNA probes can be used as NiF8 markers and Ni9 markers. In this example, genotype data were obtained on the basis of signals obtained from DNA probes corresponding to the NiF8 markers and the Ni9 markers, genotype data obtained through repeated measurements of two hybrid progeny lines (Fl_01 and Fl_02) were compared, and the accuracy for genotype identification was evaluated on the basis of the data reproducibility.

In this example, genotype data were obtained with the use of PCR markers in order to compare such data with the results of the DNA microarray experiment described above. Specifically, the primers described in 3.5 of Example 1 (Table 22) were used for the 3 NiF8 markers and the 3 Ni9 markers described in 3.5 of Example 1 (i.e., a total of 6 markers). PCR and electrophoresis were performed in the manner as described in 3.5.2 of Example 1, and the results were compared with the signals obtained from the DNA microarray.

In this example, the DNA probes shown below were designed for the 6 markers shown in Table 22 (Table 29).

**[Table 29]**

| Marker name | DNA probe sequence |
|---|---|
| N80521152 | CACACACCATGAAGCTTGAACTAATTAACATTCTCAAACTAATTAACAAGCATGCAAGCA (SEQ ID NO:2041) |
| N80997192 | CAAGTCCTCAATGTCATAGGCGAGATCGCAGTAGTTCTGTAACCATTCCCTGCTAAACTG (SEQ ID NO:2042) |
| N80533142 | GTTTATCAAGATGGGTCATCGAGCTCTTGGTGTCTTCAACCTTCTTGACATCAACTTCTC (SEQ ID NO:2043) |
| N91552391 | CTGAAGGTCTAGGTATGCCTCTTCATCTCCCTGCACCTCTGGTGCTAGCA (SEQ ID NO:2044) |
| N91653962 | CTGTCTGCCATTGCCATGTGAGACAAGGAAATCTACTTCACCCCCATCTATCGA (SEQ ID NO:2045) |
| N91124801 | TAAGATTAACTATGAACAAATTCACGGGTCCGATTCCTTTGGGATTTGCAGCTTGCAAGA (SEQ ID NO:2046) |

### Results and Examination

### DNA microarray analysis

The sugarcane varieties NiF8 and Ni9 and 22 hybrid progeny lines were analyzed with the use of the DNA microarray produced in the manner described above. As a result, 3,570 markers exhibiting apparently different signals between parent varieties were identified as shown in Table 30 (Fig. 108).

**[Table 30]**

| | Number of markers | F1_01 | | F1_02 | | Total | |
|---|---|---|---|---|---|---|---|
| | | Consistency | Reproducibility | Consistency | Reproducibility | Consistency | Reproducibility |
| NiF8 type | 1,695 | 1,695 | 100.00% | 1,695 | 100.00% | 3,390 | 100.00% |
| Ni9 type | 1,875 | 1,874 | 99.95% | 1,875 | 100.00% | 3,749 | 99.97% |
| Total | 3,570 | 3,569 | 99.97% | 3,570 | 100.00% | 7,139 | 99.99% |

Concerning Ni9, signals obtained trough repeated procedures were compared, and a high correlation was found therebetween as a consequence (Fig. 109: r = 0.9989). On the basis of the results, the use of random primers at a high concentration was predicted to enable the production of a DNA library with excellent reproducibility and the use of a DNA probe was predicted to enable the detection of a DNA fragment contained in a DNA library (i.e., a marker).

As a result of DNA microarray analysis using the 22 hybrid progeny lines, a total of 78,540 genotype data were obtained, and no missing values were observed for any markers. In order to evaluate the accuracy for genotype identification, the data obtained through repeated analyses of Fl_01 and those of F1_02 were compared. As a result, all the data concerning the NiF8 markers were consistent. Concerning the Ni9 marker, a result concerning Fl_01 was different, although all the results concerning Fl_02 were consistent. With respect to all the markers, 7,139 data out of 7,140 genotype data were consistent; that is, a very high degree of reproducibility was observed (i.e., the degree of consistency: 99.99%).

### Experiment for confirmation with the use of PCR marker

Concerning a total of 6 markers (i.e., 3 NiF8 markers and 3 Ni9 markers), primers designed on the basis of the paired-end marker sequence information were used to subject NiF8, Ni9, and 22 hybrid progeny lines to PCR, the genotypes thereof were identified via electrophoresis, and the results were compared with the signals obtained from the DNA microarray. Fig. 110 shows the results of measurement of signal levels obtained from the DNA probes corresponding to the marker (N80521152), Fig. 111 shows the results of measurement of signal levels obtained from the DNA probes reacting with the marker (N80997192), Fig. 112 shows the results of measurement of signal levels obtained from the DNA probes reacting with the marker (N80533142), Fig. 113 shows the results of measurement of signal levels obtained from the DNA probes reacting with the marker (N91552391), Fig. 114 shows the results of measurement of signal levels obtained from the DNA probes reacting with the marker (N91653962), and Fig. 115 shows the results of measurement of signal levels obtained from the DNA probes reacting with the marker (N91124801). Fig. 55 shows the electrophoretic pattern for the marker (N80521152), Fig. 57 shows the electrophoretic pattern for the marker (N80997192), Fig. 59 shows the electrophoretic pattern for the marker (N80533142), Fig. 61 shows the electrophoretic pattern for the marker (N91552391), Fig. 63 shows the electrophoretic pattern for the marker (N91653962), and Fig. 65 shows the electrophoretic pattern for the marker (N91124801). As a result of comparison of the results of electrophoretic patterns and the results of measurement of signal values obtained from DNA probes, the results for all markers are found to be consistent among all the markers. The results demonstrate that a DNA probe may be designed on the basis of the nucleotide sequence of the DNA fragment contained in the DNA library resulting from the use of a random primer at a high concentration, so that the DNA fragment can be detected with high accuracy.

## Claims

1. A method for producing a plurality of DNA probes comprising steps of:
conducting a nucleic acid amplification reaction in a reaction solution containing genomic DNA and random primers having 9-30 bases using genomic DNA as a template to obtain DNA fragments, wherein when the random primers comprise 9 to 10 bases, the concentration of the random primers is 40 to 60 microM; when the random primers comprise 10 to 14 bases, the concentration of the random primers satisfy the conditions defined by an inequation: y > 3E + 08x^{-6.974} and be 100 microM or less, provided that the base lengths of the random primers are represented by "y" and the concentration of the random primers is represented by "x"; when the random primers comprise 14 to 18 bases, the concentration of the random primers is 4 to 100 microM; when the random primers comprise 18 to 28 bases, the concentration of such random primers be 4 microM or more and satisfy the conditions defined by an inequation: y < 8E + 08x^{-5.533}; when the random primers comprise 28 to 29 bases, the concentration of the random primers is 6 to 10 microM; when the random primers comprise 30 bases, the concentration of the random primers is 6 microM;
determining the nucleotide sequences of the obtained DNA fragments; and
designing a plurality of DNA probes used for detecting DNA fragments obtained in the above step on the basis of the nucleotide sequences of the DNA fragments by identifying a region or a plurality of regions of the base lengths shorter than those of the DNA fragments and covering at least a part of the DNA fragments, wherein the DNA probes comprise nucleotide sequences complementary to the identified one or more regions.

2. The method for producing a plurality of DNA probes according to claim 1, wherein DNA fragments are obtained from a plurality of different genomic DNAs with the use of the random primers and, on the basis of the nucleotide sequence of the DNA fragments, the DNA probes containing regions different between the genomic DNAs is designed.

3. The method for producing a plurality of DNA probes according to claim 1, wherein the DNA fragments contain 100 to 500 nucleotides.

4. A method for analyzing genomic DNA comprising steps of: bringing the DNA probes produced by the method for producing a plurality of DNA probes according to any one of claims 1 to 3 into contact with DNA fragments derived from genomic DNA subjected to analysis; and detecting hybridization occurring between the plurality of DNA probes and the DNA fragments.

5. The method for analyzing genomic DNA according to claim 4, which further comprises, before the first step of claim 4, a step of conducting a nucleic acid amplification reaction with the use of the genomic DNA subjected to analysis and the random primers to obtain the DNA fragments.

6. The method for analyzing genomic DNA according to claim 4, wherein the DNA fragments derived from genomic DNA are DNA markers and the presence or absence of the DNA markers is detected with the use of the plurality of DNA probes.

## Patentansprüche

1. Verfahren zur Herstellung einer Vielzahl von DNA-Sonden, umfassend die Schritte:
Durchführen einer Nukleinsäureamplifikationsreaktion in einer Reaktionslösung, die genomische DNA und Zufallsprimer mit 9-30 Basen enthält, unter Verwendung von genomischer DNA als Matrize, um DNA-Fragmente zu erhalten, wobei, wenn die Zufallsprimer 9 bis 10 Basen umfassen, die Konzentration der Zufallsprimer 40 bis 60 mikroM beträgt; wenn die Zufallsprimer 10 bis 14 Basen umfassen, die Konzentration der Zufallsprimer die Bedingungen erfüllt, die durch eine Ungleichung: y > 3E + 08x^{-6,974} definiert sind und 100 mikroM oder weniger beträgt, vorausgesetzt, dass die Basenlängen der Zufallsprimer durch "y" dargestellt sind und die Konzentration der Zufallsprimer durch "x" dargestellt ist; wenn die Zufallsprimer 14 bis 18 Basen umfassen, die Konzentration der Zufallsprimer 4 bis 100 mikroM beträgt; wenn die Zufallsprimer 18 bis 28 Basen umfassen, die Konzentration solcher Zufallsprimer 4 mikroM oder mehr beträgt und die Bedingungen erfüllt, die durch eine Ungleichung: y < 8E + 08x^{-5,553} definiert sind; wenn die Zufallsprimer 28 bis 29 Basen umfassen, die Konzentration der Zufallsprimer 6 bis 10 mikroM beträgt; wenn die Zufallsprimer 30 Basen umfassen, die Konzentration der Zufallsprimer 6 mikroM beträgt;
Bestimmen der Nukleotidsequenzen der erhaltenen DNA-Fragmente; und
Entwerfen einer Vielzahl von DNA-Sonden, die zum Nachweis von DNA-Fragmenten verwendet werden, die in dem obigen Schritt erhalten wurden, auf der Basis der Nukleotidsequenzen der DNA-Fragmente durch Identifizieren einer Region oder einer Vielzahl von Regionen der Basenlängen, die kürzer als diejenigen der DNA-Fragmente sind und mindestens einen Teil der DNA-Fragmente abdecken, wobei die DNA-Sonden Nukleotidsequenzen umfassen, die zu der einen oder den mehreren identifizierten Regionen komplementär sind.

2. Verfahren zur Herstellung einer Vielzahl von DNA-Sonden nach Anspruch 1, wobei DNA-Fragmente aus einer Vielzahl von verschiedenen genomischen DNAs unter Verwendung der Zufallsprimer erhalten werden und auf der Basis der Nukleotidsequenz der DNA-Fragmente die DNA-Sonden, die Regionen enthalten, die zwischen den genomischen DNAs verschieden sind, entworfen werden.

3. Verfahren zur Herstellung einer Vielzahl von DNA-Sonden nach Anspruch 1, wobei die DNA-Fragmente 100 bis 500 Nukleotide enthalten.

4. Verfahren zur Analyse von genomischer DNA, umfassend die Schritte: Inkontaktbringen der DNA-Sonden, die durch das Verfahren zur Herstellung einer Vielzahl von DNA-Sonden nach einem der Ansprüche 1 bis 3 hergestellt wurden, mit DNA-Fragmenten, die von genomischer DNA stammen, die einer Analyse unterzogen wird; und Erfassen einer Hybridisierung, die zwischen der Vielzahl von DNA-Sonden und den DNA-Fragmenten erfolgt.

5. Verfahren zur Analyse von genomischer DNA nach Anspruch 4, das ferner vor dem ersten Schritt von Anspruch 4 einen Schritt des Durchführens einer Nukleinsäureamplifikationsreaktion unter Verwendung der genomischen DNA, die einer Analyse unterzogen wird, und der Zufallsprimer, um die DNA-Fragmente zu erhalten, umfasst.

6. Verfahren zur Analyse von genomischer DNA nach Anspruch 4, wobei die DNA-Fragmente, die von genomischer DNA stammen, DNA-Marker sind und das Vorhandensein oder Nichtvorhandensein der DNA-Marker unter Verwendung der Vielzahl von DNA-Sonden erfasst wird.

## Revendications

1. Procédé de production d'une pluralité de sondes d'ADN comprenant les étapes consistant à :
réaliser une réaction d'amplification d'acide nucléique dans une solution réactionnelle contenant de l'ADN génomique et des amorces aléatoires présentant de 9 à 30 bases en utilisant l'ADN génomique comme matrice pour obtenir des fragments d'ADN, dans lequel lorsque les amorces aléatoires comprennent de 9 à 10 bases, la concentration des amorces aléatoires est de 40 à 60 microM ; lorsque les amorces aléatoires comprennent de 10 à 14 bases, la concentration des amorces aléatoires satisfait aux conditions définies par une inéquation : y > 3E + 08x^{-6,974} et être de 100 microM ou moins, à condition que les longueurs de base des amorces aléatoires soient représentées par « y » et que la concentration des amorces aléatoires soit représentée par « x » ; lorsque les amorces aléatoires comprennent de 14 à 18 bases, la concentration des amorces aléatoires est de 4 à 100 microM ; lorsque les amorces aléatoires comprennent de 18 à 28 bases, la concentration de ces amorces aléatoires est de 4 microM ou plus et satisfait aux conditions définies par une inéquation : y < 8E + 08x^{-5,533}; lorsque les amorces aléatoires comprennent de 28 à 29 bases, la concentration des amorces aléatoires est de 6 à 10 microM ; lorsque les amorces aléatoires comprennent 30 bases, la concentration des amorces aléatoires est de 6 microM ;
déterminer les séquences nucléotidiques des fragments d'ADN obtenus ; et
concevoir une pluralité de sondes d'ADN utilisées pour détecter des fragments d'ADN obtenus à l'étape ci-dessus sur la base des séquences nucléotidiques des fragments d'ADN en identifiant une région ou une pluralité de régions des longueurs de base plus courtes que celles des fragments d'ADN et en couvrant au moins une partie des fragments d'ADN, dans lequel les sondes d'ADN comprennent des séquences nucléotidiques complémentaires des une ou plusieurs régions identifiées.

2. Procédé de production d'une pluralité de sondes d'ADN selon la revendication 1, dans lequel des fragments d'ADN sont obtenus à partir d'une pluralité d'ADN génomiques différents en utilisant les amorces aléatoires et, sur la base de la séquence nucléotidique des fragments d'ADN, les sondes d'ADN contenant des régions différentes entre les ADN génomiques sont conçues.

3. Procédé de production d'une pluralité de sondes ADN selon la revendication 1, dans lequel les fragments d'ADN contiennent de 100 à 500 nucléotides.

4. Procédé d'analyse d'ADN génomique comprenant les étapes consistant à : mettre les sondes d'ADN produites par le procédé de production d'une pluralité de sondes d'ADN selon l'une quelconque des revendications 1 à 3 en contact avec des fragments d'ADN dérivés d'ADN génomique soumis à analyse ; et détecter une hybridation survenant entre la pluralité de sondes d'ADN et les fragments d'ADN.

5. Procédé d'analyse d'ADN génomique selon la revendication 4, qui comprend en outre, avant la première étape de la revendication 4, une étape consistant à réaliser une réaction d'amplification d'acide nucléique en utilisant l'ADN génomique soumis à analyse et les amorces aléatoires pour obtenir les fragments d'ADN.

6. Procédé d'analyse d'ADN génomique selon la revendication 4, dans lequel les fragments d'ADN dérivés d'ADN génomique sont des marqueurs d'ADN et la présence ou l'absence des marqueurs d'ADN est détectée en utilisant la pluralité de sondes d'ADN.
